(19) Europäisches Patentamt
European Patent Office
Office européen des brevets

(11) EP 4 317 188 A1

(12) EUROPEAN PATENT APPLICATION
published in accordance with Art. 153(4) EPC

(43) Date of publication:
**07.02.2024 Bulletin 2024/06**

(21) Application number: **22781261.7**

(22) Date of filing: **31.03.2022**

(51) International Patent Classification (IPC):
***C07K 16/28*** (2006.01) ***A61K 9/08*** (2006.01)
***A61K 9/20*** (2006.01) ***A61K 39/395*** (2006.01)
***A61K 47/42*** (2017.01) ***A61K 47/68*** (2017.01)
***A61K 47/69*** (2017.01) ***A61K 51/02*** (2006.01)
***A61K 51/08*** (2006.01) ***A61K 51/10*** (2006.01)
***A61P 35/00*** (2006.01) ***C07K 7/00*** (2006.01)
***C07K 7/08*** (2006.01) ***C07K 19/00*** (2006.01)
***C12N 15/13*** (2006.01) ***C12P 21/08*** (2006.01)
***G01T 1/161*** (2006.01)

(52) Cooperative Patent Classification (CPC):
**A61K 9/08; A61K 9/20; A61K 39/395; A61K 47/42; A61K 47/68; A61K 47/69; A61K 51/02; A61K 51/08; A61K 51/10; A61P 35/00; C07K 7/00; C07K 7/08; C07K 16/00; C07K 16/28; C07K 19/00;**

(Cont.)

(86) International application number:
**PCT/JP2022/016647**

(87) International publication number:
**WO 2022/211051 (06.10.2022 Gazette 2022/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.03.2021 JP 2021062104**
**02.11.2021 JP 2021179348**
**15.02.2022 JP 2022021670**

(71) Applicant: **Nihon Medi-Physics Co., Ltd.**
**Tokyo 136-0075 (JP)**

(72) Inventors:
- **KAWATANI, Minoru**
  **Tokyo 136-0075 (JP)**
- **HANADA, Takahisa**
  **Tokyo 136-0075 (JP)**
- **TONOYA, Gota**
  **Tokyo 136-0075 (JP)**
- **TAKEDA, Takuya**
  **Tokyo 136-0075 (JP)**

(74) Representative: **J A Kemp LLP**
**80 Turnmill Street**
**London EC1M 5QU (GB)**

Remarks:
The complete document including Reference Table(s) and the Sequence Listing(s) can be downloaded from the EPO website

# (54) RADIOACTIVE COMPLEX OF ANTI-EGFR ANTIBODY, AND RADIOPHARMACEUTICAL

(57) A conjugate having stability improved more than conventional conjugates without impairing the efficacy is provided by the present invention. The conjugate of the present invention is a conjugate of an anti-EGFR antibody site-specifically modified with a peptide and a chelating agent, wherein the chelating agent is chelated with a metal radionuclide, the peptide and the chelating agent are linked by a linker (L), and the linker (L) does not contain a thiourea bond.



Processed by Luminess, 75001 PARIS (FR)

(Cont. next page)

[Fig. 1]

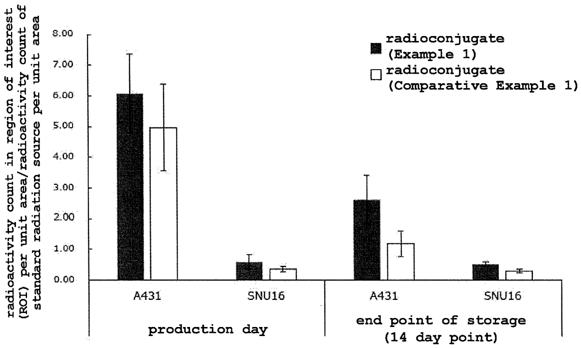


(52) Cooperative Patent Classification (CPC): (Cont.)
**G01T 1/161**

## Description

[Technical Field]

The present invention relates to a radioconjugate of an anti-EGFR antibody, and a radiopharmaceutical.

[Background Art]

EGFR (Epidermal Growth Factor Receptor) is a tyrosine kinase type receptor that recognizes a growth factor that controls proliferation and growth of cells, and performs signal transduction, and is a transmembrane protein with a molecular weight of about 170 kDa. It is also called HER1 or ErbB1.

As an anti-EGFR antibody, cetuximab is known. Cetuximab is a monoclonal antibody that inhibits the action of EGFR, used as an anticancer agent, and one of the molecule-targetting therapeutic drugs that target specific molecules involved in cancer growth and the like.

It is known that cetuximab is an antibody used in ADCs (Antibody Drug Conjugates) on the market. Examples of ADC using cetuximab include a medicament in which payload (drug), which is a chemotherapeutic agent or photosensitive substance, is covalently bonded to an antibody via a linker.

Antibody drugs have high target selectivity and relatively few side effects, but the efficacy thereof is sometimes insufficient. Chemotherapeutic agents as one type of payload have strong efficacy, but their low target selectivity increases the minimum effective dose necessary for killing cancer cells, and decreases the maximum tolerated dose because the dose cannot be increased much from the aspect of toxicity, thus causing a problem of narrow range of the therapeutic dosage.

According to ADC, higher amounts of chemotherapeutic agents can be selectively delivered to cancer cells. This is expected to result in wider therapeutic dosage ranges because lower doses achieve effects, chemotherapeutic agents that reach normal cells decrease, and the maximum tolerated doses increase.

One approach to ADC is a radioimmunoconjugate. In radioimmunoconjugates, radionuclides are used instead of payloads.

Patent Literature 1 describes a radioactive pharmaceutical composition in which cetuximab is modified with radioactive copper by using a bifunctional chelating agent such as 3,6,9,15-tetraazabicyclo[9.3.1]-pentadeca-1(15),11,13-triene-3,6,9-triacetic acid (PCTA) and the like.

In addition, radioimmunoconjugates using radionuclides that emit gamma-ray and positron as radionuclides can be utilized for nuclear medicine examinations. Patent Literature 2 describes that cetuximab can be modified by introducing DTPA into a peptide that site-specifically modifies the Fc region of an antibody, and labeled with a radioactive metal nuclide.

[Citation List]

[Patent Literature]

[PTL 1]
JP-A-2017-214308
[PTL 2]
WO 2017/217347

[Summary of Invention]

However, it has been clarified from the findings of the present inventors that conventional radioconjugates of the anti-EGFR antibody have problems such as low stability.

Patent Literature 1 does not describe site-specific modification of anti-EGFR antibody with peptides. In addition, it does not disclose or suggest the problem of anti-EGFR antibody that forms a thiourea bond.

One embodiment of the present invention is a conjugate of an anti-EGFR antibody site-specifically modified with a peptide and a chelating agent, wherein the chelating agent is chelated with a metal radionuclide, the peptide and the chelating agent are linked by a linker (L), and the linker (L) does not contain a thiourea bond.

Another embodiment of the present invention is a radiopharmaceutical containing the above-mentioned conjugate as an active ingredient.

In addition, another embodiment of the present invention is a radiopharmaceutical containing a conjugate of a

chelating agent chelated with a metal radionuclide and an anti-EGFR antibody as active ingredients, wherein the linkage between the anti-EGFR antibody and the chelating agent does not contain a thiourea bond, and the conjugate has a radiochemical purity of not less than 90% when stored at room temperature for 7 days.

The "linkage" in the present invention means both direct connection and indirect connection, unless otherwise specified.

According to the present invention, a radioconjugate of an anti-EGFR antibody is provided which has stability improved more than conventional conjugates without impairing the efficacy.

[Brief Description of Drawings]

[Fig. 1]
A graph showing evaluation results of the antigen-binding properties of radioconjugates prepared as described in Example 1 and Comparative Example 1. The vertical axis indicates values obtained by normalizing the value obtained by dividing the count value in the region of interest (ROI) set on the tumor section used by the area of the ROI, with the value obtained by dividing the count value of the standard radiation source by the area of the standard radiation source. The horizontal axis indicates the cell type of the tumor section used for evaluation. The graph represents the mean±standard deviation of each sample (n=10).

[Fig. 2]
Diagrams showing representative PET imagings (MIP images) of radioconjugate produced according to Example 2 in tumor-bearing mouse. Arrows indicate tumors derived from A431 cells used for tumor-bearing.

[Fig. 3]
Diagrams showing representative PET imagings (MIP images) of radioconjugate produced according to Comparative Example 2 in tumor-bearing mouse. Arrows indicate tumors derived from A431 cells used for tumor-bearing.

[Fig. 4]
A graph showing changes in tumor volume over time in tumor-bearing mice of each radioconjugate (Example 1) administration group, radioconjugate (Comparative Example 1) administration group, an antibody control group, and a Vehicle group. The vertical axis indicates relative values when the tumor volume at the time of administration of each medicament is set to 1, and the horizontal axis indicates the number of days elapsed since administration of each medicament. The graph represents the mean±standard deviation of tumor volume in each group, "**" is the time point when a significant difference ($p<0.01$) was observed from the antibody control group, "†" is the time point when a significant difference ($p<0.05$) was observed from the Vehicle group, and "‡" is the time point when a significant difference ($p<0.01$) was observed from the Vehicle group.

[Fig. 5]
A graph showing changes in body weight over time in tumor-bearing mice of a radioconjugate (Example 1) administration group, a radioconjugate (Comparative Example 1) administration group, an antibody control group, and a vehicle group. The vertical axis indicates relative values when the body weight at the time of administration of each medicament is set to 1, and the horizontal axis indicates the number of days elapsed since administration of each medicament. The graph represents the mean±standard deviation of body weight in each group.

[Fig. 6]
A graph showing evaluation results of the specificity of radioconjugates prepared as described in Example 6 for EGFR. The vertical axis indicates values obtained by normalizing the value obtained by dividing the count value in ROI set on the tumor section used by the area of the ROI, with the value obtained by dividing the count value of the standard radiation source by the area of the standard radiation source. The horizontal axis indicates the cell type of the tumor section used for evaluation. The graph represents the mean±standard deviation of each sample (n=10).

[Fig. 7A]
Graphs showing the cytotoxic effect of $^{225}$Ac complex-labeled panitumumab on COLO205 cells (upper row) and HCT-116 cells (lower row). Unlabeled panitumumab was added as a comparison object. The vertical axis shows the relative value when the number of viable cells under conditions where no antibody was added is set to 1, and the horizontal axis shows the concentration of the added antibody. The graphs show the mean±standard deviation in each group.

[Fig. 7B]
Graphs showing the cytotoxic effect of $^{225}$Ac complex-labeled panitumumab on SW48 cells (upper row) and MIAPaCa-2 cells (lower row). Unlabeled panitumumab was added as a comparison object. The vertical axis shows the relative value when the number of viable cells under conditions where no antibody was added is set to 1, and the horizontal axis shows the concentration of the added antibody. The graphs show the mean±standard deviation in each group.

[Fig. 7C]
A graph showing the cytotoxic effect of $^{225}$Ac complex-labeled panitumumab on NCI-H358 cells. Unlabeled panitumumab was added as a comparison object. The vertical axis shows the relative value when the number of viable cells under conditions where no antibody was added is set to 1, and the horizontal axis shows the concentration of the added antibody. The graph represents the mean±standard deviation in each group.
[Fig. 8]
Graphs showing the apoptosis induction potency of $^{225}$Ac complex-labeled panitumumab on COLO205 cells and SW48 cells. Unlabeled panitumumab was added as a comparison object. The vertical axis shows the relative value when the Caspase-3/7 activity under conditions where no antibody was added is set to 1, and the horizontal axis shows the concentration of the added antibody. The graphs show the mean±standard deviation in each group.
[Fig. 9A]
A diagram showing the DNA double-strand break effect of $^{225}$Ac complex-labeled panitumumab on SW48 cells. Cell nuclei were stained and detected with DAPI (upper image), and γH2AX was stained and detected (lower image).
[Fig. 9B]
A diagram showing the DNA double-strand break effect of $^{225}$Ac complex-labeled panitumumab on MIAPaCa-2 cells. Cell nuclei were stained and detected with DAPI (upper image), and γH2AX was stained and detected (lower image).
[Fig. 9C]
A diagram showing the DNA double-strand break effect of $^{225}$Ac complex-labeled panitumumab on NCI-H358 cells. Cell nuclei were stained and detected with DAPI (upper image), and γH2AX was stained and detected (lower image).
[Fig. 10]
A graph showing changes in tumor volume over time in tumor-bearing mice (COLO205 cells) of radioconjugate (Example 6) high radioactivity administration group, radioconjugate (Example 6) low radioactivity administration group, antibody peritoneal administration group, antibody control group, and Vehicle group. The vertical axis indicates mean tumor volume of each group, and the horizontal axis indicates the number of days elapsed since administration of each medicament. The graph represents the mean±standard deviation of tumor volume in each group, "*" is the time point when a significant difference (p<0.05) was observed from the antibody control group; "**" is the time point when a significant difference (p<0.01) was observed from the antibody control group, "†" is the time point when a significant difference (p<0.05) was observed from the Vehicle group, and "§" is the time point when a significant difference (p<0.01) was observed from the antibody peritoneal administration group.
[Fig. 11]
A graph showing changes in tumor volume over time in tumor-bearing mice (HCT-116 cells) of radioconjugate (Example 6) high radioactivity administration group, radioconjugate (Example 6) low radioactivity administration group, antibody peritoneal administration group, antibody control group, and Vehicle group. The vertical axis indicates mean tumor volume of each group, and the horizontal axis indicates the number of days elapsed since administration of each medicament. The graph represents the mean±standard deviation of tumor volume in each group, "**" is the time point when a significant difference (p<0.01) was observed from the antibody control group, "‡" is the time point when a significant difference (p<0.01) was observed from the Vehicle group, "§" is the time point when a significant difference (p<0.01) was observed from the antibody peritoneal administration group, and "II" is the time point when a significant difference (p<0.05) was observed from the radioconjugate (Example 6) high radioactivity administration group.
[Fig. 12]
A graph showing changes in tumor volume over time in tumor-bearing mice (SW48 cells) of radioconjugate (Example 6) high radioactivity administration group, radioconjugate (Example 6) low radioactivity administration group, antibody peritoneal administration group, antibody control group, and Vehicle group. The vertical axis indicates mean tumor volume of each group, and the horizontal axis indicates the number of days elapsed since administration of each medicament. The graph represents the mean±standard deviation of tumor volume in each group, "**" is the time point when a significant difference (p<0.01) was observed from the antibody control group and "‡" is the time point when a significant difference (p<0.01) was observed from the Vehicle group.
[Fig. 13]
A graph showing changes in tumor volume over time in COLO205 tumor-bearing mice of radioconjugate (Example 6) administration group, oxaliplatin administration group, and Vehicle group.
[Fig. 14]
Graphs showing measurement results of hepatotoxicity (upper graph and middle graph) and kidney toxicity (bottom graph) markers on the final day of observation in COLO205 tumor-bearing mice of radioconjugate (Example 6) administration group, oxaliplatin administration group, and Vehicle group.
[Fig. 15]
A diagram showing representative PET imaging (MIP image) of COLO205 cell tumor-bearing mouse administered

with a radioconjugate produced as described in Example 2.

[Description of Embodiments]

(1) Radioconjugate

The present invention is directed to a conjugate of an anti-EGFR antibody site-specifically modified with a peptide and a chelating agent, wherein the chelating agent is chelated with a metal radionuclide, the peptide and the chelating agent are linked by a linker (L), and the linker (L) does not contain a thiourea bond (hereinafter to be also referred to as the radioconjugate of the present invention).

(1-1) Metal radionuclide

The metal radionuclide contained in the radioconjugate of the present invention is a radionuclide that emits $\alpha$-ray, a radionuclide that emits $\beta$-ray, a radionuclide that emits positron, or a radionuclide that emits $\gamma$-ray. When the radioconjugate of the present invention is used for cancer therapy, it is preferable to use a radionuclide that emits $\alpha$-ray or a radionuclide that emits $\beta$-ray. When the radioconjugate of the present invention is used for cancer diagnosis or detection, it is preferable to use a radionuclide that emits positron, or a radionuclide that emits $\gamma$-ray. Examples of the radionuclide that emits $\alpha$-ray include Bi-212, Bi-213, Ac-225, and Th-227. Examples of the radionuclide that emits $\beta$-ray include Cu-64, $\gamma$-90, and Lu-177. Examples of the radionuclide that emits positron include Cu-64, Ga-68, Y-86, and Zr-89. Examples of the radionuclide that emits $\gamma$-ray include Tc-99m and In-111. The metal radionuclide to be contained in the radioconjugate of the present invention is more preferably Ga-68, Zr-89, $\gamma$-90, In-111, Lu-177, or Ac-225, further preferably Zr-89, $\gamma$-90, Lu-177, or Ac-225.

(1-2) Antibody

The antibody to be contained in the radioconjugate of the present invention is an immunoglobulin that specifically binds to EGFR (hereinafter also to be referred to as the antibody used in the present invention). The antibody to be used in the present invention may be a polyclonal antibody or a monoclonal antibody, preferably a monoclonal antibody. The origin of the antibody is not particularly limited, and examples include antibodies of non-human animals, non-human mammals, and humans, preferably antibodies of human, rat, mouse, and rabbit. When the antibody is derived from species other than human, it is preferably chimerized or humanized using well-known techniques. The antibody to be used in the present invention may be a chimera antibody, a humanized antibody, or a human antibody. The antibody to be used in the present invention may be a bispecific antibody. The antibody to be used in the present invention is, for example, IgG, for example, IgG1, IgG2 (e.g., IgG2a or IgG2b), IgG3, or IgG4.

The antibody used in the radioconjugate of the present invention is more preferably cetuximab or panitumumab.

Cetuximab is a human-mouse chimeric monoclonal antibody of the $IgG_1$ subclass that specifically recognizes EGFR, and it is known that inhibition of the activation and dimerization of EGFR affords a tumor growth suppressive effect on colorectal cancer, head and neck cancer, non-small cell lung cancer, gastric cancer, and the like.

The chemical name (nomenclature) of cetuximab is a glycoprotein composed of two light chain molecules consisting of 214 amino acid residues ($C_{1025}H_{1595}N_{281}O_{338}S_5$; molecular weight :23,422.64) and two heavy chain molecules consisting of 449 amino acid residues ($C_{2208}H_{3400}N_{582}O_{674}S_{15}$; molecular weight :49,363.09), which is produced by the mouse hybridoma SP2/0-Ag14 cell line by introducing cDNA encoding a human/mouse chimeric monoclonal antibody consisting of a mouse anti-human epidermal growth factor receptor monoclonal antibody variable region and human $IgG_1$ constant region.

In the present specification, cetuximab is an antibody described in JP-A-2005-047934, and specifically, a humanized antibody containing a light chain amino acid sequence (SEQ ID NO: 1):

DILLTQSPVILSVSPGERVSFSCRASQSIGTNIHWYQQRTNGSPRLLIKYASESISGIPS 60

RFSGSGSGTDFTLSINSVESEDIADYYCQQNNNWPTTFGAGTKLELKRTVAAPSVFIFPP 120

SDEQLKSGTASVVCLLNNFYPREAKVQWKVDNALQSGNSQESVTEQDSKDSTYSLSSTLT 180

LSKADYEKHKVYACEVTHQGLSSPVTKSFNRGEC

and a heavy chain amino acid sequence (SEQ ID NO: 2):

QVQLKQSGPGLVQPSQSLSITCTVSGFSLTNYGVHWVRQSPGKGLEWLGVIWSGGNTDYN 60

TPFTSRLSINKDNSKSQVFFKMNSLQSNDTAIYYCARALTYYDYEFAYWGQGTLVTVSAA 120

STKGPSVFPLAPSSKSTSGGTAALGCLVKDYFPEPVTVSWNSGALTSGVHTFPAVLQSSG 180

LYSLSSVVTVPSSSLGTQTYICNVNHKPSNTKVDKRVEPKSCDKTHTCPPCPAPELLGGP 240

SVFLFPPKPKDTLMISRTPEVTCVVVDVSHEDPEVKFNWYVDGVEVHNAKTKPREEQYNS 300

TYRVVSVLTVLHQDWLNGKEYKCKVSNKALPAPIEKTISKAKGQPREPQVYTLPPSREEM 360

TKNQVSLTCLVKGFYPSDIAVEWESNGQPENNYKTTPPVLDSDGSFFLYSKLTVDKSRWQ 420

QGNVFSCSVMHEALHNHYTQKSLSLSPGK

Cetuximab is used clinically as an antitumor agent indicated for unresectable advanced/recurrent colorectal cancer and head and neck cancer with wild-type RAS gene, and is available as Erbitux (registered trademark).

Panitumumab is a humanized IgG2 monoclonal antibody that specifically recognizes EGFR. Its chemical name (nomenclature) is a glycoprotein (molecular weight: about 147,000) composed of two light chain molecules consisting of 214 amino acid residues ($C_{1028}H_{1588}N_{274}O_{336}S_6$; molecular weight: 23,353.63) and two heavy chain molecules consisting of 445 amino acid residues ($C_{2171}H_{3355}N_{573}O_{672}S_{18}$; molecular weight : 48,811.47), which is produced by Chinese hamster ovary cells introduced with a genomic DNA encoding IgG2 which is a human anti-human EGFR monoclonal antibody, in which the main component of heavy chain subunit lacks a C-terminal lysine.

The amino acid sequence of panitumumab is shown as follows in the review report by the Pharmaceuticals and Medical Devices Agency for Vectibix Intravenous Injection 100 mg "Takeda Bio".

light chain (SEQ ID NO: 3):

Asp - Ile - Gln - Met - Thr - Gln - Ser - Pro - Ser - Ser - Leu - Ser - Ala - Ser - Val - Gly - Asp - Arg - Val - Thr -
Ile - Thr - Cys - Gln - Ala - Ser - Gln - Asp - Ile - Ser - Asn - Tyr - Leu - Asn - Trp - Tyr - Gln - Gln - Lys - Pro -
Gly - Lys - Ala - Pro - Lys - Leu - Leu - Ile - Tyr - Asp - Ala - Ser - Asn - Leu - Glu - Thr - Gly - Val - Pro - Ser -
Arg - Phe - Ser - Gly - Ser - Gly - Ser - Gly - Thr - Asp - Phe - Thr - Phe - Thr - Ile - Ser - Ser - Leu - Gln - Pro -
Glu - Asp - Ile - Ala - Thr - Tyr - Phe - Cys - Gln - His - Phe - Asp - His - Leu - Pro - Leu - Ala - Phe - Gly - Gly -
Gly - Thr - Lys - Val - Glu - Ile - Lys - Arg - Thr - Val - Ala - Ala - Pro - Ser - Val - Phe - Ile - Phe - Pro - Pro -
Ser - Asp - Glu - Gln - Leu - Lys - Ser - Gly - Thr - Ala - Ser - Val - Val - Cys - Leu - Leu - Asn - Asn - Phe - Tyr -
Pro - Arg - Glu - Ala - Lys - Val - Gln - Trp - Lys - Val - Asp - Asn - Ala - Leu - Gln - Ser - Gly - Asn - Ser - Gln -
Glu - Ser - Val - Thr - Glu - Gln - Asp - Ser - Lys - Asp - Ser - Thr - Tyr - Ser - Leu - Ser - Ser - Thr - Leu - Thr -
Leu - Ser - Lys - Ala - Asp - Tyr - Glu - Lys - His - Lys - Val - Tyr - Ala - Cys - Glu - Val - Thr - His - Gln - Gly -
Leu - Ser - Ser - Pro - Val - Thr - Lys - Ser - Phe - Asn - Arg - Gly - Glu - Cys[1]

heavy chain (SEQ ID NO: 4):

Gln* - Val - Gln - Leu - Gln - Glu - Ser - Gly - Pro - Gly - Leu - Val - Lys - Pro - Ser - Glu - Thr - Leu - Ser - Leu -
Thr - Cys - Thr - Val - Ser - Gly - Gly - Ser - Val - Ser - Ser - Gly - Asp - Tyr - Tyr - Trp - Thr - Trp - Ile - Arg -
Gln - Ser - Pro - Gly - Lys - Gly - Leu - Glu - Trp - Ile - Gly - His - Ile - Tyr - Tyr - Ser - Gly - Asn - Thr - Asn -
Tyr - Asn - Pro - Ser - Leu - Lys - Ser - Arg - Leu - Thr - Ile - Ser - Ile - Asp - Thr - Ser - Lys - Thr - Gln - Phe -
Ser - Leu - Lys - Leu - Ser - Ser - Val - Thr - Ala - Ala - Asp - Thr - Ala - Ile - Tyr - Tyr - Cys - Val - Arg - Asp -
Arg - Val - Thr - Gly - Ala - Phe - Asp - Ile - Trp - Gly - Gln - Gly - Thr - Met - Val - Thr - Val - Ser - Ser - Ala -
Ser - Thr - Lys - Gly - Pro - Ser - Val - Phe - Pro - Leu - Ala - Pro - Cys - Ser - Arg - Ser - Thr - Ser - Glu - Ser -
Thr - Ala - Ala - Leu - Gly - Cys - Leu - Val - Lys - Asp - Tyr - Phe - Pro - Glu - Pro - Val - Thr - Val - Ser - Trp -
Asn - Ser - Gly - Ala - Leu - Thr - Ser - Gly - Val - His - Thr - Phe - Pro - Ala - Val - Leu - Gln - Ser - Ser - Gly -
Leu - Tyr - Ser - Leu - Ser - Ser - Val - Val - Thr - Val - Pro - Ser - Ser - Asn - Phe - Gly - Thr - Gln - Thr - Tyr -
Thr - Cys - Asn - Val - Asp - His - Lys - Pro - Ser - Asn - Thr - Lys - Val - Asp - Lys - Thr - Val - Glu - Arg - Lys -
Cys - Cys$^{1}$ - Val - Glu - Cys$^{2}$ - Pro - Pro - Cys$^{3}$ - Pro - Ala - Pro - Pro - Val - Ala - Gly - Pro - Ser - Val - Phe - Leu -
Phe - Pro - Pro - Lys - Pro - Lys - Asp - Thr - Leu - Met - Ile - Ser - Arg - Thr - Pro - Glu - Val - Thr - Cys - Val -
Val - Val - Asp - Val - Ser - His - Glu - Asp - Pro - Glu - Val - Gln - Phe - Asn - Trp - Tyr - Val - Asp - Gly - Val -
Glu - Val - His - Asn - Ala - Lys - Thr - Lys - Pro - Arg - Glu - Glu - Gln - Phe - Asn - Ser - Thr - Phe - Arg - Val -
Val - Ser - Val - Leu - Thr - Val - Val - His - Gln - Asp - Trp - Leu - Asn - Gly - Lys - Glu - Tyr - Lys - Cys - Lys -
Val - Ser - Asn - Lys - Gly - Leu - Pro - Ala - Pro - Ile - Glu - Lys - Thr - Ile - Ser - Lys - Thr - Lys - Gly - Gln -
Pro - Arg - Glu - Pro - Gln - Val - Tyr - Thr - Leu - Pro - Pro - Ser - Arg - Glu - Glu - Met - Thr - Lys - Asn - Gln -
Val - Ser - Leu - Thr - Cys - Leu - Val - Lys - Gly - Phe - Tyr - Pro - Ser - Asp - Ile - Ala - Val - Glu - Trp - Glu -
Ser - Asn - Gly - Gln - Pro - Glu - Asn - Asn - Tyr - Lys - Thr - Thr - Pro - Pro - Met - Leu - Asp - Ser - Asp - Gly -
Ser - Phe - Phe - Leu - Tyr - Ser - Lys - Leu - Thr - Val - Asp - Lys - Ser - Arg - Trp - Gln - Gln - Gly - Asn - Val -
Phe - Ser - Cys - Ser - Val - Met - His - Glu - Ala - Leu - His - Asn - His - Tyr - Thr - Gln - Lys - Ser - Leu - Ser -
Leu - Ser - Pro - Gly - Lys**

Here, intramolecular disulfide bonds are shown by solid lines. Intermolecular disulfide bonds are formed between the Cys222 residue of the heavy chain and the Cys214 residue of the light chain, between the Cys225 residues of the heavy chains, and between the Cys228 residues of the heavy chains.

The underlined Asn (Asn) is the sugar chain binding position, and Gln marked with an asterisk (Gln*) is partially cyclized to pyroglutamic acid. Lys marked with two asterisks (Lys**) is almost completely missing.

The sugar chain structure is shown below.

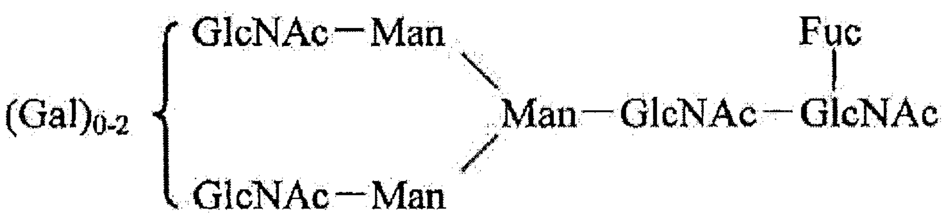

Here, Fuc is L-fucose, (Gal)0-2 is 0, 1 or 2 molecules of D-galactose, GlucNac is D-N-acetylglucosamine, and Man is D-mannose.

Panitumumab is used clinically as an antitumor agent indicated for unresectable advanced/recurrent colorectal cancer with wild-type KRAS gene, and is available as Vectibix (registered trademark).

(1-3) Chelating agent

In the present invention, the chelating agent is not particularly limited as long as it has a site in the structure thereof where metal radionuclide is coordinated. Examples of the chelating agent include CB-TE2A (1,4,8,11-Tetraazabicyclo[6.6.2]hexadecane-4,11-diacetic acid), CDTA(Cyclohexane-trans-1,2-diamine tetra-acetic acid), CDTPA (4-cyano-4-[[(dodecylthio)thioxomethyl]thio]-Pentanoic acid), DOTA (1,4,7,10-Tetraazacyclododecane-1,4,7,10-tetraacetic acid), DOTMA ((1R,4R,7R,10R)-α,α',α'',α'''-tetramethyl-1,4,7,10-tetraazacyclododecane-1,4,7,10-tetraacetic acid), DOTAM (1,4,7,10-tetrakis(carbamoylmethyl)-1,4,7,10-tetraazacyclododecane), DOTPA (1,4,7,10-tetraazacyclododecane-1,4,7,10-tetra propionic acid), 1,4,7,10-tetrakis(pyridin-2-ylmethyl)-1,4,7,10-tetraazacyclododecane ($L^{py}$), DOTA-GA (α-(2-Carboxyethyl)-1,4,7,10-tetraazacyclododecane-1,4,7,10-tetraacetic acid), DOTP (((1,4,7,10-Tetraazacyclododecane-1,4,7,10-tetrayl)tetrakis(methylene))tetraphosphonic acid), DOTMP (1,4,7,10-Tetraazacyclododecane-1,4,7,10-tetrakis(methylenephosphonic acid)), DOTA-4AMP (1,4,7,10-tetraazacyclododecane-1,4,7,10-tetrakis(acetamidomethylenephosphonic acid), D02P (Tetraazacyclododecane dimethanephosphonic acid), Deferoxamine (DFO), DTPA (Glycine, N,N-bis[2-[bis(carboxymethyl)amino]ethyl]-), CHX-A''-DTPA (2,2'-((2-(((1S,2R)-2-(bis(carboxymethyl)amino)cyclohexyl)(carboxymethyl)amino)ethyl) azanediyl)diacetic acid), DTPA-BMA (5,8-Bis(carboxymethyl)-11-[2-(methylamino)-2-oxoethyl]-3-oxo-2,5,8,11-tetraazatridecan-13-oic acid), EDTA (2,2',2'',2'''-(ethane-1,2-diylbis(azanetriyl))tetraacetic acid), NOTA (1,4,7-Triazacyclononane-1,4,7-triacetic acid), NOTP (1,4,7-Triazacyclononane-1,4,7-triyltris(methylenephosphonic acid)), TETPA (1,4,8,11-tetraazacyclotetradecane-1,4,8,11-tetrapropionic acid), TETA (1,4,8,11-Tetraazacyclotetradecane-N,N',N'',N'''-tetraacetic acid), TTHA (3,6,9,12-Tetrakis(carboxymethyl)-3,6,9,12-tetraazatetradecanedioic acid), HEHA (1,2,7,10,13-hexaazacyclooctadecane-1,4,7,10,13,16-hexaacetic acid), 1,2-HOPO (N,N',N'',N'''-tetra(1,2-dihydro-1-hydroxy-2-oxopyridine-6-carbonyl)-1,5,10,14-tetraazatetradecane), PEPA (1,4,7,10,13-pentaazacyclopentadecane-N,N',N'',N''',N''''-pentaacetic acid), H4octapa (N,N'-bis(6-carboxy-2-pyridylmethyl)-ethylenediamine-N,N'-diacetic acid), H2bispa2 (6,6'-({9-hydroxy-1,5-bis(methoxycarbonyl)-2,4-di(pyridin-2-yl)-3,7-diazabicyclo[3.3.1]nonane-3,7-diyl}bis(-methylene))dipicolinic acid), H2dedpa (1,2-[{6-(carboxy)-pyridin-2-yl}-methylamino]ethane), H2macropa (6-(1,4,10,13-tetraoxa-7,16-diazacyclooctadecan-N,N'-methyl)picolinic acid), H5decapa (N,N''-bis(6-carboxy-2-pyridylmethyl)-diethylenetriamine-N,N',N''-triacetic acid), H6phospa (N,N'-(methylenephosphonate)-N,N'-[6-(methoxycarbonyl)pyridin-2-yl]-methyl-1,2-diaminoethane), HP-D03A (Hydroxypropyltetraazacyclododecanetriacetic acid), and porphyrin. A compound represented by the following formula (A) is preferred.

(A)

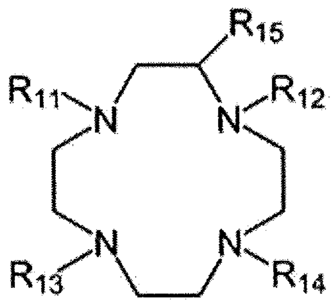

In the formula (A), $R_{11}$, $R_{12}$, $R_{13}$ and $R_{14}$ are each independently a group consisting of $-(CH_2)_pCOOH$, $-(CH_2)_pC_5H_5N$, $-(CH_2)_pPO_3H_2$, $-(CH_2)_pCONH_2$ or $-(CHCOOH)(CH_2)_pCOOH$, $R_{15}$ is a hydrogen atom, and p is an integer of not less than 0 and not more than 3.

The compound represented by the formula (A) is preferably a compound containing a structure derived from 1,4,7,10-tetraazacyclododecane-1,4,7,10-tetraacetic acid (DOTA), or a derivative thereof. Specifically, the compound can contain, in its structure, a structure derived from one chelating agent selected from DOTA (1,4,7,10-Tetraazacyclododecane-1,4,7,10-tetraacetic acid), DOTMA ((1R,4R,7R,10R)-α,α',α'',α'''-tetramethyl-1,4,7,10-tetraazacyclododecane-1,4,7,10-tetraacetic acid), DOTAM (1,4,7,10-tetrakis(carbamoylmethyl)-1,4,7,10-tetraazacyclododecane), DOTPA (1,4,7,10-tetraazacyclododecane-1,4,7,10-tetrapropionic acid), 1,4,7,10-tetrakis(pyridin-2-ylmethyl)-1,4,7,10-tetraazacyclododecane ($L^{py}$), DOTA-GA (α-(2-Carboxyethyl)-1,4,7,10-tetraazacyclododecane-1,4,7,10-tetraacetic acid), DOTP (((1,4,7,10-Tetraazacyclododecane-1,4,7,10-tetrayl)tetrakis(methylene))tetraphosphonic acid), DOTMP (1,4,7,10-Tetraazacyclododecane-1,4,7,10-tetrakis(methylenephosphonic acid)), DOTA-4AMP (1,4,7,10-tetraazacyclododecane-1,4,7,10-tetrakis(acetamidomethylenephosphonic acid), and D02P (Tetraazacyclododecane dimethanephosphonic acid). More preferably, compounds represented by the following formulas (A-1) to (A-6) can be mentioned. The chelating agent to be used in the radioconjugate of the present invention is further preferably DOTA-GA (compound represented by the formula (A-6)). The chelating agent used in the radioconjugate of the present invention is further more preferably DOTA-GA (a compound represented by formula (A-6)). When the chelating agent used is DOTA-GA, the aforementioned chelating agent may be a stereoisomer (S-form, R-form) or a racemate. The stereoisomers of S-form and R-form may be mixed in any ratio.

(A-1)

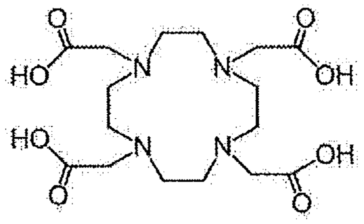

DOTA

(A-2)

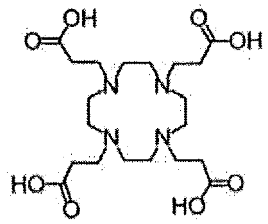

DOTPA

(A-3)

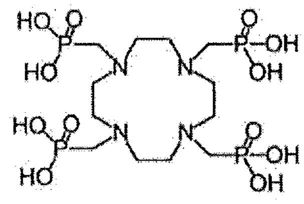

DOTMP

(A-4)

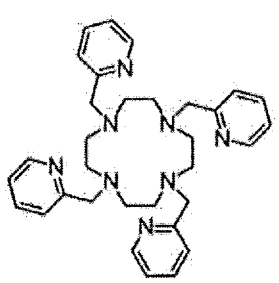

$L^{py}$

(A-5)

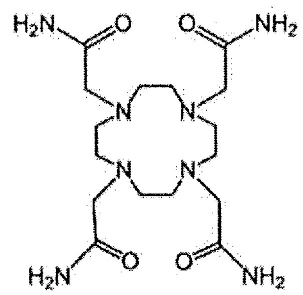

DOTAM

(A-6)

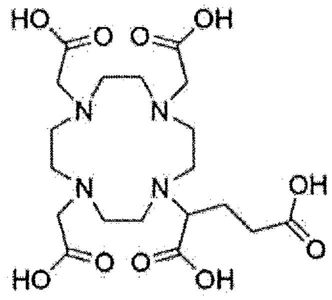

DOTA-GA

A chelating agent used in the present invention is linked with a peptide via a linker (L). In the radioconjugate of the present invention, the chelating agent and the linker (L) are preferably connected by a covalent bond. Therefore, in the radioconjugate of the present invention, some groups in the compound of the aforementioned chelating agent may be substituted by groups that form covalent bonds with the linker (L). For example, when the chelating agent used in the present invention is a compound represented by the formula (A), $R_{12}$ or $R_{15}$ may be substituted by a group that forms a covalent bond with the linker (L). Preferably, when $R_{12}$ is substituted by a group that forms a covalent bond with the linker (L), $R_{15}$ is a hydrogen atom, when $R_{12}$ is a group consisting of $-(CH_2)_pCOOH$, $-(CH_2)_pC_5H_5N$, $-(CH_2)_pPO_3H_2$, $-(CH_2)_pCONH_2$, or $-(CHCOOH)(CH_2)_pCOOH$, $R_{15}$ is substituted by a group that forms a covalent bond with the linker (L).

The covalent bond between the chelating agent and the linker (L) only needs to be free of a thiourea bond, and is exemplified by a carbon-carbon bond, an amide bond, an ether bond, an ester bond, and the like.

The connection between the chelating agent and the linker (L) can be formed, for example, by the reaction of an N-hydroxysuccinimide ester (NHS) group of the following formula (A-7) or (A-8), or a 2,6-dioxotetrahydro-2H-pyranyl group of the following formula (A-9), with the primary amine of linker (L) .

(A-7)

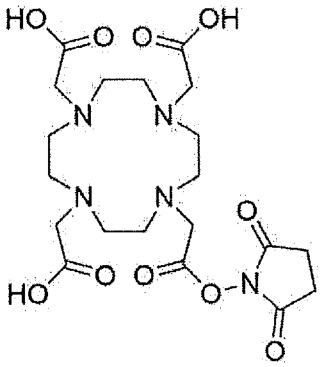

DO3A-NHS

(A-8)

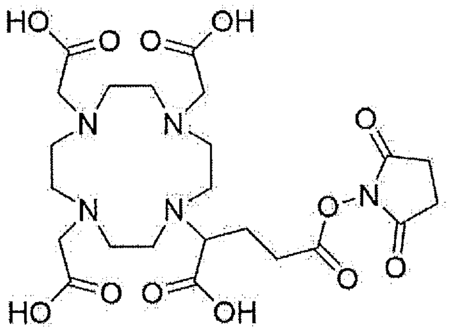

DOTA-GA-NHS

(A-9)

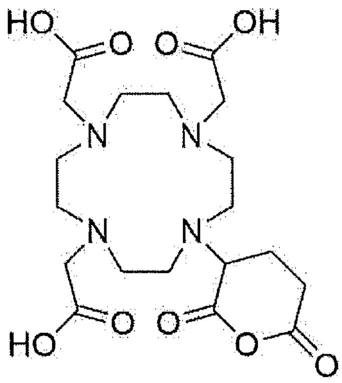

DOTA-GA-anhydride

(1-4) Antibody-modification peptide

In the present invention, the peptide is not particularly limited as long as it modifies the antibody site-specifically, preferably the Fc region site-specifically, more preferably the lysine residue in the Fc region of the antibody site-specifically. As a result, it is possible to maintain the activity of the antibody itself (antigen recognition action, neutralizing action, complement activating action and/or opsonin action).

The peptide to be used in the present invention may be a chain peptide or a cyclic peptide, and cyclic peptide is preferred. More preferably, it contains an amino acid sequence consisting of not less than 13 and not more than 17 amino acid residues represented by the following formula (i) (hereinafter, to be also referred to as "antibody-modification peptide"), and is modified with a crosslinking agent. In the explanation of the formula (i), the left side of the paper surface of the amino acid sequence indicates the N-terminal side, and the right side of the paper surface of the amino acid sequence indicates the C-terminal side.

(Xa)-Xaa1-(Xb)-Xaa2-(Xc)-Xaa3-(Xd)... (i)

In the formula (i), Xa, Xb, Xc and Xd are each continuous

X in the number of a, continuous X in the number of b, continuous X in the number of c, and continuous X in the number of d, respectively,

X is an amino acid residue having neither a thiol group nor a haloacetyl group in the side chain,

a, b, c and d are each independently an integer of not less than one and not more than 5, and satisfy a+b+c+d≤14,

Xaa1 and Xaa3 are each independently an amino acid residue derived from an amino acid having a thiol group in the side chain, or an amino acid residue derived from an amino acid having a haloacetyl group in the side chain, wherein either Xaa1 or Xaa3 is an amino acid residue derived from an amino acid having a thiol group, preferably Xaa1 and Xaa3 are linked to form a ring structure, and

Xaa2 is a lysine residue, arginine residue, cysteine residue, aspartic acid residue, glutamic acid residue, 2-aminosuberic acid, or diamino propionic acid, preferably lysine residue, and Xaa2 is modified with a crosslinking agent.

Examples of the amino acid residue that may be contained in X in the above-mentioned formula (i) include those derived from amino acids such as glycine, alanine, phenylalanine, proline, asparagine, aspartic acid, glutamic acid, arginine, histidine, serine, threonine, tyrosine, methionine and the like, and X may be an amino acid residue consisting of the same type of amino acid, or different types of amino acids.

In the formula (i), a, b, c and d are not particularly limited as long as they are numbers within the aforementioned range. From the aspect of the stability of binding between the peptide and anti-EGFR antibody, a+b+c+d≤14 is to be satisfied, and a is preferably an integer of not less than 1 and not more than 3, b is preferably an integer of not less than 1 and not more than 3, c is preferably an integer of not less than 3 and not more than 5, and d is preferably an integer of not less than 1 and not more than 3.

At least one of Xaa1 and Xaa3 is an amino acid residue derived from an amino acid having a thiol group in the side chain, and Xaa1 and Xaa3 may be the same or different. Examples of the amino acid having a thiol group in the side chain include cysteine and homocysteine. Such amino acid residues are preferably bonded by a disulfide bond, or a sulfide group is preferably bonded thereto via a structure shown by the following formula (4). In the formula (4), the

wavy line indicates the binding part with the sulfide group.

(4) 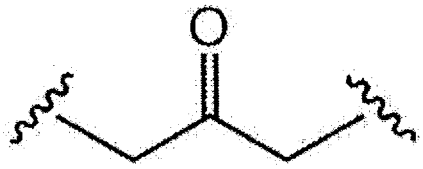

Instead of the aforementioned combination of Xaa1 and Xaa3, one of Xaa1 and Xaa3 may be an amino acid residue derived from an amino acid having a thiol group in the side chain, and the other may be an amino acid residue derived from an amino acid having a haloacetyl group in the side chain. These are bonded via a thioether bond. The terminal of the haloacetyl group is substituted with a halogen such as iodine or the like, and the halogen is eliminated by a reaction with the thiol group in the other side chain, whereby a thioether bond is formed.

Specific examples of the amino acid sequence of the antibody-modification peptide represented by the formula (i) include the peptides described in WO 2016/186206, WO 2017/217347 and WO 2018/230257, and these can also be used.

Preferably, the antibody-modification peptide to be used in the present invention is an amino acid sequence consisting of 13 to 17 amino acid residues represented by the following formula (i)'.

$$(X_{1-3})\text{-C-(Xaa3')-(Xaa4')-H-(Xaa1')-G-(Xaa2')-L-V-W-C-}(X_{1-3}) \qquad \text{(i)'}$$

In the formula (i)', each X is independently any amino acid residue other than cysteine,

C is a cysteine residue,
H is a histidine residue,
Xaa1' is a lysine residue, a cysteine residue, an aspartic acid residue, a glutamic acid residue, a 2-aminosuberic acid, or a diamino propionic acid,
G is a glycine residue,
Xaa2' is a glutamic acid residue or an asparagine residue,
L is a leucine residue,
V is a valine residue,
W is a tryptophan residue,
Xaa3' is an alanine residue, a serine residue, or a threonine residue, and
Xaa4' is a tyrosine residue or a tryptophan residue.

In the above-mentioned formula (i)', N-terminal or C-terminal $X_{1-3}$ means that any 1 to 3 amino acid residues X other than cysteine (C or Cys) are independently consecutive, and is a sequence consisting of the same or different amino acid residues, preferably all three different amino acid residues.

Among these, the amino acid sequence of the antibody-modification peptide preferably has any one of the following sequences (1) to (14), more preferably the following sequence (1), (2), (13) or (14). In the following amino acid sequences (1) to (14), (Xaa2) is a lysine residue, a cysteine residue, an aspartic acid residue, a glutamic acid residue, a 2-aminosuberic acid, or a diamino propionic acid, preferably a lysine residue, (Xaa2) is preferably modified with a crosslinking agent, and (Xaa1) and (Xaa3) are each a homocysteine residue. In the following amino acid sequences (1) to (14), the amino acids other than (Xaa1), (Xaa2) and (Xaa3) are indicated by one-letter abbreviations.

(1) DCAYH(Xaa2)GELVWCT (SEQ ID NO: 5)
(2) GPDCAYH(Xaa2)GELVWCTFH (SEQ ID NO: 6)
(3) RCAYH(Xaa2)GELVWCS (SEQ ID NO: 7)
(4) GPRCAYH(Xaa2)GELVWCSFH (SEQ ID NO: 8)
(5) SPDCAYH(Xaa2)GELVWCTFH (SEQ ID NO: 9)
(6) GDDCAYH(Xaa2)GELVWCTFH (SEQ ID NO: 10)
(7) GPSCAYH(Xaa2)GELVWCTFH (SEQ ID NO: 11)
(8) GPDCAYH(Xaa2)GELVWCSFH (SEQ ID NO: 12)
(9) GPDCAYH(Xaa2)GELVWCTHH (SEQ ID NO: 13)
(10) GPDCAYH(Xaa2)GELVWCTFY (SEQ ID NO: 14)
(11) SPDCAYH(Xaa2)GELVWCTFY (SEQ ID NO: 15)

(12) SDDCAYH(Xaa2)GELVWCTFY (SEQ ID NO: 16)
(13) RGNCAYH(Xaa2)GQLVWCTYH (SEQ ID NO: 17)
(14) G(Xaa1)DCAYH(Xaa2)GELVWCT(Xaa3)H (SEQ ID NO: 18)

The peptide represented by the above-mentioned formula (i) or (i)', or the peptide having the sequences (1) to (14) preferably has a linker (L) introduced at the N-terminal and is amidated at the C-terminal. Furthermore, Xaa2 (or a part corresponding to Xaa2) of these peptides is modified with a crosslinking agent, which allows the peptides to covalently bind to the Fc region of the anti-EGFR antibody via the crosslinking agent. In formula (i)', the part corresponding to Xaa2 is Xaa1'.

A crosslinking agent can be appropriately selected by those of ordinary skill in the art, and can be a compound having at least two sites bindable to desired amino acids (e.g., lysine, cysteine, aspartic acid, glutamic acid, 2-aminosuberic acid, diaminopropionic acid, arginine, etc.). Examples thereof include, but are not limited to, a crosslinking agent preferably containing two or more succinimidyl groups such as DSG (disuccinimidyl glutarate), DSS (disuccinimidyl suberate), and the like, a crosslinking agent preferably containing two or more imide acid moieties such as DMA (dimethyl adipimidate·2HCl, dimethyl adipimidate dihydrochloride), DMP (dimethyl pimelimidate·2HCl, dimethyl pimelimidate dihydrochloride), DMS (dimethyl suberimidate·2HCl, dimethyl suberimidate dihydrochloride), and the like, and a crosslinking agent having an SS bond such as DTBP (dimethyl 3,3'-dithiobispropionimidate·2HCl, dimethyl 3,3'-dithiobispropionimidate dihydrochloride) and DSP (dithiobis(succinimidyl propionate)), and the like, and SBAP (succinimidyl 3-(bromoacetamido)propionate). A crosslinking agent containing a succinimidyl group such as DSS or DSG reacts with a primary amine present at the N-terminal. Therefore, by blocking the N-terminal and reacting with DSS or DSG, only the amino group of Xaa2 can be specifically modified with DSS or DSG. For example, linker (L) may be previously introduced into the N-terminal of the antibody-modification peptide and then reacted with DSS or DSG. The anti-EGFR antibody is site-specifically modified with the peptide when the succinimidyl group of DSS or DSG reacts with, for example, Lys248 residue or Lys250 residue, preferably Lys250 residue, according to Eu numbering in cetuximab as an anti-EGFR antibody, and for example, Lys244 residue or Lys246 residue, preferably Lys246 residue, according to Eu numbering in panitumumab as an anti-EGFR antibody. These Lys residues are present in the Fc region of human IgG, and even if the antibody is anti-EGFR antibody other than cetuximab, those skilled in the art can align the amino acid sequence of the antibody and identify the corresponding Lys residue.

(1-5) Linker (L)

Linker (L) is not particularly limited as long as it can link a chelating agent and a peptide in the radioconjugate of the present invention. Linker (L) to be used in the present invention is not particularly limited as long as it does not contain a thiourea bond. Examples thereof include substituted or unsubstituted alkyl group, substituted or unsubstituted heteroalkyl group, polyethylene glycol (PEG) group, peptides, sugar chain, disulfide group, amide group, combination of these and the like.

In the present specification, linker (L) is a general term for linkers used for the connection of an anti-EGFR antibody modified with a peptide and a chelating agent, and includes antibody-modification linker ($L_1$) and chelate linker ($L_2$). The antibody-modification linker ($L_1$), which is described in detail later, is introduced into the N-terminal side of the peptide described in (1-4), and the chelate linker ($L_2$), which is described in detail later, is introduced into the functional group of the chelating agent described in (1-3).

The linker (L) used in the present invention may contain a binding site formed by a click reaction, and preferably, the antibody-modification linker ($L_1$) and the chelate linker ($L_2$) are bound by a click reaction. In the present invention, it is preferred that a thiourea bond is not contained between the binding site formed by the click reaction and the chelating agent. In other words, it is preferred that the chelate linker ($L_2$) does not contain a thiourea bond. As used herein, the binding site formed by the click reaction is preferably a triazole skeleton-containing structure represented by the following formula (10a) or (10b) or a pyridazine skeleton-containing structure represented by the following formula (10c) can be considered. Since the formula (10a) and the formula (10b) are isomers, they may be contained at any ratio.

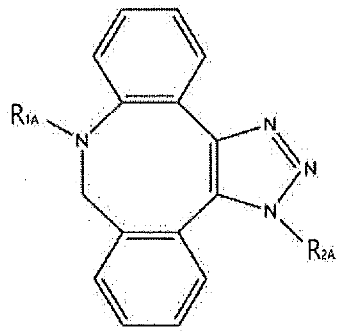

(10a)

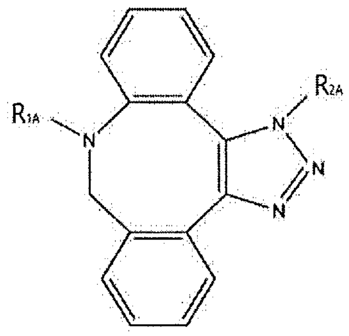

(10b)

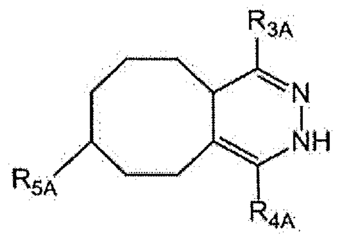

(10c)

In the formula (10a) and the formula (10b), $R_{1A}$ is a linkage site with a chelating agent, and $R_{2A}$ is a linkage site with an antibody-modification peptide. In the formula (10c), one of $R_{3A}$ and $R_{4A}$ is a hydrogen atom, a methyl group, a phenyl group or a pyridyl group, and the other is a linkage site with a chelating agent, and $R_{5A}$ is a linkage site with an antibody-modification peptide. In the formula (10a), the formula (10b), and the formula (10c), the linkage site with the antibody-modification peptide is linked with the peptide via the antibody-modification linker ($L_1$), and the linkage site with the chelating agent is linked with the chelating agent via the chelate linker ($L_2$).

In the radioconjugate of the present invention, the antibody is site-specifically modified with a peptide, and the peptide and a chelating agent are linked via a linker (L). Thus, one molecule or two molecules of the chelating agent are conjugated to one molecule of the anti-EGFR antibody.

(1-6) Production method of conjugate

The production method of the radioconjugate of the present invention includes two steps which are a conjugation step of conjugating a chelating agent and an anti-EGFR antibody, and a complex formation step of forming a complex of a metal radionuclide and a chelating agent. The conjugation step may be performed before the complex formation step or after the complex formation step.

In the conjugation step, the Fc region of an antibody is site-specifically modified with a chelating agent or linker (L) having the antibody-modification peptide shown in the aforementioned formula (i).

In the complex formation step, the chelating agent is chelated with a metal radionuclide (complex formation). The metal radionuclide used here is preferably used in a manner permitting ionization, more preferably in the form of an ion, from the viewpoint of increasing the complex formation efficiency. In the complex forming step, the order of addition of the metal radionuclide to the chelating agent does not matter as long as a complex can be formed with the metal radionuclide. For example, a solution in which radioactive metal ions are dissolved in a solvent mainly composed of water can be used as a radionuclide.

After complex formation, the obtained complex may be purified using a filtration filter, a membrane filter, a column filled with various fillers, chromatography or the like.

In the production method of the radioconjugate of the present invention, a conjugation step is preferably performed after the complex formation step.

In a more preferred embodiment, in complex formation step (A), a complex is formed between a metal radionuclide and a chelating agent having a first atomic group capable of click reaction as a substituent for enabling conjugate formation with the antibody. Then, in conjugation step (B), using an antibody-modification peptide shown by the aforementioned formula (i) and an antibody-modification linker ($L_1$) having a second atomic group capable of click reaction, a click reaction is performed between the peptide-modification antibody in which Fc region is site-specifically modified and the chelating agent with a formed complex which is obtained in step (A) to obtain the radioconjugate of the present invention.

The steps (A) and (B) are described in detail below.

As the combination of the first atomic group and the second atomic group capable of click reaction, an appropriate combination is selected according to the type of the click reaction. For example, a combination of alkyne and azide, a combination of 1,2,4,5-tetrazine and alkene, and the like can be mentioned. In these atomic groups, the first atomic group has one of the above-mentioned atomic group combination, and the second atomic group has one atomic group which is different from the first atomic group of the above-mentioned atomic group combination. To achieve both the stability of the chelating agent and the antibody and the improvement of the binding efficiency thereof, the chelate linker ($L_2$) is preferably alkyne and the antibody-modification linker ($L_1$) is preferably azide, or the chelate linker ($L_2$) is preferably 1,2,4,5-tetrazine and the antibody-modification linker ($L_1$) is preferably alkene. Specific examples of the click reaction

by such combinations of atomic groups include a Husgen cyclization addition reaction, an inverse electron-requested Diels-Alder reaction, and the like.

Specific examples of the combination of the atomic groups capable of click reaction include, as shown in the following formulas, a combination of an atomic group containing dibenzylcyclooctyne (DBCO) as alkyne of the first atomic group (the formula (1a)) and an atomic group containing an azide group as azide of the second atomic group (the formula (2a)), or else a combination of an atomic group containing 1,2,4,5-tetrazine as the first atomic group (the formula (1b)) and an atomic group containing trans-cyclooctene (TCO) as alkene of the second atomic group (the formula (2b)). Preferred is the combination of the formula (1a) and the formula (2a).

(1a) 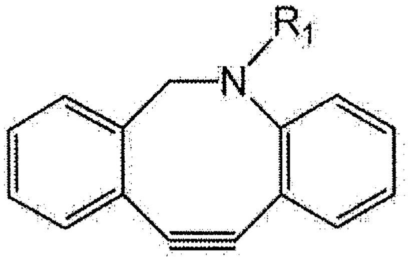

Dibenzylcyclooctyne

(2a) 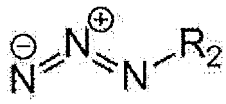

Azide

In the formula (1a), $R_1$ is a linkage site with a chelating agent, and in the formula (2a), $R_2$ is a linkage site with an antibody-modification peptide.

(1b) 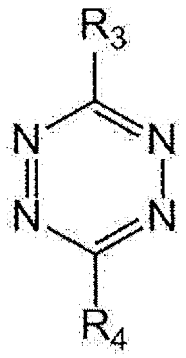

1,2,4,5-tetrazine

(2b) 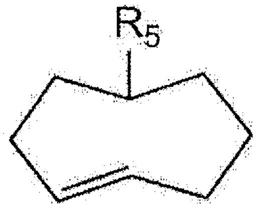

*trans*-cyclooctene

In the formula (1b), one of $R_3$ and $R_4$ is a linkage site with a chelating agent or an antibody-modification peptide, and the other is a hydrogen atom, a methyl group, a phenyl group or a pyridyl group. When the atomic group in the formula (2b) is linked to the chelating agent, $R_5$ is a linkage site to an antibody-modification peptide and when the atomic group in the formula (2b) is linked to the antibody-modification peptide, $R_5$ is a linkage site to the chelating agent.

When an atomic group containing dibenzylcyclooctyne (DBCO) represented by the above-mentioned formula (1a) as alkyne of the first atomic group is used, various commercially available DBCO reagents can be mentioned. Specifically, for example, DBCO-C6-Acid, Dibenzylcyclooctyne-Amine, Dibenzylcyclooctyne Maleimide, DBCO-PEG acid, DBCO-PEG-Alcohol, DBCO-PEG-amine, DBCO-PEG-NH-Boc, Carboxyrhodamine-PEG-DBCO, Sulforhodamine-PEG-DBCO, TAMRA-PEG-DBCO, DBCO-PEG-Biotin, DBCO-PEG-DBCO, DBCO-PEG-Maleimide, TCO-PEG-DBCO, DBCO-mPEG and the like can be selected, and Dibenzylcyclooctyne Maleimide is preferably used.

In complex formation step (A), more preferably, a compound having a structure represented by the following formula (ii) is used.

A-B-C ··· (ii)

In the formula (ii), A is the aforementioned chelating agent, and the generic term of B and C is a chelate linker ($L_2$)

In the formula (ii), B is represented by the following formula (iib).

(iib) 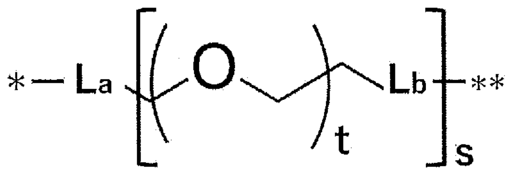

In the formula (iib), La and Lb are each independently a bond linker containing at least an amide bond and not less than 1 and not more than 50 carbon atoms, t is an integer of not less than 0 and not more than 30, s is 0 or 1, * is a binding site with A, and ** is a binding site with C.

In the formula (ii), C is either an alkyne derivative represented by the following formula (iic) or a tetrazine derivative represented by the formula (iid).

(iic) 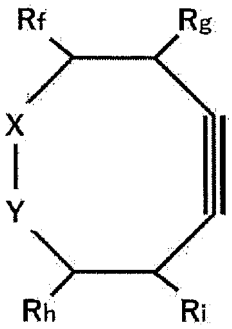

(iid) 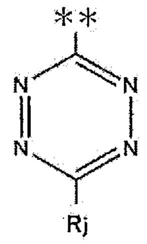

In the formula (iic), X is CHRk-** or N-**, Y is CHRk or C=O, Rk is independently a hydrogen atom or an alkyl group having not less than 1 and not more than 5 carbon atoms, when X is CHRk-** and Y is CHRk, then Rk moieties may be joined to form a cycloalkyl group, Rf, Rg, Rh and Ri are each independently a hydrogen atom, a halogen atom, or an alkyl group having not less than 1 and not more than 5 carbon atoms, Rf and Rg may be joined, or Rh and Ri may be joined to form a hydrocarbon ring, ** is a binding site with B, in the formula (iid), ** is a binding site with B, and Rj is a hydrogen atom, a methyl group, a phenyl group or a pyridyl group.

As the chelating agent used in step (A), a DOTA derivative of the above-mentioned formula (A) wherein $R_{11}$ to $R_{14}$ are $-(CH_2)_pCOOH$, p is 1, $R_{15}$ is a binding site with B; or DO3A derivative or DOTAGA derivative wherein $R_{11}$ to $R_{14}$ are - $(CH_2)_pCOOH$, p is 1, $R_{12}$ is a binding site (*) with B, and $R_{15}$ is a hydrogen atom is more preferred.

In the formula (ii), a DOSA-PEGt-DBCO wherein A is the above-mentioned DO3A, in B, La is a bond linker containing an amide bond and having not less than 1 and not more than 50 carbon atoms, s is 0 or 1, when s is 1, t is an integer of not less than 0 and not more than 30, Lb is a bond linker containing an amide bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is preferred.

In the formula (ii), a DOTAGA-PEGt-DBCO derivative wherein A is the above-mentioned DOTAGA derivative, in B, La is a bond linker containing an amide bond and having not less than 1 and not more than 50 carbon atoms, s is 0 or 1, when s is 1, t is an integer of not less than 0 and not more than 30, Lb is a bond linker containing an amide bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is preferred. More preferred is the following DOTAGA-DBCO.

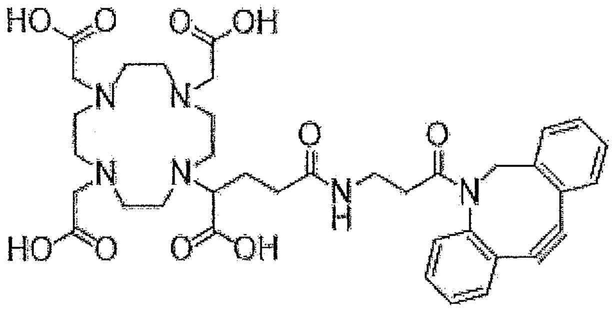

DOTAGA-DBCO

In the molar ratio of the chelating agent and metal radionuclide as chelate site/metal radionuclide, the lower limit is preferably not less than 10/1, more preferably not less than 100/1, further preferably not less than 500/1, and the upper limit is preferably not more than 10000/1, more preferably not more than 8000/1, further preferably not more than 7000/1. For example, the range of not less than 100/1 and not more than 7000/1 is preferred, and not less than 500/1 and not more than 7000/1 is more preferred.

The complex formation reaction is preferably performed in a solvent. As the solvent, water, saline, buffers such as sodium acetate buffer, ammonium acetate buffer, phosphate buffer, phosphate buffered saline, tris hydroxymethylaminomethane buffer (Tris buffer), 4-(2-hydroxyethyl)-1-piperazineethanesulfonic acid buffer (HEPES buffer), tetramethylammonium acetate buffer and the like, and the like can be used.

While the amount of the solvent is not particularly limited, from the aspect of practicality in the production step, the lower limit at the start of step (A) is not less than 0.01 mL, preferably not less than 0.1 mL, more preferably not less than 1.0 mL, further preferably not less than 10 mL, further more preferably not less than 100 mL, and upper limit is preferably not more than 1000 mL, more preferably not more than 100 mL, further preferably not more than 10 mL, further more preferably not more than 1.0 mL. For example, it is within the range of not less than 0.01 mL and not more than 100 mL.

As the concentration of the chelating agent in the reaction mixture of the complex formation reaction, from the aspect of the yield of the desired chelating agent, the lower limit at the start of step (A) is each independently preferably not less than 0.001 μmol/L, more preferably not less than 0.01 μmol/L, further preferably not less than 0.1 μmol/L, more preferably not less than 1 μmol/L, and the upper limit is preferably not more than 1000 μmol/L, more preferably not more than 100 μmol/L, further preferably not more than 10 μmol/L. For example, it is within the range of not less than 1 μmol/L and not more than 100 μmol/L.

The temperature of the complex formation reaction may be, for example, room temperature (25°C) or under heating conditions. To simultaneously achieve suppression of decomposition of the chelating agent and improvement of complex formation efficiency, the lower limit is preferably not less than 20°C, more preferably not less than 30°C, further preferably not less than 35°C, further more preferably not less than 37°C, particularly preferably not less than 45°C. The upper limit is preferably not more than 150°C, more preferably not more than 120°C, further preferably not more than 100°C, further more preferably not more than 90°C. For example, a range of not less than 30°C and not more than 100°C is preferred, and a range of not less than 35°C and not more than 90°C is more preferred.

The antibody to be used in step (B) is a peptide-modified antibody in which Fc region (constant region) of anti-EGFR antibody as described in detail in the above-mentioned "(1-2) Antibody" is site-specifically modified using the antibody-modification peptide shown in the aforementioned formula (i), and an antibody-modification linker ($L_2$) having the second atomic group capable of click reaction.

The antibody-modification peptide can be produced using a combination of amino acids regardless of natural amino acids and unnatural amino acids, by subjecting to peptide synthesis methods such as liquid phase synthesis process, solid phase synthesis process, automatic peptide synthesis method, gene recombinant method, phage display method and the like. In the synthesis of the peptide, where necessary, the functional groups of the amino acids to be used may be protected. These methods can be performed according to the method described in, for example, WO 2017/217347 and WO 2018/230257.

The antibody-modification linker ($L_2$) may be one in which an antibody-modification peptide and a linker ($L_2$) represented by the following formula (S1) are bonded.

$$*-((L_i)_m-Z)_k-L_{ii}-AG2 \cdots \qquad (S1)$$

wherein * is a binding site with the N-terminal or C-terminal of peptide,

$L_i$ is a linker moiety of polyethylene glycol (PEG),
m is an integer of not less than 1 and not more than 50,
Z is a second linker moiety that binds $(L_i)_m$ and $L_{ii}$,
k is 0 or 1,
$L_{ii}$ is the second PEG linker moiety, and
AG2 is a second atomic group.

In the aforementioned formula (S1), the structure of Z is not particularly limited as long as it is a linker structure that binds $(L_i)_m$ and $L_{ii}$ to each other, and includes, for example, an amino acid sequence consisting of not less than 1 and not more than 5 amino acid residues. In this case, the amino acid sequence contained in Z preferably contains a cysteine residue, and is more preferably bonded to L2 via a thioether group formed by the bond between the thiol group of the cysteine residue and a maleimide group.

In the present invention, the polyethylene glycol (PEG) linker moiety constituting $L_{ii}$ preferably has the structure shown by the following formula (P2). In the formula (P2), n is an integer of preferably not less than 1 and not more than 50, more preferably not less than 1 and not more than 20, further preferably not less than 2 and not more than 10, further more preferably not less than 2 and not more than 6.

(P2) 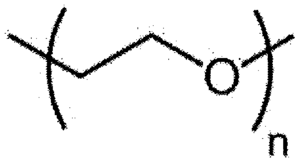

One end of the structure of the PEG linker moiety may be modified by a structure derived from a commercially available PEGylation reagent or a structure derived from a reagent generally used for PEGylation. Although not particularly limited, examples thereof include structures derived from diglycolic acid or a derivative thereof, and maleimide or a derivative thereof.

As a method for introducing the aforementioned second atomic group into an antibody-modification linker ($L_2$), an introduction method including obtaining an antibody-modification peptide having a desired amino acid sequence by the aforementioned method, dissolving the peptide in a solution containing a solubilizing agent and a reducing agent and, where necessary, an acid, adding an organic solvent solution of an atomic group containing an azide group or trans-cyclooctene (TCO) as the second atomic group to the solution, and stirring the mixture at room temperature can be mentioned.

When an atomic group containing an azide group is introduced as the second atomic group, the azide group is directly introduced into the N-terminal or C-terminal of a peptide by using a commercially available azide group-introducing reagent according to a conventional method, or an atomic group containing an azide group can be introduced via the aforementioned linker structure. Examples of the azide group-introducing reagent to be used include silyl azide, azide phosphate, alkyl ammonium azide, inorganic azide, sulfonyl azide, PEG azide, and the like.

When an atomic group containing TCO is introduced as the second atomic group, TCO is directly introduced into the N-terminal or C-terminal of a peptide by using a commercially available click chemistry reagent containing TCO according to a conventional method, or an atomic group containing TCO can be introduced via the aforementioned linker structure.

The method for binding an antibody-modification peptide to an anti-EGFR antibody to obtain a peptide-modified antibody can be performed, for example, by dispersing the aforementioned antibody-modification peptide, an anti-EGFR antibody, a crosslinking agent, and a catalyst as necessary in an appropriate buffer, according to the description of WO 2017/217347. As the crosslinking agent, those mentioned above can be used. In addition, when binding the antibody-modification peptide and the anti-EGFR antibody, where necessary, buffer replacement including treating the solution containing the anti-EGFR antibody with an ultrafiltration filter, etc., and dispersing same in a buffer may be performed once or twice before binding with the antibody-modification peptide.

In one embodiment, the present disclosure relates to a method for producing a conjugate of an antibody-modification peptide and an anti-EGFR antibody, comprising a step of mixing an antibody-modification peptide modified with a crosslinking agent and an anti-EGFR antibody. By this step, a crosslinking reaction can occur between the antibody-modification peptide modified with the crosslinking agent and the anti-EGFR antibody. For example, in cetuximab, the crosslinking reaction may occur site-specifically between the above-mentioned Xaa2 amino acid residue of the antibody-modification peptide and Lys248 residue or Lys250 residue, preferably Lys250 residue, according to Eu numbering in human IgG Fc. For example, in panitumumab, it may occur site-specifically between the above-mentioned Xaa2 amino acid residue of the antibody-modification peptide and Lys244 or Lys246 residue, preferably Lys246 residue, according

to Eu numbering in human IgG Fc.

The conditions of the mixing step are not particularly limited as long as a crosslinking reaction occurs between the antibody-modification peptide and the anti-EGFR antibody. For example, the reaction can be performed by mixing an antibody-modification peptide and an anti-EGFR antibody in an appropriate buffer at room temperature (e.g., about 15°C to 30°C). The mixing step may be performed by adding as necessary an appropriate amount of a catalyst that promotes the crosslinking reaction.

In one embodiment, a solvent containing at least water is added to dissolve the anti-EGFR antibody. The solvent other than water includes, for example, dimethyl sulfoxide, acetonitrile, saline, and buffers such as sodium acetate buffer, ammonium acetate buffer, phosphate buffer, phosphate buffered saline, Tris buffer, HEPES buffer, tetramethylammonium acetate buffer, histidine buffer, and the like. When a buffer is used, the pH at 25°C is preferably set to 4.0 or more and 10.0 or less, more preferably 5.5 or more and 8.5 or less, from the aspect of the stability of the antibody. At the start of the crosslinking reaction, the concentration of the antibody is preferably set to 1.0 μmol/L or more as the lower limit and 1000 μmol/L or less, more preferably 500 μmol/L or less, as the upper limit.

Then, an antibody-modification peptide modified with a crosslinking agent and, where necessary, a catalyst are added and the mixture is dispersed at 10°C or higher and 30°C or lower.

The mixing ratio of the antibody-modification peptide and the anti-EGFR antibody in the mixing step is not particularly limited. The molar ratio of the antibody-modification peptide to the anti-EGFR antibody can be set to, for example, 1:1 to 20:1, preferably 2:1 to 20:1 or 5:1 to 10:1.

In a preferred embodiment, the molar ratio of the antibody-modification peptide to the anti-EGFR antibody in the above-mentioned mixing step can be 0.5 to 2.2, preferably 0.8 to 1.8. In this way, an antibody in which one molecule of antibody-modification peptide is bound to one molecule of anti-EGFR antibody (hereinafter referred to as "monovalent antibody") can be obtained efficiently.

The mixing time (reaction time) in the mixing step is not particularly limited as long as a crosslinking reaction occurs between the antibody-modification peptide and the anti-EGFR antibody. It is, for example, 1 min to 5 hr, preferably 10 min to 2 hr.

The peptide-modification antibody obtained through the above steps is a mixture containing an antibody in which one molecule of antibody-modification peptide is bound to one molecule of anti-EGFR antibody (i.e., monovalent antibody) and an antibody in which two molecules of antibody-modification peptide are bound to one molecule of anti-EGFR antibody (hereinafter referred to as "divalent antibody") at any ratio. This may be used as it is for the subsequent steps, or an unmodified antibody, a monovalent antibody, and a divalent antibody are separated and purified by a method such as filtration filter, membrane filter, column filled with various fillers, various chromatographies and the like, and only the antibody having any valence may be subjected to the subsequent steps. When the unmodified antibody cannot be separated from the antibody having other valence as a result of purification, a mixture containing these may be subjected to the subsequent steps.

When an unmodified antibody, a monovalent antibody, and a divalent antibody are separated and purified, any of the above-mentioned purification methods may be used for separation and purification. It is possible to use a column packed with various fillers. For example, a column packed with a filler in which a protein such as protein A, protein G, or the aforementioned antibody-modification peptide is bound to a carrier can be used. The shape of the carrier of the filler packed in such a column includes gel (e.g., column gel), particle, bead, nanoparticle, microparticle, macrobead, and the like. The materials of the carrier include magnetic substance, latex, agarose, glass, cellulose, sepharose, nitrocellulose, polystyrene, and other polymeric materials. Specific example is an IgG-BP column in which the aforementioned antibody-modification peptide is bound to a column gel (see WO 2021/080008).

An IgG-BP column is a column in which an IgG-binding peptide is immobilized. Divalent antibody cannot bind to the column because the binding sites are already occupied by IgG-binding peptides, and only monovalent antibodies show affinity for the column. Using the IgG-BP column and utilizing the difference in the interaction with respective antibody-modification peptides, the first antibody composition containing relatively large amounts of the unmodified antibody and the monovalent antibody and the second antibody composition containing a relatively large amount of the divalent antibody can be respectively separated and purified. In one preferred embodiment, the molar ratio of unmodified antibody to monovalent antibody in the first antibody composition is 4-47:53-96, preferably 4-30:70-96, more preferably 4-20:80-96, further preferably 4-10:90-96.

The first antibody composition or the second antibody composition separated and purified in this manner may be used as it is for the click reaction in the subsequent step (B), or may be used for the click reaction in step (B) after adjusting the protein concentration of the peptide-modified antibody contained.

The click reaction in step (B) is performed between the first atomic group capable of click reaction which is contained in the chelating agent, and the second atomic group capable of click reaction which is contained in the peptide-modified antibody. By such click reaction, a binding group (substituent capable of conjugating with antibody) that links a chelating agent and an antibody is formed.

When the peptide-modified antibody and the complex obtained in step (A) are capable of click reaction, the

order of addition of these does not matter. For example, one of the complex and the peptide-modified antibody is added to a reaction container containing a solvent, and then the other is added to perform the reaction, or one of the chelating agent and the antibody is dispersed in a solvent and the other is added to the dispersion to perform the reaction. Alternatively, these may be simultaneously added to a reaction container containing a solvent to perform the reaction.

As the solvent to be used for the click reaction in step (B), a solvent containing water can be used. For example, water, saline, buffers such as sodium acetate buffer, ammonium acetate buffer, phosphate buffer, phosphate buffered saline, Tris buffer, HEPES buffer, tetramethylammonium acetate buffer and the like, and the like can be used. When a buffer is used, to simultaneously achieve the stability of the complex and the antibody, and the bond efficiency of these, the pH at 25°C is preferably set to not less than 4.0 and not more than 10.0, further preferably not less than 5.5 and not more than 8.5.

While the amount of the reaction mixture is not particularly limited, from the aspect of practicality in the production step, the lower limit at the start of step (B) is preferably not less than 0.001 mL, more preferably not less than 0.01 mL, further preferably not less than 0.1 mL, further more preferably not less than 1 mL, and the upper limit is preferably not more than 1000 mL, more preferably not more than 100 mL, further preferably not more than 10 mL, further more preferably not more than 1 mL. For example, the range of not less than 0.001 mL and not more than 1000 mL is preferable, and the range of not less than 0.1 mL and not more than 10 mL is more preferable.

As the concentrations of the chelating agent and the antibody in the reaction mixture, each independently, the lower limit at the start of step (B) is preferably not less than 0.001 μmol/L, more preferably not less than 0.01 μmol/L, further preferably not less than 0.1 μmol/L, further more preferably not less than 1.0 μmol/L, and the upper limit is preferably not more than 1000 μmol/L, more preferably not more than 100 μmol/L. For example, the range of not less than 0.1 μmol/L and not more than 1000 μmol/L is preferable, and the range of not less than 1 μmol/L and not more than 100 μmol/L is more preferable, from the aspect of the yield of the desired conjugate.

To prevent unintended denaturation of the antibody and increase the reaction efficiency, the upper limit of the reaction temperature of the click reaction in step (B) is preferably not more than 50°C, more preferably not more than 40°C. The lower limit of the reaction temperature is not particularly limited as long as the reaction proceeds, and is preferably not less than 15°C. The reaction time of the click reaction is, on the condition that it is the aforementioned reaction temperature, preferably not less than 5 min, more preferably not less than 10 min, preferably not more than 24 hr, more preferably not more than 20 hr. For example, the range of not less than 5 min and not more than 24 hr is preferable, and the range of not less than 10 min and not more than 20 hr is more preferable.

The obtained conjugate may be used as it is or purified using a filtration filter, a membrane filter, a column filled with various fillers, chromatography or the like.

In the conjugate produced by steps (A) and (B), the lysine residue in the Fc region of anti-EGFR antibody is specifically modified with a chelating agent. This conjugate comprises one or two molecules of the aforementioned chelating agent per one molecule of the antibody. The chelating agent site-specifically modifies the Fc region of the antibody of the present invention via a linker (L). The linker (L) is constituted of a chelate linker ($L_2$) that connects to a chelating agent, a first atomic group that connects to the linker ($L_2$), a second atomic group that can perform click reaction with the first atomic group, and an antibody-modification linker ($L_1$) that connects to the second atomic group (including an antibody-modification peptide represented by the above-mentioned formula (i)). Therefore, the linker (L) has a chemical structure derived from the first atomic group and the second atomic group. As such chemical structure, a triazole skeleton-containing structure represented by the aforementioned formula (10a) or (10b) or a pyridazine skeleton-containing structure represented by the aforementioned formula (10c) can be considered. Since the formula (10a) and the formula (10b) are isomers, they may be contained at any ratio.

(1-7) Radiopharmaceutical (Radiopharmaceutical (1))

A radiopharmaceutical refers to a composition that contains the radioconjugate of the present invention and is in a form suitable for in vivo administration to a subject. The radiopharmaceutical may be, for example, a radioconjugate produced by the method shown in the aforementioned (1-6) as it is, or can be produced by purifying same and dissolving same in a solvent mainly containing water and isotonic with the living body. In this case, the radiopharmaceutical is preferably in the form of an aqueous solution, and may contain other pharmaceutically acceptable components as necessary. An effective amount of the radiopharmaceutical is orally or parenterally, for example, intravenously, subcutaneously, intraperitoneally, intramuscularly, or the like, administered to a living body, and is used for treatment of cancer, diagnosis of cancer, detection of a lesion, or the like.

As used herein, the subject of administration is a human, or an animal such as mouse, rat, monkey, guinea pig, chimpanzee, sheep, goat, dog, cat, swine, bovine, horse or the like, but is not particularly limited. Preferred is a human.

A preferred target disease is cancer that overexpresses EGFR. The type of EGFR-overexpressing cancer to be treated, diagnosed or detected in the present invention is not particularly limited as long as it overexpresses EGFR. Examples include colorectal cancer (particularly unresectable advanced/recurrent cancer with wild-type RAS gene), and

head and neck cancer. The EGFR-overexpressing cancer may also be cancer of any stage, and may be localized or metastatic, or primary or recurrent. As used herein, the "overexpression" refers to a state in which, when measured by a known test method, significant amplification of the EGFR gene in tumor tissue compared to non-tumor tissue, or significant enhancement of EGFR protein expression compared to non-tumor tissue is observed.

As used herein, the "effective amount" is an amount that can afford useful diagnostic or therapeutic effects in a subject of administration. The effective amount to be administered to a subject varies depending on the type of subject, body weight of the subject, dosage form (tablet, injection, etc.) and route (oral administration, parenteral administration, etc.) of administration, severity of disease (e.g., cancer), and the like. Physicians and veterinarians can consider these factors and determine the appropriate effective amount.

The radiopharmaceutical of the present invention when stored at room temperature has a radiochemical purity of a certain level or above at the time of expiry of a period that is a multiple of not less than 1 and not more than 5 of the half-life, based on the half-life of the metal radionuclide constituting the radiopharmaceutical. When the above-mentioned metal radionuclide is a β-ray nuclide (e.g., Lu-177 or Y-90), the radiochemical purity of the conjugate is preferably not less than 90%, more preferably not less than 93%, when stored at room temperature for 7 days after production. When the metal radionuclide is an α-ray nuclide (e.g., Ac-225), the radiochemical purity of the conjugate after storage for 14 days at room temperature from the completion of the production is preferably not less than 90%, more preferably not less than 94%. The "room temperature" in the present specification preferably refers to the "ordinary temperature" defined in the Japanese Pharmacopoeia, which is specifically 15 to 25°C.

As used herein, the radiochemical purity refers to the percentage of the peak radioactivity (count) corresponding to the conjugate with respect to the total radioactivity (count) detected when the sample is analyzed with a commercially available radiation detector. High performance liquid chromatography and thin-layer chromatography can be used for the analysis of radiochemical purity, and thin-layer chromatography is preferably used. More preferably, thin-layer chromatography is used under the conditions described in the below-mentioned Examples.

As described above, the radiopharmaceutical of the present invention is preferably in the form of an aqueous solution. It is more preferably in the form of a buffer from the aspect of maintaining the radiochemical purity as described above. As the buffer, any buffer used in an antibody drug containing an anti-EGFR antibody or ADC of an anti-EGFR antibody as an active ingredient can be used. As a nonlimiting example, citrate buffer or acetate buffer can be used. The citrate buffer is composed of citric acid and a salt thereof, and can be composed of, for example, citric acid and a sodium salt thereof. The acetate buffer is composed of acetic acid and a salt thereof and can be composed of, for example, acetic acid and a sodium salt thereof. The radiopharmaceutical of the present invention may contain any amino acid such as glycine, and may also contain a solubilizer such as polysorbate 80.

The radiopharmaceutical of the present invention can be used for radionuclide therapy of cancer by selecting metal radionuclides that have a therapeutic effect, specifically radionuclide that emits α-ray or nuclide that emits β rays (preferably Ac-225, γ-90, Lu-177, more preferably Ac-225). In this radionuclide therapy, the radiopharmaceutical of the present invention is administered by intravenous injection or orally to cause accumulation of the radioconjugate of the present invention in a lesion site such as a cancer primary lesion or metastatic lesion, and cancer cells at the lesion site are destroyed by the radiation emitted from the metal radionuclide. The amount to be administered and dose of the radiopharmaceutical of the present invention is appropriately determined by the efficacy of the active ingredient, the mode and route of administration, the stage of cancer progression, the body type, body weight and age of the patient, and the type and amount of the therapeutic drug used in combination for other diseases.

In addition, by selecting a radionuclide that emits positrons or a radionuclide that emits γ rays (preferably, Ga-68, Zr-89, In-111, more preferably Zr-89) as the metal radionuclide, it can be used for cancer diagnosis or lesion detection. A radiopharmaceutical using radionuclide that emits positrons can be preferably used for PET (Positron Emission Tomography) examination, and a radiopharmaceutical using radionuclide that emits γ-rays can be preferably used for SPECT (Single Photon Emission Computed Tomography) examination. This may also be used in combination with cancer diagnosis or lesion detection in the aforementioned radionuclide therapy of cancer. The diagnostic radiopharmaceutical for cancer of the present invention may be used for diagnosis before performing radionuclide therapy for cancer, or may be used for diagnosis after performing radionuclide therapy for cancer. Using the radiopharmaceutical for diagnosis before conducting a radionuclide therapy for cancer, selection of treatment can be performed based on whether or not to perform radionuclide therapy for cancer using the radiopharmaceutical of the present invention provided with a metal nuclide that emits α-ray. Using the radiopharmaceutical for diagnosis after conducting a radionuclide therapy for cancer, moreover, whether or not radionuclide therapy for cancer using the radiopharmaceutical of the present invention is effective can be judged, and a treatment plan including an increase or a decrease of dose and the like can be optimized.

(2) Radiopharmaceutical (Radiopharmaceutical (2))

Another embodiment of the present invention is a radiopharmaceutical containing a conjugate of a chelating

agent chelated with a metal radionuclide and an anti-EGFR antibody as an active ingredient, wherein the linkage between the anti-EGFR antibody and the chelating agent does not contain a thiourea bond, when stored at room temperature, it has a radiochemical purity of a certain level or above at the time of expiry of a period that is a multiple of not less than 1 and not more than 5 of the half-life, based on the half-life of the metal radionuclide constituting the radiopharmaceutical. When the above-mentioned metal radionuclide is a β-ray nuclide (e.g., Lu-177 or Y-90), the radiochemical purity of the aforementioned conjugate is preferably not less than 90%, more preferably not less than 93%, when stored at room temperature for 7 days after production. Also, when the metal radionuclide is an α-ray nuclide (e.g., Ac-225), the radiochemical purity of the conjugate is preferably not less than 90%, more preferably not less than 94%, when stored at room temperature for 14 days after production. The room temperature is as defined in the aforementioned radiopharmaceutical (1).

In the radiopharmaceutical (2), the following methods (a) to (d) can also be used in conjugating the chelating agent and the anti-EGFR antibody, in addition to the site-specific modification method using a peptide. Since other part is the same as in the radiopharmaceutical (1), the explanation is omitted.

(a) method for modifying a sulfhydryl (SH) group generated by partially reducing a disulfide bond (SS bond) between polypeptide chains at the hinge site of an antibody with a chelating agent or linker (L) having a maleimide group reactive with the SH group

(b) method for modifying cysteine newly introduced into an antibody by an amino acid mutation by genetic engineering with a chelating agent or linker (L) having a maleimide group

(c) method for modifying an azide group of azidized lysine newly introduced into an antibody by an amino acid mutation by genetic engineering with a chelating agent or linker (L) having alkyne (e.g., Dibenzocyclooctyne: DBCO) by using a click reaction

(d) method for modifying glutamine introduced into a specific position of an antibody with a chelating agent or linker (L) having a side chain of lysine by using transglutaminase

In the present invention, a peptide that site-specifically modifies an anti-EGFR antibody and a chelating agent are linked without using a thiourea bond. Therefore, a radioconjugate and a radiopharmaceutical that are stable even at room temperature can be obtained. Since the site-specific modification of antibody can contain monovalent antibody or divalent antibody or both of these in any proportion, unlike random modification, radioconjugate and radiopharmaceutical with stable quality can be obtained. In addition, the radioconjugate of the present invention maintains efficacy equivalent to conventional one. Therefore, according to the present invention, an anti-EGFR antibody conjugate and a radiopharmaceutical thereof with higher quality while maintaining efficacy can be provided.

The following embodiments are also encompassed in the technical idea of the present invention.

[1] A conjugate of an anti-EGFR antibody site-specifically modified with a peptide and a chelating agent, wherein the aforementioned chelating agent is chelated with a metal radionuclide, the aforementioned peptide and the chelating agent are linked by a linker (L), and the aforementioned linker (L) does not contain a thiourea bond.

[2] The conjugate of [1], wherein the aforementioned chelating agent is DOTAGA (α-(2-Carboxyethyl)-1,4,7,10-tetraazacyclododecane-1,4,7,10-tetraacetic acid).

[3] The conjugate of [1] or [2], wherein the aforementioned peptide is an amino acid sequence consisting of not less than 13 and not more than 17 amino acid residues and is represented by the following formula (i):

$$(Xa)\text{-}Xaa1\text{-}(Xb)\text{-}Xaa2\text{-}(Xc)\text{-}Xaa3\text{-}(Xd) \cdots \quad (i)$$

in the formula (i), Xa, Xb, Xc and Xd are continuous X in the number of a, continuous X in the number of b, continuous X in the number of c, and continuous X in the number of d, respectively,

X is an amino acid residue having neither a thiol group nor a haloacetyl group in the side chain,

a, b, c and d are each independently an integer of not less than one and not more than 5, and satisfy $a+b+c+d \leq 14$,

Xaa1 and Xaa3 are each independently an amino acid residue derived from an amino acid having a thiol group in the side chain, or an amino acid residue derived from an amino acid having a haloacetyl group in the side chain, provided that one of Xaa1 and Xaa3 is an amino acid residue derived from an amino acid having a thiol group in the side chain,

Xaa1 and Xaa3 are connected to form a ring structure, and

Xaa2 is a lysine residue, an arginine residue, a cysteine residue, an aspartic acid residue, a glutamic acid residue, 2-aminosuberic acid, or diamino propionic acid, and modified with a crosslinking agent.

[4] The conjugate of any one of [1] to [3], wherein the aforementioned metal radionuclide is Ac-225, γ-90, Lu-177,

or Zr-89.

[5] The conjugate of any one of [1] to [4], wherein the aforementioned linker (L) comprises the formula (10a), the formula (10b), or the formula (10c):

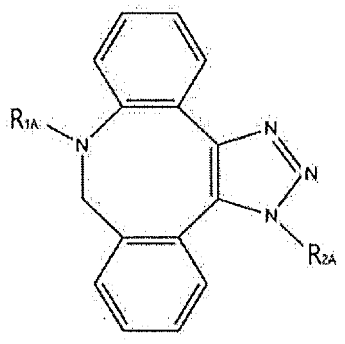

(10a)

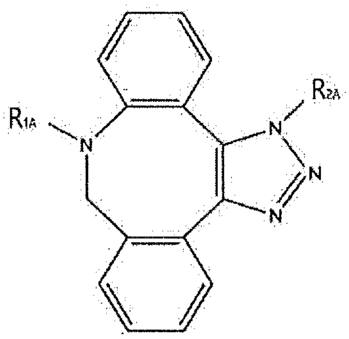

(10b)

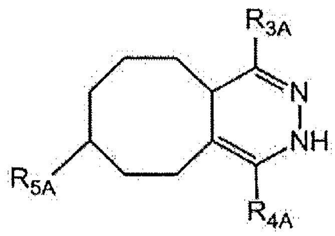

(10c)

In the formula (10a) and the formula (10b), $R_{1A}$ is a binding site with a chelating agent, and $R_{2A}$ is a binding site with the aforementioned peptide. In the formula (10c), one of $R_{3A}$ and $R_{4A}$ is a hydrogen atom, a methyl group, a phenyl group or a pyridyl group, and the other is a binding site with the aforementioned chelating agent, and $R_{5A}$ is a binding site with the aforementioned peptide.

[6] The conjugate of [5], comprising a polyethylene glycol group between the linkage site with the aforementioned peptide and the aforementioned peptide.

[7] The conjugate of any one of [1] to [6], which is conjugated by a click reaction of an anti-EGFR antibody site-specifically modified with the aforementioned peptide having an azide group introduced into the N-terminal, and a radioactive metal complex of DOTAGA-DBCO represented by the following formula:

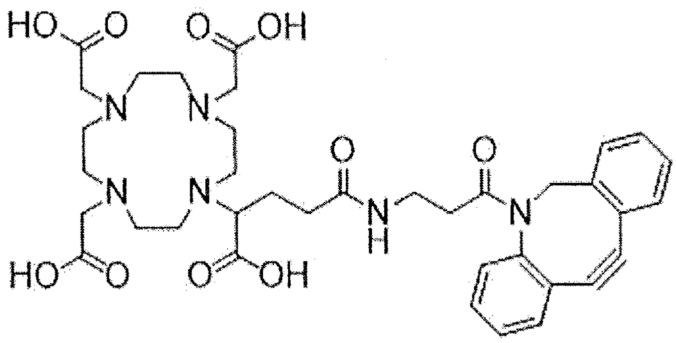

DOTAGA-DBCO

[8] The conjugate of any one of [1] to [7], wherein the aforementioned anti-EGFR antibody is cetuximab.

[9] The conjugate of any one of [1] to [7], wherein the aforementioned anti-EGFR antibody is panitumumab.

[10] A radiopharmaceutical comprising the conjugate of any one of [1] to [9] as an active ingredient.

[11] The radiopharmaceutical of [10], which is used in a radionuclide therapy for cancer.

[12] The radiopharmaceutical of [10], which is used in cancer diagnosis.

[13] The radiopharmaceutical of [12], which is used in combination with the radionuclide therapy for cancer using a radiopharmaceutical in [11].

[14] A radiopharmaceutical containing a conjugate of a chelating agent chelated with a metal radionuclide and an anti-EGFR antibody as an active ingredient, wherein the linkage between the anti-EGFR antibody and the chelating agent does not contain a thiourea bond, and the conjugate has a radiochemical purity of not less than 90% when stored at room temperature for 7 days.

[15] The radiopharmaceutical of [14], wherein the aforementioned conjugate is any one of [1] to [9].

[16] The radiopharmaceutical of [15], which is used in a radionuclide therapy for cancer.

[17] The radiopharmaceutical of [15], which is used in cancer diagnosis.

[18] The radiopharmaceutical of [17], which is used in combination with the radionuclide therapy for cancer using a

radiopharmaceutical in [16].

[19] A radiopharmaceutical containing a conjugate of a chelating agent chelated with a metal radionuclide and an anti-EGFR antibody as an active ingredient and satisfying the following condition (1) or (2), wherein the linkage between the anti-EGFR antibody and the chelating agent does not contain a thiourea bond:

(1) the aforementioned metal radionuclide is $^{177}$Lu or $^{90}$Y, and the aforementioned conjugate has a radiochemical purity of not less than 90% when stored at room temperature for 7 days

(2) the aforementioned metal radionuclide is $^{225}$Ac, and the aforementioned conjugate has a radiochemical purity of not less than 90% when stored at room temperature for 14 days.

[20] A radiopharmaceutical containing a conjugate of a chelating agent chelated with a metal radionuclide and an anti-EGFR antibody as an active ingredient, wherein the linkage between the anti-EGFR antibody and the chelating agent does not contain a thiourea bond, and the conjugate has a radiochemical purity of not less than 90% at the time of expiry of a period that is a multiple of not less than 1 and not more than 5 of the aforementioned half-life, based on the half-life of the aforementioned metal radionuclide.

[Example]

The present invention is described in more detail in the following by way of examples. However, the scope of the present invention is not limited by the examples. In the Tables below, the column with "-" indicates no performance.

[Example 1] Production of conjugate with cetuximab by using $^{225}$Ac-labeled DOTAGA-DBCO

(1. Antibody modification step)

A peptide containing 17 amino acid residues represented by the following formula (P3) (SEQ ID NO: 19) was obtained by the method described in WO 2017/217347. The amino acid sequence of this peptide was the same as the sequence in which Xaa2 of SEQ ID NO: (2) was a lysine residue, and the side chain terminal amino group of the lysine residue was modified with the structure shown by $R_1$. In addition, two cysteine residues form a disulfide bond with each other, and to the N-terminal of the peptide was added ethyl azide as an atomic group containing an azide group, which is the second atomic group, via a linker ($L_1$) structure having diglycolic acid and eight PEGs.

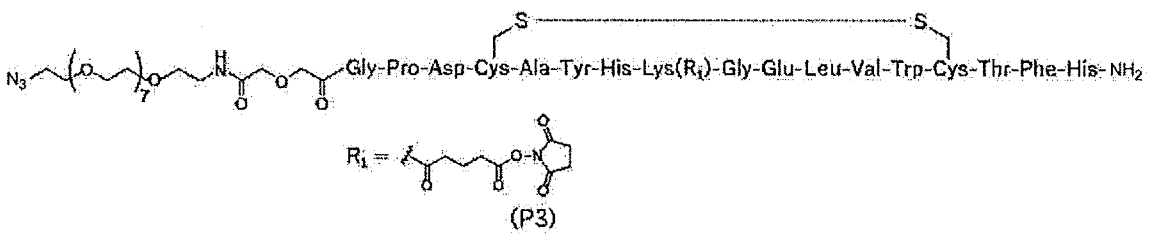

(P3)

In the formula (P3), Gly is glycine, Pro is proline, Asp is aspartic acid, Cys is cysteine, Ala is alanine, Tyr is tyrosine, His is histidine, Lys is lysine, Glu is glutamic acid, Leu is leucine, Val is valine, Trp is tryptophan, Thr is threonine, and Phe is phenylalanine.

A solution containing cetuximab (manufactured by Merck) was subjected to buffer replacement using an ultrafiltration filter (Amicon Ultra-15) added with 0.02 mol/L acetic acid-sodium acetate buffer (pH 6.0). This operation was repeated twice.

A mixture of the aforementioned peptide and cetuximab after buffer replacement in a 0.02 mol/L acetic acid·sodium acetate buffer (pH 6.0) was reacted at room temperature for 30 min to give a solution containing a peptide-modified antibody. The peptide-modified antibody has an Fc region of the antibody site-specifically modified by the above-mentioned peptide.

Then, the solution was then passed through the IgG-BP column to obtain the first antibody composition containing relatively large amounts of the unlabeled antibody and monovalent antibody. The concentration of the monovalent antibody contained in the recovered fraction was adjusted with 0.01 mol/L citrate buffer (pH 5.5) containing 0.1 mol/L sodium chloride and 0.1 mol/L glycine such that the concentration was 15 mg/mL. An obtained solution containing the first antibody composition was subjected to the below-mentioned labeling step.

(2. Complex formation step)

DOTAGA-DBCO represented by the following formula was produced based on the method described in Bernhard et al. DOTAGA-Anhydride: A Valuable Building Block for the Preparation of DOTA-Like Chelating Agents Chem. Eur. J. 2012, 18, 7834-7841. This chelating agent was dispersed in 0.1 mol/L sodium acetate buffer (pH 6.0) as a solvent to give a dispersion containing 1.7 mmol/L chelating agent. A reaction mixture of the dispersion (0.01 mL), 0.1 mol/L sodium acetate buffer (pH 6.0, 0.15 mL), and $^{225}$Ac ion-containing solution (0.2 mol/L aqueous hydrochloric acid solution, radioactivity concentration 358 MBq/mL, prepared from one produced by Oak Ridge National Laboratory, liquid amount 0.01 mL) about 3.6 MBq (calculated by attenuation from the level of radioactivity at test date and time) as a radioactive metal source was reacted under heating conditions to give a $^{225}$Ac complex solution. The molar ratio of the chelating agent and the radioactive metal ion was chelating agent:$^{225}$Ac ion = about 2290:1, and the heating temperature of the reaction mixture was set to 70°C, and the heating time was set to 30 min.

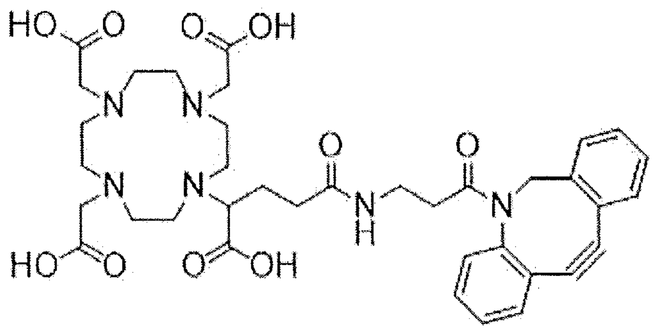

DOTAGA-DBCO

The radiochemical purity (RCP) of the obtained $^{225}$Ac complex was measured by the following method. That is, a part of the $^{225}$Ac complex solution was developed by thin layer chromatography (manufactured by Agilent, model number: SGI0001, eluent: acetonitrile/water mixed solution (volume ratio 1:1)), and then measured by radio γ-TLC Analyzer (manufactured by raytest, MODEL GITA Star). The percentage of the radioactivity (count) of the peak detected near the origin with respect to the detected total radioactivity (count) was defined as the RCP (%) of the $^{225}$Ac complex. As a result, the RCP of the $^{225}$Ac complex was 91%. The obtained $^{225}$Ac complex solution was directly used for the labeling step.

(3. Labeling step)

To a solution of the unpurified $^{225}$Ac complex obtained via the aforementioned step (2) was added a solution containing a peptide-modified antibody (monovalent antibody) obtained in the above-mentioned step (1), and a click reaction was performed at 37°C for 120 min to give $^{225}$Ac complex-labeled antibody. The amount of the $^{225}$Ac complex and the amount of the peptide-modified antibody (monovalent antibody) were 17 nmol and 20 nmol, respectively, and the molar ratio of the DBCO group and the azide group was about 1:1.2. The reaction rate (%) of the unpurified $^{225}$Ac complex-labeled antibody is shown in the following Table 1. Here, the reaction rate (%) means the RCP of the $^{225}$Ac complex-labeled antibody with respect to the labeling rate (%) in the complex formation step, and the labeling rate (%) means the proportion (%) of the radioactivity of the $^{225}$Ac complex with respect to the charged radioactivity level.

Furthermore, a solution of the $^{225}$Ac complex-labeled antibody obtained by reacting at 37°C for 2 hr was purified using ultrafiltration filter (manufactured by Merck, model number: UFC505096). The RCP and the radiochemical yield (RCY) of the $^{225}$Ac complex-labeled antibody after purification are shown in the following Table 1.

The measurement method of the RCP and RCY of the $^{225}$Ac complex-labeled antibody was as follows. That is, thin layer chromatography (manufactured by Agilent, model number: SGI0001, developing solvent was mixed solution of acetonitrile:0.1 mmol/L EDTA solution (volume ratio 1:1)) was measured by radio γ-TLC Analyzer (manufactured by ray test, MODEL GITA Star), and the percentage of the radioactivity (count) of the peak detected near the origin to the total radioactivity (count) detected was defined as the RCP (%). In addition, the percentage of the radioactivity (radioactivity level calculated from the count measured by γ ray spectrometer (Ge semiconductor detector: GMX10P4-70 (manufactured by ORTEC), Multi Channel Analyzer: M7-000 (manufactured by SEIKO EG&G), data processing: Spectrum Navigator:DS-P300 (manufactured by SEIKO EG&G) and Gamma Studio:DS-P600 (manufactured by SEIKO EG&G)) recovered after ultrafiltration purification with respect to the total radioactivity (similar to the above, the radioactivity level calculated from the count measured by γ ray spectrometer) added at the start of the labeling step was

defined as the RCY (%).

[Table 1]

| | chelating agent (A) | antibody (B) | molar ratio (A) : (B) | reaction rate | after purification | |
|---|---|---|---|---|---|---|
| | | | | 37°C 2 hr reaction (%) | RCP (%) | RCY (%) |
| Example 1 | DOTAGA | cetuximab | 1:1.2 | 89% | 98% | 74% |

[Comparative Example 1] Production of conjugate with cetuximab by using $^{225}$Ac-labeled DOTA-DBCO

The operation was performed according to Example 1 except that DOTAGA-DBCO was changed to the following DOTA-DBCO. The results are shown in Table 2.

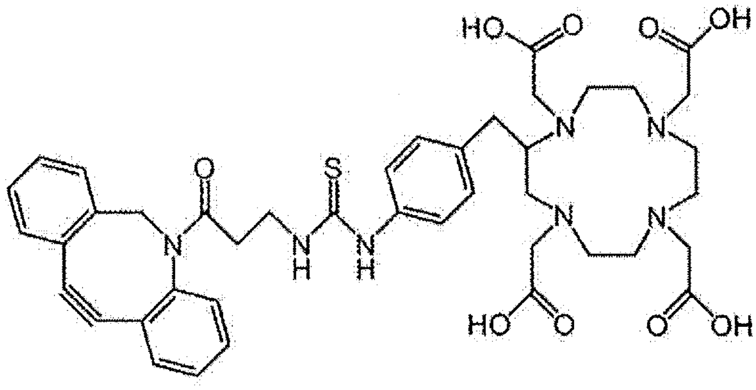

DOTA-DBCO

[Table 2]

| | chelating agent (A) | antibody (B) | molar ratio (A) : (B) | reaction rate | after purification | |
|---|---|---|---|---|---|---|
| | | | | 37°C 2 hr reaction (%) | RCP (%) | RCY (%) |
| Comparative Example 1 | DOTA | cetuximab | 1:1 | 66% | 99% | 62% |

[Example 2] Production of conjugate with cetuximab by using $^{89}$Zr-labeled DOTAGA-DBCO

(1. Complex formation step)

DOTAGA-DBCO was dispersed in 0.195 mol/L sodium acetate buffer (pH 5.5) as a solvent to give a dispersion containing 0.3 mmol/L chelating agent. A reaction mixture of the dispersion (0.0375 mL), and $^{89}$Zr ion-containing solution (0.1 mol/L aqueous hydrochloric acid solution, radioactivity concentration 5.2 GBq/mL, prepared from one manufactured by Nihon Medi-Physics Co., Ltd., liquid amount 0.0375 mL) 195 MBq as a radioactive metal source was reacted under heating conditions to give a $^{89}$Zr complex solution. The molar ratio of the chelating agent and the radioactive metal ion was chelating agent:$^{89}$Zr ion = about 85:1, and the heating temperature of the reaction mixture was set to 70°C, and the heating time was set to 60 min.

The RCP of the obtained $^{89}$Zr complex was measured by the following method. That is, a part of the $^{89}$Zr complex solution was developed by thin layer chromatography (manufactured by Agilent, model number: SGI0001, developing solvent: acetonitrile/water mixed solution (volume ratio 1:1)), and then measured by radio γ-TLC Analyzer (manufactured by raytest, MODEL GITA Star PS). The percentage of the radioactivity (count) of the peak detected near the origin with respect to the detected total radioactivity (count) was defined as the RCP (%) of the $^{89}$Zr complex. As a

result, the RCP of the $^{89}Zr$ complex was 96%. The obtained $^{89}Zr$ complex solution was used as it was in the labeling step.

(2. Labeling step)

A solution of the unpurified $^{89}Zr$ complex obtained in the aforementioned step (1), and a solution containing a peptide-modified antibody (monovalent antibody) obtained in the same manner as in Example 1 were each added without purification to 0.01 mol/L citrate buffer (pH 5.5) containing 0.1 mol/L sodium chloride and 0.1 mol/L glycine, and a click reaction was performed at 37°C for 90 min to give $^{89}Zr$ complex-labeled antibody of Example 2. The amount of the $^{89}Zr$ complex and the amount of the peptide-modified antibody (monovalent antibody) were 11.3 nmol and 12 nmol, respectively, and the molar ratio of DBCO and azide was each about 1:1.1. The reaction rate (%) of the unpurified $^{89}Zr$ complex-labeled antibody of the Example is shown in the following Table 3. Here, the reaction rate (%) means RCP of the $^{89}Zr$ complex-labeled antibody with respect to the labeling rate (%) in the complex formation step, and the labeling rate (%) means the amount of radioactivity (%) of the $^{89}Zr$ complex with respect to the charged radioactivity amount.

Furthermore, a solution of the $^{89}Zr$ complex-labeled antibody obtained by reacting at 37°C for 2 hr was purified using ultrafiltration filter (manufactured by Merck, model number: UFC505096). The RCP and RCY of the $^{89}Zr$ complex-labeled antibody after purification are shown in the following Table 3.

The measurement method of the RCP and RCY of the $^{89}Zr$ complex-labeled antibody was similar to that in Example 1.

[Table 3]

| | chelating agent (A) | antibody (B) | molar ratio (A) : (B) | reaction rate | after purification | |
|---|---|---|---|---|---|---|
| | | | | 37°C 2 hr reaction (%) | RCP(%) | RCY (%) |
| Example 2 | DOTAGA | cetuximab | 1:1.1 | 70% | 97% | 55% |

[Comparative Example 2] Production of conjugate with cetuximab by using $^{89}Zr$-labeled DOTA-DBCO

The operation was performed according to Example 2 except that DOTAGA-DBCO was changed to DOTA-DBCO. The results are shown in Table 4.

[Table 4]

| | chelating agent (A) | antibody (B) | molar ratio (A) : (B) | reaction rate | after purification | |
|---|---|---|---|---|---|---|
| | | | | 37°C 2 hr reaction (%) | RCP (%) | RCY (%) |
| Comparative Example 2 | DOTA | cetuximab | 1:1.1 | 56% | 93% | 38% |

[Example 3] Production of conjugate with cetuximab by using $^{177}Lu$-labeled DOTAGA-DBCO

(1. Antibody modification step)

This step was performed by a method similar to the method described in the antibody modification step in Example 1.

(2. Complex formation step)

DOTAGA-DBCO was produced in the same manner as in Example 1. This chelating agent was dispersed in 0.156 mol/L sodium acetate buffer (pH 5.5) as a solvent to give a dispersion containing 0.45 mmol/L chelating agent. A reaction mixture of the dispersion (0.015 mL), 0.156 mol/L sodium acetate buffer (pH 5.5, 0.015 mL) dissolving 0.225 mmol/L gentisic acid, and $^{177}Lu$ ion-containing solution (0.04 mol/L aqueous hydrochloric acid solution, radioactivity concentration 3.4 GBq/mL, prepared from one produced by POLATOM, liquid amount 0.0375 mL) 127 MBq as a radioactive metal source was reacted under heating conditions to give a $^{177}Lu$ complex solution. The molar ratio of the chelating agent and the radioactive metal ion was chelating agent:$^{177}Lu$ ion = about 38:1, and the heating temperature of the reaction mixture was set to 70°C, and the heating time was set to 5 min.

The RCP of the obtained $^{177}Lu$ complex was measured in the same manner as in the measurement of RCP of the radioconjugate of Example 1. As a result, the RCP of the $^{177}Lu$ complex was 100%. The obtained $^{177}Lu$ complex solution was directly used for the labeling step.

(3. Labeling step)

A solution of the unpurified $^{177}Lu$ complex obtained in the aforementioned step (2), and a solution containing a peptide-modified antibody (monovalent antibody) obtained in the above-mentioned step (1) were each added to 0.01 mol/L citrate buffer (pH 5.5) containing 0.1 mol/L sodium chloride and 0.1 mol/L glycine, and a click reaction was performed at 37°C for 120 min to give $^{177}Lu$ complex-labeled antibody. The amount of the $^{177}Lu$ complex and the amount of the peptide-modified antibody (monovalent antibody) were 6.75 nmol and 7.5 nmol, respectively, and the molar ratio of the DBCO group and the azide group was about 1:1.1. The reaction rate (%) of the unpurified $^{177}Lu$ complex-labeled antibody is shown in the following Table 5.

In addition, the RCP and RCY of the $^{177}Lu$ complex-labeled antibody after purification using an ultrafiltration filter in the same manner as in Example 1 are shown in the following Table 5.

The RCP and RCY of the $^{177}Lu$ complex-labeled antibody were measured in the same manner as in Example 1.

[Table 5]

| | chelating agent (A) | antibody (B) | molar ratio (A) : (B) | reaction rate | after purification | |
|---|---|---|---|---|---|---|
| | | | | 37°C 2 hr reaction (%) | RCP (%) | RCY(%) |
| Example 3 | DOTAGA | cetuximab | 1:1.1 | 98% | 100% | 48% |

[Comparative Example 3] Production of conjugate with cetuximab by using $^{177}Lu$-labeled DOTA-DBCO

The operation was performed according to Example 3 except that DOTAGA-DBCO was changed to DOTA-DBCO. The results are shown in Table 6.

[Table 6]

| | chelating agent (A) | antibody (B) | molar ratio (A) : (B) | reaction rate | after purification | |
|---|---|---|---|---|---|---|
| | | | | 37°C 2 hr reaction (%) | RCP (%) | RCY (%) |
| Comparative Example 3 | DOTA | cetuximab | 1:1.1 | 91% | 100% | 64% |

[Example 4] Formulation step

A portion of each of the radioconjugates prepared as described in Example 1, Example 3, Comparative Example 1 or Comparative Example 3 was placed in a 0.5 mL Eppen tube (LoBind, manufactured by Eppendorf) and diluted with a storage buffer (0.01 mol/L citrate buffer (pH 5.5) containing 0.1 mol/L sodium chloride and 0.1 mol/L glycine, and 0.01 mol/L citrate buffer (pH 5.5) containing 1.0 weight/% by volume polysorbate 80, 0.1 mol/L sodium chloride and 0.1 mol/L glycine mixed solution).

[Evaluation 1] Stability evaluation

Each radioconjugate obtained in Example 4 was stored at room temperature for 2 weeks, and RCP and antigen binding activity were evaluated at each time point (0 day point, 1 day point, 7 day point, and/or 14 day point). Note that 7 days after completion of production corresponds to about 1 half-life when the metal radionuclide is Lu-177. Also, 14 days after the end of production corresponds to about 1.5 half-life when the metal radionuclide is Ac-225 and about 2 half-life when the metal radionuclide is Lu-177.

[Evaluation 1-1] RCP (Radiochemical purity)

RCP was analyzed by thin layer chromatography (TLC). The TLC conditions were similar to those used for

examining the reaction rate in Example 1. The results are shown in Table 7.

[Table 7]

| | radiochemical purity (%) | | | |
|---|---|---|---|---|
| | 0 day point | 1 day point | 7 day point | 14 day point |
| radioconjugate (Example 1) | 100.0 | 99.7 | 97.9 | 94.9 |
| radioconjugate (Comparative Example 1) | 99.8 | 99.5 | 91.5 | 71.4 |
| radioconjugate (Example 3) | 99.7 | 97.5 | 93.9 | 88.2 |
| radioconjugate (Comparative Example 3) | 99.4 | 93.5 | 79.6 | 61.4 |

The radioconjugate produced as described in Example 1 containing no thiourea bond maintained an RCP of 95% or more when stored at room temperature for 7 days after completion of the production. It maintained an RCP of 90% or more when stored at room temperature for 14 days after completion of the production.

The radioconjugate produced as described in Comparative Example 1 containing a thiourea bond maintained an RCP of 90% or more but below 95% when stored at room temperature for 7 days after completion of the production. When stored at room temperature for 14 days after completion of the production, the RCP was less than 75%.

The radioconjugate produced as described in Example 3 containing no thiourea bond maintained an RCP of 90% or more when stored at room temperature for 7 days after completion of the production. Even when stored at room temperature for 14 days after completion of the production, 85% or more of RCP was maintained.

The radioconjugate produced as described in Comparative Example 3 containing a thiourea bond maintained an RCP of 75% or more but below 90% when stored at room temperature for 7 days after completion of the production. When stored at room temperature for 14 days after completion of the production, the RCP was less than 65%.

[Evaluation 1-2] Antigen binding activity

Antigen binding activity was confirmed by in vitro autoradiography (ARG) (production day (0) and preservation final day (14 day point) alone). A431 cells (human epithelioid cancer cell line with high EGFR expression) purchased from ECACC (European Collection of Authenticated Cell Cultures) and SNU-16 cells (human gastric cancer cell line with low EGFR expression) purchased from ATCC (American Type Culture Collection) were administered subcutaneously to the flanks of female SCID Beige mice (manufactured by Charles River Laboratories Japan, Inc.) at $5\times10^6$ cells and $2\times10^6$ cells, respectively, to prepare tumor-bearing mice. Thereafter, A431 tumor and SNU-16 tumor were excised and embedded in Tissue-Tek O.C.T. Compound (Japanese Sakura Finetek Japan Co., Ltd.) to prepare frozen sections. The radioconjugates obtained in Example 1 and Comparative Example 1 were added to 1% bovine serum albumin-containing PBS each at 1 kBq/mL, and A431 tumor section and SNU-16 tumor section were immersed therein. After contacting the sections with the imaging plate, they were read with a scanner-type image analyzer to evaluate the level of radioactivity bound to the sections. The results are shown in Fig. 1.

By performing the same evaluation of each solution with cetuximab added thereto, the specificity of each radioconjugate for EGFR can be confirmed.

At the end point of storage (14 day point), binding activity to EGFR was confirmed in all of the radioconjugates prepared as described in Example 1 and Comparative Example 1. Both radioconjugates prepared as described in Example 1 and Comparative Example 1 bound to the A431 tumor section and SNU-16 tumor section, more strongly bound to the A431 tumor section, demonstrating EGFR selective binding. In the solution added with cetuximab, binding to the A431 tumor section was inhibited, and EGFR specificity of binding was confirmed. At the end point of storage (14 day point), selectivity of binding to EGFR was maintained in all samples. The radioactivity bound to A431 tumor section was higher in the samples using the radioconjugates prepared as described in Example 1 than in the radioconjugates prepared as described in Comparative Example 1.

[Evaluation 2] in vivo tumor accumulation

According to Evaluation 1-2, a subcutaneous tumor-bearing model of A431 cells was prepared using a mouse, and the tumor accumulation of the radioconjugates prepared according to the description of Example 2 or Comparative Example 2 was confirmed.

EGFR-positive human epithelioid cancer cell line A431 purchased from ATCC were suspended in DMEM medium (gibco, manufactured by Thermo Fisher Scientific) and administered subcutaneously to the flanks of 5-week-old female BALB/c nu/nu (Charles River Laboratories Japan, Inc.) at $5\times10^6$ cells to prepare tumor-bearing mice. The tumor was allowed to grow to a volume of about 100 to 250 $mm^3$, and the radioconjugate produced as described in Example 2 or Comparative Example 2 was administered at a dose of 3.7 MBq/mouse (each n=3) into the tail vein. After 96 hours from the administration, images were taken under the conditions of Table 8 and using a small animal PET imaging device (PET/CT Si78, manufactured by Bruker).

Typical examples of the results of the PET imaging are shown in Fig. 2 (Example 2) and Fig. 3 (Comparative Example 2). A higher level of radioactivity was accumulated in the tumor as compared with other organs, and EGFR positive tumor could be depicted. Furthermore, the radioconjugate produced as described in Example 2 and the radioconjugate produced as described in Comparative Example 2 showed visually similar tumor accumulation. When analyzed by setting a VOI (volume of interest) on the tumor in the example image shown in Fig. 2 or Fig. 3, the radioconjugate produced as described in Example 2 showed 5.1% ID/cc (Injected dose/cc) as a ratio of radioactivity per unit volume to the administered radioactivity, and the radioconjugate produced as described in Comparative Example 2 showed a similar level of 4.6%ID/cc.

[Table 8]

| | |
|---|---|
| Isotope | 89-Zr |
| Acquisition time | 600 sec |
| Energy Window | 30% (357.7-664.3keV) |
| PET image reconstruction | MLEM GPU 32x32 0.25 (Iterations: 12) |
| correction | Scatter, Randoms, Decay, Partial volume, Attenuation |

[Example 5] Efficacy evaluation using $^{225}Ac$ complex-labeled antibody (antitumor effect of $^{225}Ac$ complex-labeled cetuximab)

A subcutaneous tumor-bearing model of A431 was prepared using mice, and the antitumor effect of the radioconjugate produced as described in Example 1 and Comparative Example 1 was confirmed.

A431 cells of EGFR high expression human epithelioid cancer cell line purchased from ECACC were suspended in DMEM medium (gibco, manufactured by Thermo Fisher Scientific) and administered subcutaneously to the flanks of female 5-week-old BALB/c nu/nu (Charles River Laboratories Japan, Inc.) at $5\times10^6$ cells to prepare tumor-bearing mice. The tumor volume was allowed to grow to 200 to 350 $mm^3$, and individuals with a shape suitable for tumor diameter measurement were randomly grouped. The tumor volume and body weight of each mouse at that time are shown in Table 9. The tumor volume was calculated according to the following formula.

$$\text{tumor volume } (mm^3) = (\text{tumor major axis x (tumor minor axis)}^2) \times 1/2$$

[Table 9]

| | tumor volume mean±standard deviation ($mm^3$) | body weight mean±standard deviation (g) |
|---|---|---|
| radioconjugate (Example 1) administration group | 316.2±68.0 | 20.5±1.5 |
| radioconjugate (Comparative Example 1) administration group | 309.2181.5 | 20.7±1.4 |
| antibody control group | 311.9±51.6 | 20.5±1.1 |
| Vehicle group | 307.3±68.0 | 20.5±1.1 |

The radioconjugates prepared as described in Example 1 and Comparative Example 1 were administered into the tail vein at a dose of 15 kBq/mouse (100 ug/mouse as cetuximab). As a control group, a group administered with cetuximab with the same amount of antibody as each radioconjugate (antibody control group) and a Vehicle group

administered with a storage buffer were set. Each group contained 6 mice, and observation of general condition and measurement of the body weight and tumor volume were performed over time for 37 days after administration. The changes in tumor volume over time are shown in Fig. 4, and the change in body weight over time is shown in Fig. 5.

The groups administered with the radioconjugates prepared as described in Example 1 and Comparative Example 1 showed a significant difference in the antitumor effect as compared with the two control groups (antibody control group and Vehicle group) at 37 day point after the administration ($P<0.05$ or $P<0.01$). For determination of significant difference, Tukey test was performed using statistical analysis software Stat Preclinica (manufactured by Takumi Information Technology Inc.). In each group, no significant change was found in the general condition, and no sign of toxicity such as significant loss of weight was observed.

[Example 6] Production of conjugate with panitumumab by using $^{225}$Ac-labeled DOTAGA-DBCO

(1. Antibody modification step)

A peptide containing 17 amino acid residues represented by the above-mentioned (P3) was obtained by the method described in WO 2017/217347.

A mixture of the aforementioned peptide and a solution containing panitumumab (manufactured by Amgen) in a 0.02 mol/L acetic acid·sodium acetate buffer (pH 6.0) was reacted at room temperature for 60 min to give a solution containing a peptide-modified antibody. The peptide-modified antibody has an Fc region of the antibody site-specifically modified by the above-mentioned peptide.

Then, the solution was then passed through the IgG-BP column to obtain an antibody composition containing relatively large amounts of the unlabeled antibody and monovalent antibody. The concentration of the monovalent antibody contained in the recovered fraction was adjusted with 0.05 mol/L sodium acetate buffer (pH 5.8) containing 0.1 mol/L sodium chloride such that the concentration was 15 mg/mL. An obtained solution containing relatively large amounts of the unlabeled antibody and monovalent antibody was subjected to the below-mentioned labeling step.

(2. Complex formation step)

DOTAGA-DBCO was produced in the same manner as in Example 1. This chelating agent was dispersed in 0.156 mol/L sodium acetate buffer (pH 6.0) as a solvent to give a dispersion containing 0.3 mmol/L chelating agent. A reaction mixture of the dispersion (0.0136 mL), 0.156 mol/L sodium acetate buffer (pH 5.5, 0.0136 mL), and $^{225}$Ac ion-containing solution (0.1 mol/L aqueous hydrochloric acid solution, radioactivity concentration 210 - 222 MBq/mL, prepared from one produced by Oak Ridge National Laboratory, liquid amount 0.0093 mL) 1.95 to 2.06 MBq (calculated by attenuation from the level of radioactivity at test date and time) as a radioactive metal source was reacted under heating conditions to give a $^{225}$Ac complex solution. The molar ratio of the chelating agent and the radioactive metal ion then was chelating agent:$^{225}$Ac ion = about 989:1, and the heating condition of the reaction mixture was set to 70°C, and the heating time was set to 30 min.

The RCP of the obtained $^{225}$Ac complex was measured in the same manner as in Example 1. As a result, the RCP of the $^{225}$Ac complex was 95%. The obtained $^{225}$Ac complex solution was directly used for the labeling step.

(3. Labeling step)

To a solution of the unpurified $^{225}$Ac complex obtained via the aforementioned step (2) was added a solution containing a peptide-modified antibody (monovalent antibody) obtained in the above-mentioned step (1), and a click reaction was performed at 37°C for 2 hr to give $^{225}$Ac complex-labeled antibody. The molar ratio of the DBCO group and the azide group was about 1:1.2. The reaction rate of the unpurified $^{225}$Ac complex-labeled antibody is shown in the following Table 10.

Furthermore, a solution of the $^{225}$Ac complex-labeled antibody obtained by reacting at 37°C for 2 hr was purified using ultrafiltration filter (manufactured by Merck, model number: UFC803096). The RCP and RCY of the $^{225}$Ac complex-labeled antibody after purification are shown in the following Table 10. The RCP and RCY of the $^{225}$Ac complex-labeled antibody were calculated based on the radioactivity level obtained by a method similar to that in Example 1.

[Table 10]

| | chelating agent (A) | antibody (B) | molar ratio (A) : (B) | reaction rate | after purification | |
|---|---|---|---|---|---|---|
| | | | | 37°C 2 hr reaction (%) | RCP (%) | RCY (%) |
| Example 6 | DOTAGA | panitumumab | 1:1.2 | 70 | 99 | 61 |

[Comparative Example 4] Production of conjugate with panitumumab by using $^{225}$Ac-labeled DOTA-DBCO

The operation was performed according to Example 6 except that DOTAGA-DBCO was changed to DOTA-DBCO. The results are shown in Table 11. The RCP and RCY of the $^{225}$Ac complex-labeled antibody were calculated based on the radioactivity level obtained by a method similar to that in Example 1.

[Table 11]

| | chelating agent (A) | antibody (B) | molar ratio (A) : (B) | reaction rate | after purification | |
|---|---|---|---|---|---|---|
| | | | | 37°C 2 hr reaction (%) | RCP (%) | RCY (%) |
| Comparative Example 4 | DOTA | panitumumab | 1:1.2 | 50 | 99 | 44 |

[Example 7] Production of complex with panitumumab using $^{89}$Zr-labeled DOTAGA-DBCO

(1. Complex formation step)

Similar to Example 2, a dispersion containing a chelating agent was prepared, and the dispersion (0.0939 mL) was mixed with 0.150 mol/L gentisic acid-containing 0.156 mmol/L sodium acetate solution (pH 5.5) (0.0626 mL), and $^{89}$Zr ion-containing solution (prepared from 0.1 mol/L aqueous hydrochloric acid solution, radioactivity concentration 2.6 GBq/mL, manufactured by Nihon Medi-Physics Co., Ltd., liquid amount 0.0626 mL) (161.6 MBq) as a radioactive metal source, and $^{89}$Zr complex solution was obtained according to Example 2. The molar ratio of the chelating agent and the radioactive metal ion then was chelating agent:$^{89}$Zr ion = about 236:1.

The RCP of the obtained $^{89}$Zr complex was measured by a method similar to that in Example 2. As a result, the RCP of the $^{89}$Zr complex was 98%. The obtained $^{89}$Zr complex solution was used as it was in the labeling step.

(2. Labeling step)

A solution of the unpurified $^{89}$Zr complex obtained in the aforementioned step (1), and a solution containing a peptide-modified antibody (monovalent antibody) obtained in the same manner as in Example 6 were mixed, and a click reaction was performed at 37°C for 90 min to give a $^{89}$Zr complex-labeled antibody. The molar ratio of DBCO and azide was each about 1:1. The reaction rate (%) of the unpurified $^{89}$Zr complex-labeled antibody is shown in the following Table 12.

Furthermore, a solution of the $^{89}$Zr complex-labeled antibody obtained by reacting at 37°C for 2 hr was purified using ultrafiltration filter. The RCP and RCY (%) of the $^{89}$Zr complex-labeled antibody after purification are shown in the following Table 12. The RCP and RCY of the $^{89}$Zr complex-labeled antibody were calculated based on the radioactivity level obtained by a method similar to that in Example 1.

[Table 12]

| | chelating agent (A) | antibody (B) | molar ratio (A) : (B) | reaction rate | after purification | |
|---|---|---|---|---|---|---|
| | | | | 37°C 2 hr reaction (%) | RCP (%) | RCY (%) |
| Example 7 | DOTAGA | panitumumab | 1:1 | 64 | 94 | 57 |

[Example 8] Formulation step

1.0 mL of each of the radioconjugates produced according to Example 6 and Comparative Example 4 was placed in a 5 mL Eppen tube (LoBind, manufactured by Eppendorf) and diluted with 1.5 mL of a formulation buffer (0.1 mol/L sodium chloride-containing 0.05 mol/L sodium acetate buffer (pH 5.8)).

[Evaluation 3] Stability evaluation

Each radioconjugate obtained in Example 8 was stored at room temperature (24.5-25.5°C) for 2 weeks, and RCP, proportion of aggregates, and antigen binding activity were evaluated at each time point (0 day point, 1 day point, 7 day point, and 14 day point).

[Evaluation 3-1] RCP

RCP was calculated from the TLC analysis results. The TLC conditions were similar to those used for examining the reaction rate in Example 1. The results are shown in Table 13.

[Table 13]

| | RCP (%) | | | |
|---|---|---|---|---|
| | 0 day point | 1 day point | 7 day point | 14 day point |
| radioconjugate (Example 6) | 100.0 | 99.9 | 99.8 | 99.5 |
| radioconjugate (Comparative Example 4) | 99.6 | 99.0 | 95.0 | 92.9 |

The radioconjugate produced as described in Example 6 containing no thiourea bond maintained an RCP of 99% or more when stored at room temperature for 7 days after completion of the production. Even when stored at room temperature for 14 days after completion of the production, 99% or more of RCP was maintained. The radioconjugate produced as described in Comparative Example 4 containing a thiourea bond maintained an RCP of 95% or more when stored at room temperature for 7 days after completion of the production. When stored at room temperature for 14 days after completion of the production, the RCP was less than 93%.

[Evaluation 3-2] Proportion of aggregate

The proportion of aggregates was confirmed by size-exclusion chromatography (SEC). Using Model 2695 Separation Module or e2695 Separation Module, manufactured by Waters, as a liquid chromatography device, and Model 2489 UV/Vis Detector manufactured by Waters as a UV detector, analysis was performed under the following conditions. The proportion of each component when stored for 7 days after completion of the production is shown in Table 14. When stored for 7 days after completion of the production, the proportion of aggregates of the radioconjugates prepared as described in Example 6 was equivalent to that of the aggregates of the radioconjugates prepared as described in Comparative Example 4.

[Table 14]

| | main peak proportion (%) | peak proportion (%) of aggregate |
|---|---|---|
| radioconjugate (Example 6) 0 day point | 92.20 | 7.80 |
| radioconjugate (Comparative Example 4) 0 day point | 91.49 | 8.51 |
| radioconjugate (Example 6) 7 day point | 89.92 | 10.03 |
| radioconjugate (Comparative Example 4) 7 day point | 89.77 | 10.17 |

[HPLC conditions]

column: TOSOH TSKgel guard column SWXL (6 mm × 4cm), TOSOH TSKgel G3000SWXL (5 μm, 7.8×30 cm)×2 (tandem)
column temperature: constant temperature around 25°C
mobile phase: 0.2 mol/L arginine hydrochloride-containing 0.1 mol/L phosphate buffer (pH 6.8)
flow: 1.0 mL/min
area measurement range: 30 min
detection wavelength: 280 nm

[Evaluation 3-3] Antigen binding activity

Antigen binding activity was confirmed (manufacturing date only) by in vitro ARG according to the description of Example 1 except that SW48 cell, which is a human colorectal cancer-derived cell line with high EGFR expression purchased from ATCC, HCT-116, which is a human colorectal cancer-derived cell line with low EGFR expression purchased from ATCC, and COLO205 cell, which is a human colon cancer-derived cell line with low EGFR expression

purchased from ECACC were included to the tumor section to be evaluated. In addition, a similar evaluation was performed using a solution in which unlabeled panitumumab was added to each solution (added panitumumab concentration: 10 nM), and the specificity of the radioconjugate to EGFR was confirmed. The results are shown in Fig. 6. The binding activity to EGFR was confirmed in the radioconjugate produced as described in Example 6. In the radioconjugate produced according to Example 6, binding was confirmed in the A431 tumor section, SNU-16 tumor section, SW48 tumor section, HCT-116 tumor section, and COLO205 tumor section, with stronger binding in the A431 tumor section and the SW48 tumor section, and EGFR expression dependent binding activity was confirmed. In the solution containing panitumumab, binding was inhibited in all sections used for evaluation, which confirms the EGFR specificity of binding.

[Example 9] Efficacy evaluation using $^{225}$Ac complex-labeled antibody (cytotoxic effect evaluation of $^{225}$Ac complex-labeled panitumumab)

The cytotoxic effect of a radioconjugate produced as described in Example 6 was confirmed using cultured cells. COLO205 cells (RPMI 1640 medium), HCT-116 cells (MaCoy's 5A medium), SW48 cells (Leibovitz's L-15 medium), MIAPaCa-2 cells of human pancreatic cancer-derived cell line with high EGFR expression purchased from ECACC (D-MEM medium), and NCI-H358 cells of non-small cell lung cancer-derived cell line with high EGFR expression purchased from ATCC (RPMI 1640 medium) were respectively cultured in appropriate media, a radioconjugate produced according to Example 6 was diluted with each medium to 0, 0.001, 0.01, 0.03, 0.1, 0.3, 1, 3, 10, 30 kBq/mL (0.000764, 0.00764, 0.0229, 0.0764, 0.229, 0.764, 2.29, 7.64, 22.9 μg/mL as panitumumab) and added to the cells. In addition, unlabeled panitumumab was added to an antibody concentration the same as that of the sample at each radioactivity concentration, and the cells were cultured. After 120 hours from the sample addition, CellTiter-Glo (registered trademark) 2.0 Cell Viability Assay (manufactured by Promega) was added to the medium, chemiluminescence was detected using a microplate reader (SpectraMax i3x, manufactured by Molecular Devices, LLC), and the number of viable cells was calculated. The ratio of the number of viable cells under conditions where no antibody was added to the calculated number of viable cells was taken, and the cytotoxic effect was evaluated. The results are shown in Figs. 7A to 7C. Furthermore, the obtained results were fitted by a nonlinear, linear regression method using GraphPad Prism 7 (manufactured by GraphPad Software), and $EC_{50}$ was calculated. As a result, the $EC_{50}$ of the cytotoxic effect of the radioconjugate produced as described in Example 6 was 0.5 pM against SW48 cells, 2.8 pM against NCI-H358 cells, and 7.3 pM against COLO205 cells. Furthermore, with unlabeled panitumumab, no cytotoxic effect was confirmed at not more than the maximum concentration added (antibody concentration: 156 nM).

In the evaluated cells, the cytotoxic effect of $^{225}$Ac complex-labeled panitumumab was confirmed even in cells for which unlabeled panitumumab failed to show a cytotoxic effect, including cells with mutations in KRAS (v-Ki-ras2 Kirsten rat sarcoma viral oncogene homolog) and BRAF (v-raf murine sarcoma viral oncogene homolog B1).

[Example 10] Efficacy evaluation using $^{225}$Ac complex-labeled antibody (evaluation of apoptosis induction by $^{225}$Ac complex-labeled panitumumab)

Induction of apoptosis of cultured cells of a radioconjugate produced as described in Example 6 was confirmed.

Human colorectal cancer-derived SW48 cells and COLO205 cells were cultured under conditions similar to those in Example 9, a radioconjugate produced according to Example 6 was added to 0, 0.001, 0.01, 0.03, 0.1, 0.3, 1, 3 kBq/mL (0.00764, 0.0764, 0.229, 0.764, 2.29, 7.64, 22.9 μg/mL as panitumumab). In addition, unlabeled panitumumab was added to an antibody concentration the same as that of the group at each radioactivity concentration, and the cells were cultured. After 72 hours from the sample addition, an apoptosis assay kit (Caspase-Glo (registered trademark) 3/7 Assay, manufactured by Promega) was added, and Caspase-3/7 activity was detected using a microplate reader. The results thereof are shown in Fig. 8. In both cells, apoptosis induction was not confirmed with unlabeled panitumumab, but with $^{225}$Ac complex-labeled panitumumab, Caspase-3/7 activity increased in a manner dependent on the added radioactivity, and apoptosis induction by radiation was confirmed.

[Example 11] Efficacy evaluation using $^{225}$Ac complex-labeled antibody (evaluation of DNA double strand break of panitumumab)

DNA double strand break effect on cultured cells of a radioconjugate produced as described in Example 6 was confirmed.

SW48 cells, MIAPaCa-2 cells, and NCI-H358 cells were cultured in the same manner as in Example 9, a radioconjugate produced as described in Example 6 was added to 1 kBq/mL, 10 kBq/mL, 30 kBq/mL (3.73, 37.3, 112 μg/mL as panitumumab), and the cells were cultured. After 48 hours from the sample addition, using a DNA damage detection kit (DNA Damage Detection Kit-γH2AX-Green, manufactured by DOJINDO LABORATORIES), the cells were stained with yH2AX, and nuclear staining and mounting were performed using DAPI-containing water-soluble mounting

agent (ProLong™ Diamond Antifade Mountant with DAPI, manufactured by Thermo Fisher Scientific). After mounting, γH2AX positive site and nucleus were detected using an HS ALL-in-one fluorescence microscope (BZ-9000, manufactured by KEYENCE). The results thereof are shown in Figs. 9A to 9C. In each cell, the proportion of yH2AX-positive cells increased in a manner dependent on the added radioactivity, and DNA double strand break was confirmed.

[Example 12] Efficacy evaluation using $^{225}$Ac complex-labeled antibody (antitumor effect of $^{225}$Ac complex-labeled panitumumab)

A subcutaneous tumor-bearing model of COLO205 cells was prepared using a mouse, and the antitumor effect of the radioconjugate produced as described in Example 6 was confirmed.

COLO205 cells of human colorectal cancer-derived cell line with high EGFR expression purchased from ECACC were suspended in DMEM (gibco, manufactured by Thermo Fisher Scientific), and administered subcutaneously to the flanks of 6-week-old female BALB/c nu/nu (Charles River Laboratories Japan, Inc.) at $5\times10^6$ cells to prepare tumor-bearing mice. After the tumor-bearing treatment, it was confirmed that the tumor volume was approximately 100 to 300 $mm^3$, and individuals with a shape suitable for tumor diameter measurement were randomly grouped. The tumor volume and body weight of each mouse at that time are shown in the following Table 15.

[Table 15]

| | tumor volume mean±standard deviation ($mm^3$) | body weight mean±standard deviation (g) |
|---|---|---|
| radioconjugate (Example 6) high radioactivity administration group | 189.7±35.7 | 18.5±0.8 |
| radioconjugate (Example 6) low radioactivity administration group | 199.2148.6 | 18.6±0.6 |
| antibody peritoneal administration group | 186.0136.5 | 17.9±1.9 |
| antibody control group | 289.2±159.8 | 18.2±1.3 |
| Vehicle group | 208.0131.5 | 18.111.5 |

A radioconjugate produced as described in Example 6 was administered into the tail vein at a dose of 10 kBq/mouse in the high radioactivity administration group and at 5 kBq/mouse in the low radioactivity administration group (50 μg/mouse as panitumumab for both). As a control group, a group administered with panitumumab into the tail vein at a dose of 200 μg/mouse (antibody control group) and a Vehicle group administered with a storage buffer into the tail vein were set. In addition, a group (antibody peritoneal administration group) was established by intraperitoneally administering panitumumab twice a week for 2 weeks at a dose of 200 μg/mouse according to the CTD (Common Technical Document) of Vectibix (registered trademark, manufactured by Takeda Pharmaceutical Company Limited). Each group contained 6 mice, and observation of general condition and measurement of the body weight and tumor volume were performed over time for 33 days after administration. The changes in tumor volume over time are shown in Fig. 10.

The groups administered at high radioactivity with the radioconjugate produced as described in Example 6 showed a significant difference in the antitumor effect as compared with the two control groups (antibody control group and Vehicle group) and antibody peritoneal administration group at 33 day point after the administration ($P<0.05$ or $P<0.01$). For determination of significant difference, Tukey test was performed using statistical analysis software Stat Preclinica. A group to which a radioconjugate produced as described in Example 6 was administered with low radioactivity showed, at 33 day point after the administration, a significant difference in the antitumor effect ($P<0.05$ or $P<0.01$) as compared with the antibody control group and the antibody peritoneal administration group. On the other hand, no significant difference in antitumor effect was observed between the groups to which each radioconjugate was administered. In each group, no significant change was found in the general condition, and no sign of toxicity such as significant loss of weight was observed.

[Example 13] Efficacy evaluation using $^{225}$Ac complex-labeled antibody (antitumor effect of $^{225}$Ac complex-labeled panitumumab)

A subcutaneous tumor-bearing model of HCT-116 cells was prepared using a mouse, and the antitumor effect of a radioconjugate produced as described in Example 6 was confirmed.

In the same manner as in Example 12 except that HCT-116 cells of a human colorectal cancer-derived cell

line with high EGFR expression purchased from ATCC were suspended in DMEM (gibco, manufactured by Thermo Fisher Scientific) and used, the antitumor effect was confirmed. The tumor volume and body weight of each mouse when each solution was administered are shown in the following Table 16.

[Table 16]

| | tumor volume mean±standard deviation ($mm^3$) | body weight mean±standard deviation (g) |
|---|---|---|
| radioconjugate (Example 6) high radioactivity administration group | 284.1±116.1 | 18.2±0.7 |
| radioconjugate (Example 6) low radioactivity administration group | 251.7±124.7 | 18.3±0.9 |
| antibody peritoneal administration group | 266.1190.7 | 17.6±1.3 |
| antibody control group | 289.2±159.8 | 17.9±1.1 |
| Vehicle group | 263.9±66.5 | 17.8±1.1 |

Group setting, administered radioactivity, and administered antibody amount were the same as those in Example 12. Each group contained 6 mice, and observation of general condition and measurement of the body weight and tumor volume were performed over time for 26 days after administration. The changes in tumor volume over time are shown in Fig. 11.

The groups administered at high radioactivity with the radioconjugates produced as described in Example 6 showed a significant difference in the antitumor effect as compared with the two control groups (antibody control group and Vehicle group) and the antibody peritoneal administration group at 26 day point after the administration ($P<0.01$). For determination of significant difference, Tukey test was performed using statistical analysis software Stat Preclinica. A significant difference in the antitumor effect ($P<0.05$) was observed between the groups to which each radioconjugate was administered, and a dependency on the administered radioactivity was confirmed in the antitumor effect. No remarkable change was found in the general condition in each group, and no sign of toxicity such as significant loss of weight was observed.

[Example 14] Efficacy evaluation using $^{225}Ac$ complex-labeled antibody (antitumor effect of $^{225}Ac$ complex-labeled panitumumab)

A subcutaneous tumor-bearing model of SW48 cells was prepared using mice, and the antitumor effect of the radioconjugate produced as described in Example 6 was confirmed.

In the same manner as in Example 12 except that SW48 cells of a human colorectal cancer-derived cell line with high EGFR expression purchased from ATCC were suspended in DMEM (gibco, manufactured by Thermo Fisher Scientific) and used, the antitumor effect was confirmed. The tumor volume and body weight of each mouse when each solution was administered are shown in the following Table 17.

[Table 17]

| | tumor volume mean±standard deviation ($mm^3$) | body weight mean±standard deviation (g) |
|---|---|---|
| radioconjugate (Example 6) high radioactivity administration group | 516.9±76.1 | 19.8±1.6 |
| radioconjugate (Example 6) low radioactivity administration group | 510.7±67.7 | 19.410.8 |
| antibody peritoneal administration group | 641.2±195.7 | 19.8±1.6 |
| antibody control group | 492.8±85.2 | 19.0±1.3 |
| Vehicle group | 495.0±89.9 | 19.8±1.1 |

Group setting, administered radioactivity, and administered antibody amount were the same as those in Example 12 except that the administered antibody amount in the antibody control group was set to 50 μg/mouse. Each group

contained 6 mice, and observation of general condition and measurement of the body weight and tumor volume were performed over time for 18 days after administration. The changes in the tumor volume over time are shown in Fig. 12.

The group administered with the radioconjugate produced as described in Example 6 showed a significant difference in the antitumor effect as compared with the two control groups (antibody control group, Vehicle group) at 18 day point after the administration (P<0.01). For determination of significant difference, Tukey test was performed using statistical analysis software Stat Preclinica. On the other hand, no significant difference was found in the antitumor effect between the groups administered with each radioconjugate. In each group, no significant change was found in the general condition, and no sign of toxicity such as significant loss of weight was observed.

[Example 15] Efficacy comparison of $^{225}$Ac complex-labeled panitumumab and existing drugs

A subcutaneous tumor-bearing model of COLO205 cells was prepared using a mouse, and the antitumor effect of the radioconjugate produced as described in Example 6 and existing drug was compared.

Similar to Example 12, tumor-bearing mice bearing COLO205 cells were produced, and the antitumor effect was confirmed. The tumor volume and body weight of each mouse when each solution was administered are shown in Table 18 below.

[Table 18]

| | tumor volume mean±standard deviation (mm$^3$) | body weight mean±standard deviation (g) |
|---|---|---|
| radioconjugate (Example 6) administration group | 267.8±66.4 | 20.1±1.0 |
| oxaliplatin administration group | 269.4±65.4 | 19.8±1.0 |
| Vehicle group | 260.2±77.2 | 19.7±1.0 |

It was administered into the tail vein at a dose of 10 kBq/mouse in the group administered with the radioconjugate produced as described in Example 6. As a comparison group, a group (oxaliplatin administration group) was set by administering 33 μL of oxaliplatin into the tail vein at a dose of 5 mg/mL 3 times every other week. As a control group, a Vehicle group was set by administering a storage buffer used for the radioconjugate produced as described in Example 6 into the tail vein. Each group contained 6 mice, and observation of general condition and measurement of the body weight and tumor volume were performed until the end of the observation period. The time-course changes in tumor volume are shown in Fig. 13. In the Figure, the vertical axis indicates mean tumor volume of each group, and the horizontal axis indicates the number of days elapsed since administration of each medicament. The graph represents the mean±standard deviation of tumor volume in each group, "*" is the time point when a significant difference (p<0.05) was observed from the oxaliplatin administration group, and "†" is the time point when a significant difference (p<0.01) was observed from the Vehicle group. On the final day of observation, the mice were autopsied, and the blood was collected. The collected blood was analyzed by a dry chemistry analyzer (SPOTCHEM D-02, manufactured by ARKRAY, Inc.) by measuring aspartate aminotransferase (AST), alanine aminotransferase (ALT), and blood urea nitrogen (BUN) in plasma by using SPOTCHEM D Total-A2 (manufactured by ARKRAY, Inc.), and hepatotoxicity and nephrotoxicity were evaluated. The results are shown in Fig. 14. The vertical axis shows the concentration of each marker, the horizontal axis shows evaluated groups, and the graphs show the mean±standard deviation in each group. There were no major differences in hepatotoxicity and nephrotoxicity markers in each group on the final day of observation, and no hepatotoxicity or nephrotoxicity was confirmed at the final observation point.

[Example 16] Stability evaluation of $^{225}$Ac complex-labeled panitumumab in plasma

A radioconjugate produced as described in Example 6 was stored at 37°C for 2 weeks in the plasma of each animal species (human (human plasma (pool, heparin), manufactured by Cosmo Bio Company, Limited), mouse (mouse plasma pool BALB/c-nu lineage, manufactured by Charles River Laboratories Japan, Inc.), rat (plasma (pool) Wistar, manufactured by Charles River Laboratories Japan, Inc.), monkey (Macaca fascicularis plasma, manufactured by Charles River Laboratories Japan, Inc.)), and the stability of $^{225}$Ac-labeled panitumumab in the plasma was evaluated at each time point (0 day point, 1 day point, 7 day point, 14 day point (human plasma alone)). Plasma of each animal species was incubated at 37°C, and a radioconjugate produced as described in Example 6 was added at a final concentration of 100 kBq/mL. At each evaluation time point, immunoprecipitation was performed using Dynabeads (registered trademark) Protein G (manufactured by Thermo Fisher Scientific), and after washing with PBS containing 0.1% Tween 20,

antibodies bound to Protein G were collected. The PBS containing 0.1% Tween 20 used for washing the beads was collected, and the radioactivity of the collected washing solution and the radioactivity of the collected antibody were measured using an Autowell gamma counter (2480 WIZARD$^2$ gamma counter, manufactured by PerkinElmer). The ratio of the radioactivity of the recovered antibody to the total of the radioactivity of PBS containing 0.1% Tween 20 used for washing the beads and the radioactivity of the recovered antibody was calculated, whereby the percentage of $^{225}$Ac-labeled panitumumab bound to Protein G was evaluated. The results thereof are shown in Table 19. The values in the Table indicate mean±standard deviation (n=3) of the ratio of the radioactivity of the recovered antibody to the total of the radioactivity of PBS containing 0.1% Tween 20 used for washing the beads and the radioactivity of the recovered antibody.

[Table 19]

| stability (%) | 0 day point | 1 day point | 7 day point | 14 day point |
|---|---|---|---|---|
| human plasma | 92.4±0.4 | 85.6±5.0 | 88.6±3.9 | 71.6±6.3 |
| monkey plasma | 91.0±2.0 | 89.9±4.5 | 86.9±3.0 | - |
| mouse plasma | 97.8±0.4 | 87.9±1.2 | 88.2±0.6 | - |
| rat plasma | 96.8±1.0 | 88.5±3.3 | 69.9±6.4 | - |

The radioconjugate produced as described in Example 6 maintained a stability of 85% or more when stored for 7 days in the plasma of each animal species except rat, and maintained a stability of 70% or more even when stored for 14 days in human plasma.

[Example 17] Evaluation of accumulation of $^{89}$Zr complex-labeled panitumumab in tumor

According to Example 12, a subcutaneous tumor-bearing model of COLO205 cells was prepared using mouse, and the tumor accumulation of the radioconjugate produced as described in Example 12 was confirmed.

COLO205 cells of EGFR high expression human colorectal cancer-derived cell line purchased from ATCC were suspended in DMEM medium and administered subcutaneously to the flanks of 5-week-old female BALB/c nu/nu (Charles River Laboratories Japan, Inc.) at 5×10$^6$ cells to prepare tumor-bearing mice. The tumor volume was allowed to grow to about 100 to 250 mm$^3$, and the radioconjugate produced as described in Example 7 was administered at a dose of about 6 MBq/mouse (each n=3) into the tail vein. After 72 hr and 120 hr from the administration, images were taken using a small animal PET imaging device under conditions similar to those in Evaluation 2.

Representative examples of the results of the PET imaging are shown in Fig. 15. The arrow in the image indicates a tumor derived from COLO205 cells used for tumor-bearing. When VOI was set for the tumor and muscle (femoral muscle) in each imaged mouse and the SUV (Standard Uptake Value) was calculated, the value was 3.18±0.72 for tumor and 0.48±0.02 for muscle at 72 hr after administration and 4.38±0.96 for tumor and 0.48±0.05 for muscle at 120 hr after administration. Using the values, the radioactivity accumulation ratio of tumor to muscle was calculated. As a result, the ratio was 6.67±1.52 at 72 hr after administration and 9.19±2.02 at 120 hr after administration, and a higher level of radioactivity was accumulated in the tumor as compared with other organs and EGFR positive tumor could be depicted.

This application is based on a patent application No. 2021-062104 filed in Japan (filing date: March 31, 2021), a patent application No. 2021-179348 filed in Japan (filing date: November 2, 2021), and a patent application No. 2022-021670 filed in Japan (filing date: February 15, 2022), the contents of which are incorporated in full herein.

## Claims

1. A conjugate of an anti-EGFR antibody site-specifically modified with a peptide and a chelating agent, wherein the chelating agent is chelated with a metal radionuclide, the peptide and the chelating agent are linked by a linker (L), and the linker (L) does not contain a thiourea bond.

2. The conjugate according to claim 1, wherein the chelating agent is DOTAGA (α-(2-Carboxyethyl)-1,4,7,10-tetraazacyclododecane-1,4,7,10-tetraacetic acid).

3. The conjugate according to claim 1 or 2, wherein the peptide is an amino acid sequence consisting of not less than 13 and not more than 17 amino acid residues and is represented by the following formula (i):

(Xa)-Xaa1-(Xb)-Xaa2-(Xc)-Xaa3-(Xd)··· (i)

in the formula (i), Xa, Xb, Xc and Xd are continuous X in the number of a, continuous X in the number of b, continuous X in the number of c, and continuous X in the number of d, respectively,

X is an amino acid residue having neither a thiol group nor a haloacetyl group in the side chain,
a, b, c and d are each independently an integer of not less than one and not more than 5, and satisfy $a+b+c+d \leq 14$,
Xaa1 and Xaa3 are each independently an amino acid residue derived from an amino acid having a thiol group in the side chain, or an amino acid residue derived from an amino acid having a haloacetyl group in the side chain, provided that one of Xaa1 and Xaa3 is an amino acid residue derived from an amino acid having a thiol group in the side chain,
Xaa1 and Xaa3 are connected to form a ring structure, and
Xaa2 is a lysine residue, an arginine residue, a cysteine residue, an aspartic acid residue, a glutamic acid residue, 2-aminosuberic acid, or diamino propionic acid, and modified with a crosslinking agent.

**4.** The conjugate according to any one of claims 1 to 3, wherein the metal radionuclide is Ac-225, Y-90, Lu-177, or Zr-89.

**5.** The conjugate according to any one of claims 1 to 4, wherein the linker (L) comprises the formula (10a), the formula (10b), or the formula (10c):

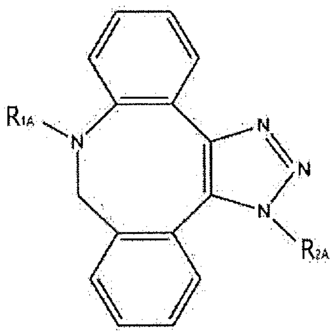

(10a)

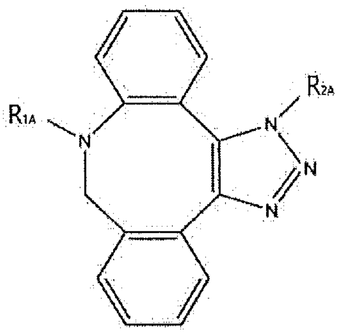

(10b)

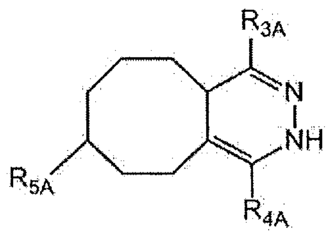

(10c)

In the formula (10a) and the formula (10b), $R_{1A}$ is a binding site with a chelating agent, and $R_{2A}$ is a binding site with the peptide. In the formula (10c), one of $R_{3A}$ and $R_{4A}$ is a hydrogen atom, a methyl group, a phenyl group or a pyridyl group, and the other is a binding site with the chelating agent, and $R_{5A}$ is a binding site with the peptide.

**6.** The conjugate according to claim 5, comprising a polyethylene glycol group between the linkage site with the peptide and the peptide.

**7.** The conjugate according to any one of claims 1 to 6, which is conjugated by a click reaction of an anti-EGFR antibody site-specifically modified with the peptide having an azide group introduced into the N-terminal, and a radioactive metal complex of DOTAGA-DBCO represented by the following formula:

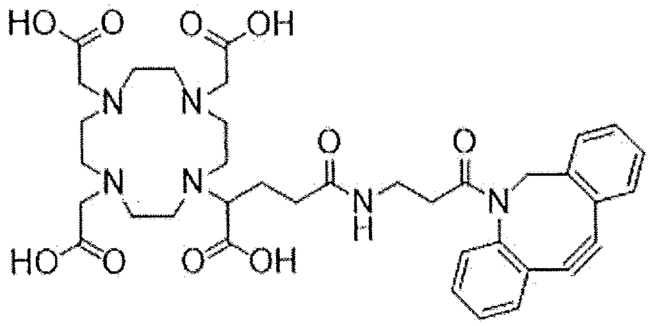

DOTAGA-DBCO

8. The conjugate according to any one of claims 1 to 7, wherein the anti-EGFR antibody is cetuximab or panitumumab.

9. A radiopharmaceutical comprising the conjugate according to any one of claims 1 to 8 as an active ingredient.

10. The radiopharmaceutical according to claim 9, which is used in a radionuclide therapy for cancer.

11. The radiopharmaceutical according to claim 9, which is used in cancer diagnosis.

12. The radiopharmaceutical according to claim 11, which is used in combination with the radionuclide therapy for cancer using the radiopharmaceutical according to claim 10.

13. A radiopharmaceutical containing a conjugate of a chelating agent chelated with a metal radionuclide and an anti-EGFR antibody as an active ingredient and satisfying the following condition (1) or (2), wherein the linkage between the anti-EGFR antibody and the chelating agent does not contain a thiourea bond:

(1) the metal radionuclide is $^{177}Lu$ or $^{90}Y$, and the conjugate has a radiochemical purity of not less than 90% when stored at room temperature for 7 days
(2) the metal radionuclide is $^{225}Ac$, and the conjugate has a radiochemical purity of not less than 90% when stored at room temperature for 14 days.

14. A radiopharmaceutical containing a conjugate of a chelating agent chelated with a metal radionuclide and an anti-EGFR antibody as an active ingredient, wherein the linkage between the anti-EGFR antibody and the chelating agent does not contain a thiourea bond, and the conjugate has a radiochemical purity of not less than 90% at the time of expiry of a period that is a multiple of not less than 1 and not more than 5 of the half-life, based on the half-life of the metal radionuclide.

[Fig. 1]

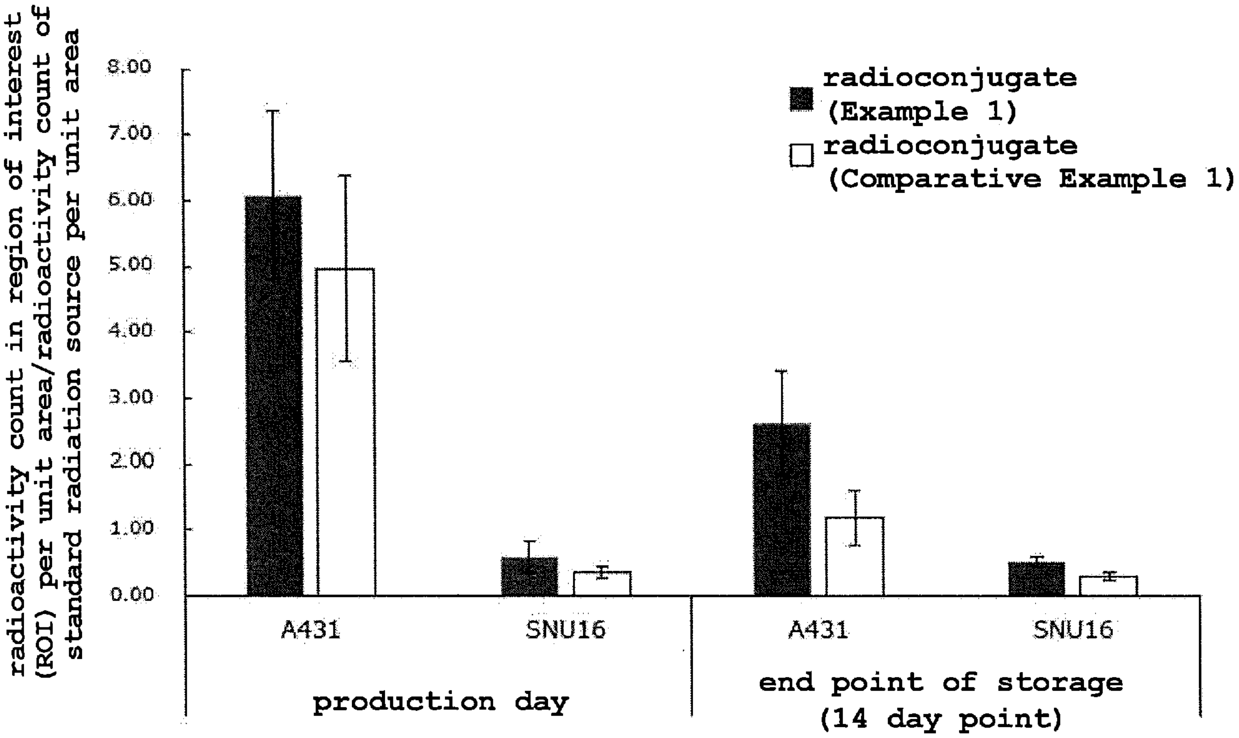
radioactivity count in region of interest (ROI) per unit area/radioactivity count of standard radiation source per unit area
8.00
7.00
6.00
5.00
4.00
3.00
2.00
1.00
0.00
radioconjugate (Example 1)
radioconjugate (Comparative Example 1)
A431
SNU16
A431
SNU16
production day
end point of storage (14 day point)

[Fig. 2]

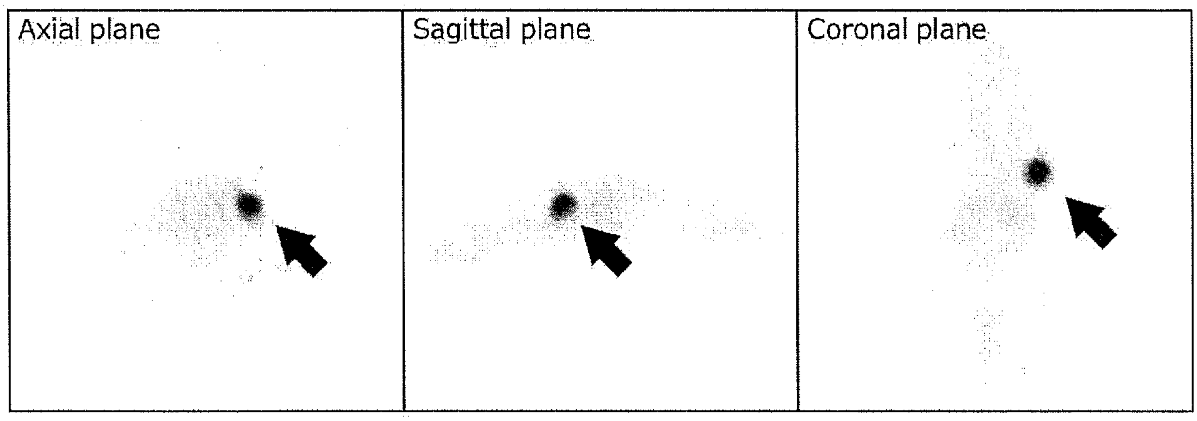
Axial plane
Sagittal plane
Coronal plane

[Fig. 3]

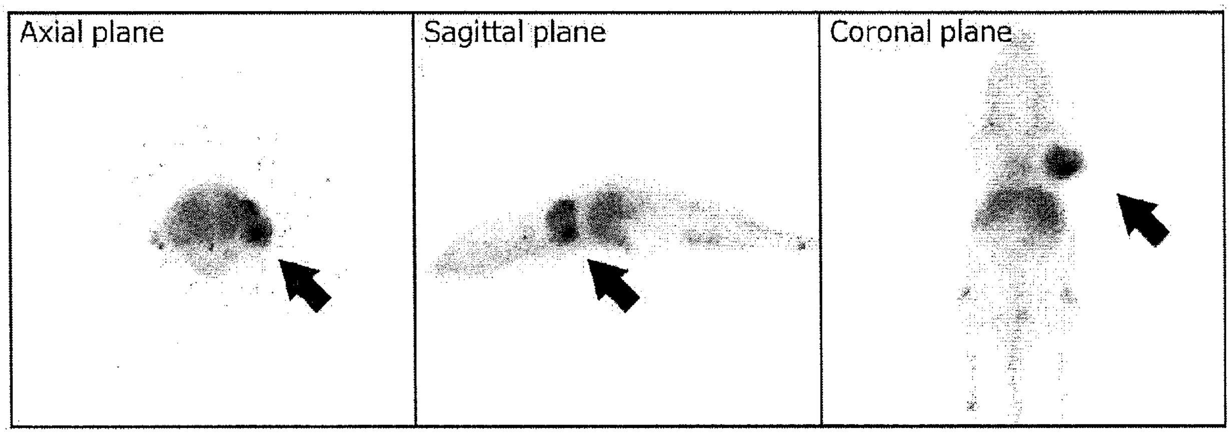

Axial plane
Sagittal plane
Coronal plane


[Fig. 4]

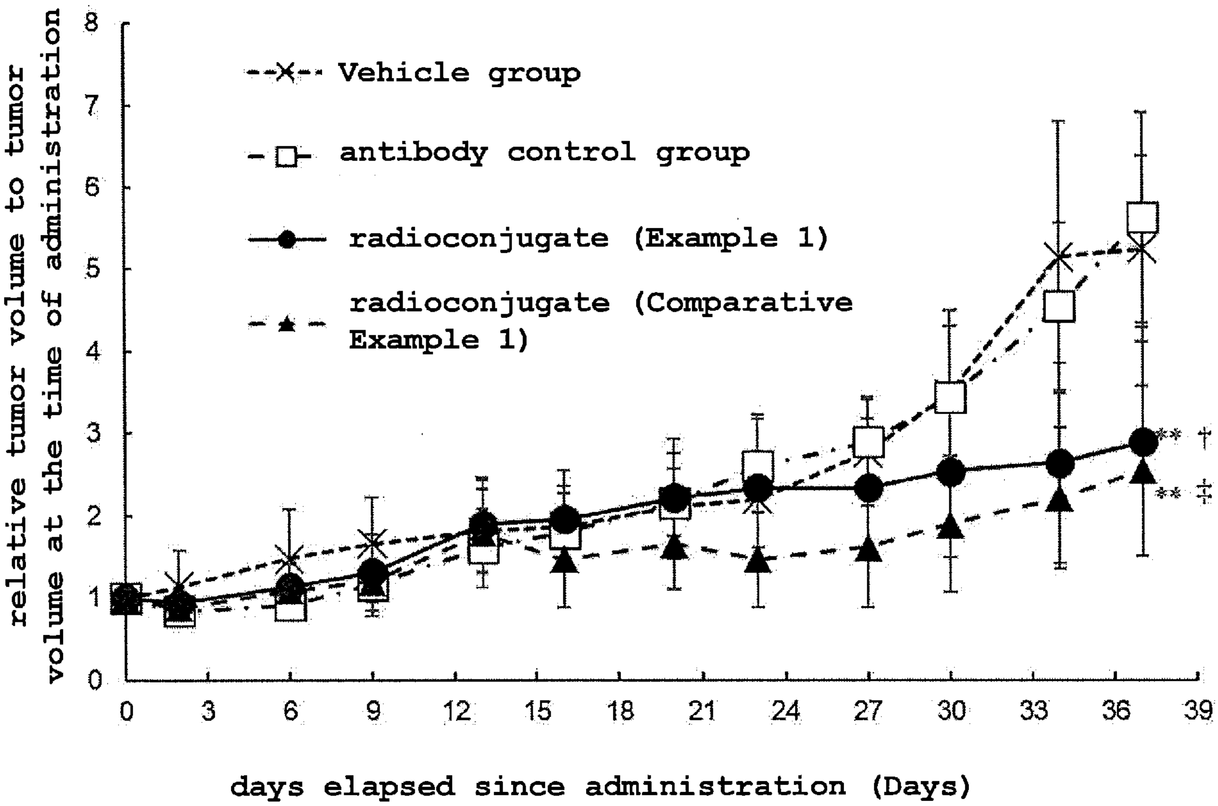

Vehicle group
antibody control group
radioconjugate (Example 1)
radioconjugate (Comparative Example 1)
relative tumor volume to tumor volume at the time of administration
days elapsed since administration (Days)
0
1
2
3
4
5
6
7
8
0
3
6
9
12
15
18
21
24
27
30
33
36
39
** †
** ‡

[Fig. 5]

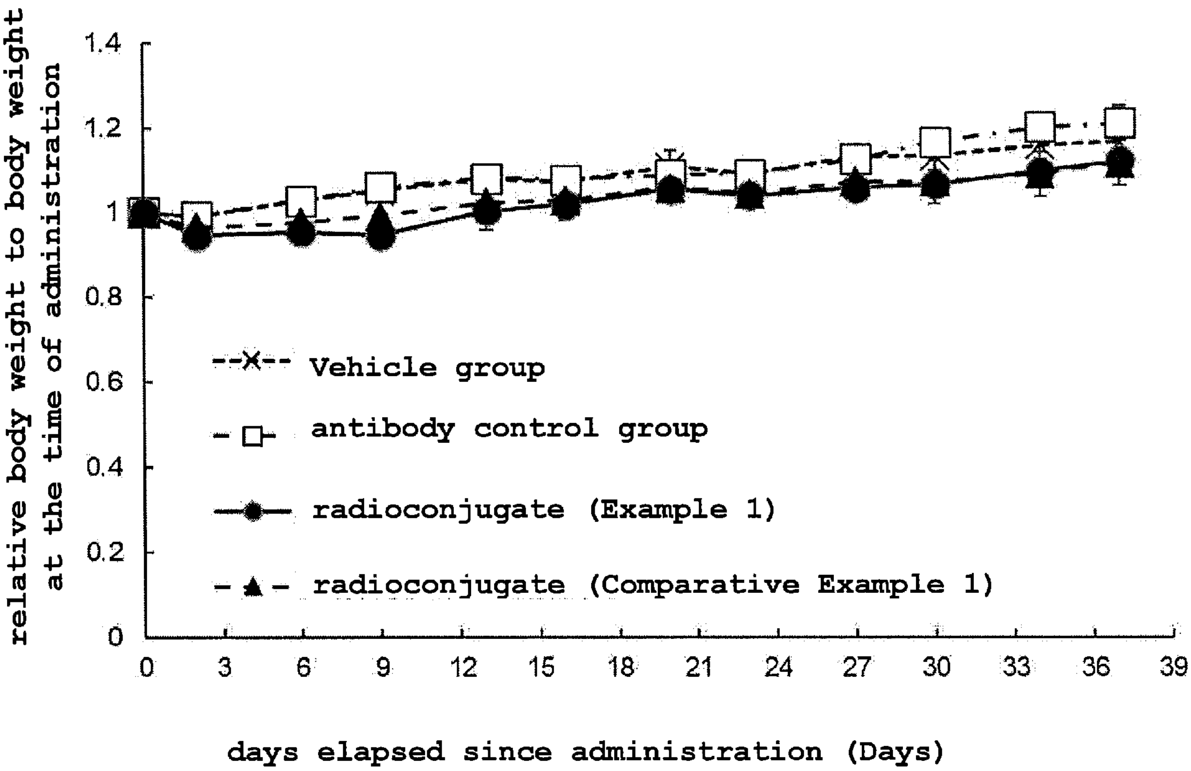
relative body weight to body weight at the time of administration
1.4
1.2
1
0.8
0.6
0.4
0.2
0
0
3
6
9
12
15
18
21
24
27
30
33
36
39
Vehicle group
antibody control group
radioconjugate (Example 1)
radioconjugate (Comparative Example 1)
days elapsed since administration (Days)

[Fig. 6]

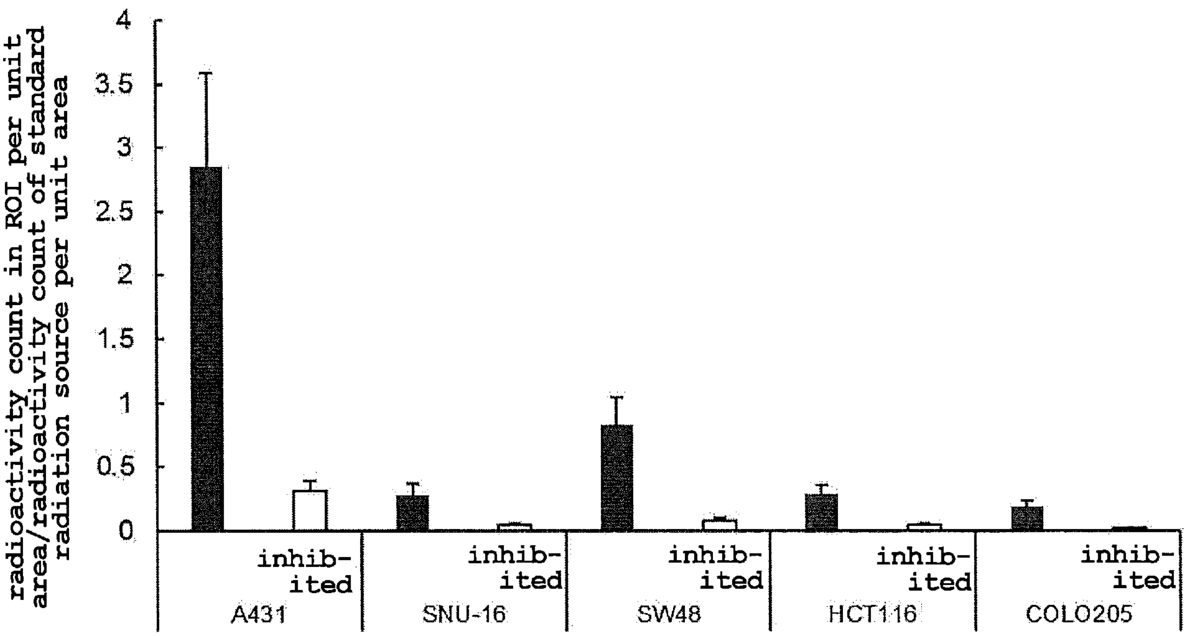
radioactivity count in ROI per unit area/radioactivity count of standard radiation source per unit area
4
3.5
3
2.5
2
1.5
1
0.5
0
inhib-ited
A431
inhib-ited
SNU-16
inhib-ited
SW48
inhib-ited
HCT116
inhib-ited
COLO205

[Fig. 7A]

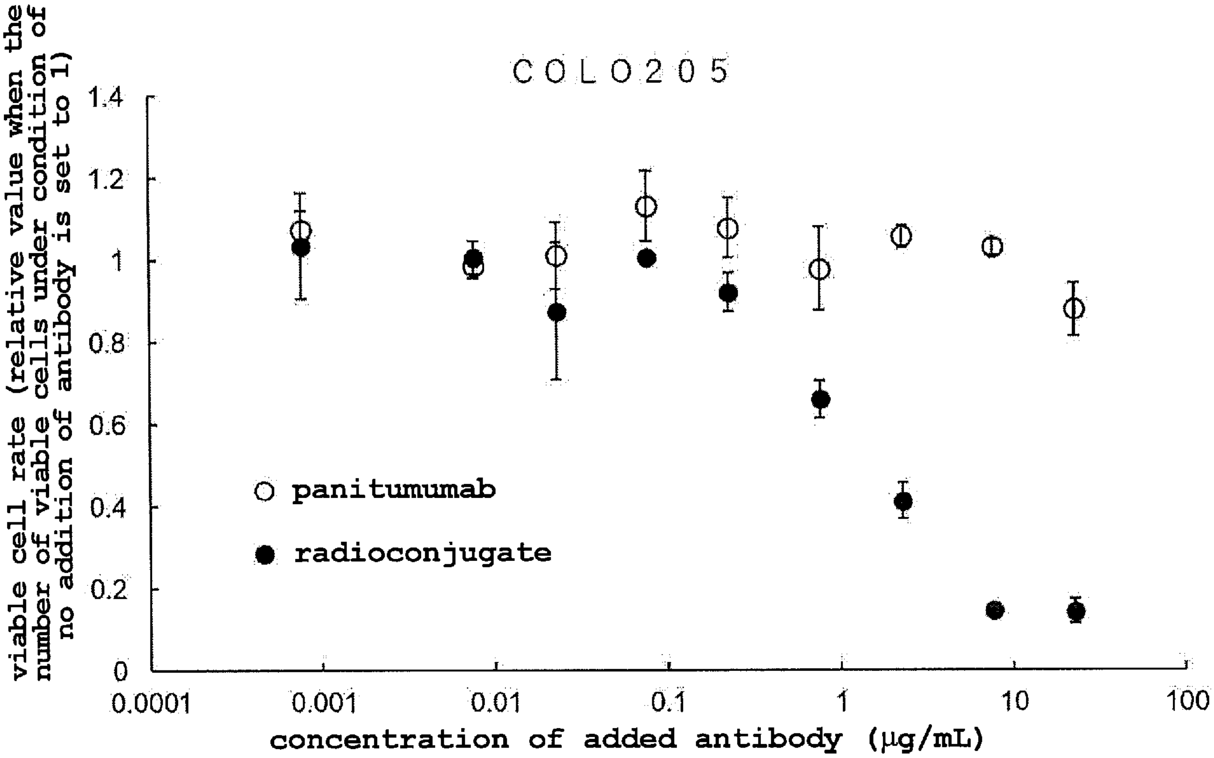
COLO205
viable cell rate (relative value when the number of viable cells under condition of no addition of antibody is set to 1)
1.4
1.2
1
0.8
0.6
0.4
0.2
0
0.0001
0.001
0.01
0.1
1
10
100
concentration of added antibody (μg/mL)
panitumumab
radioconjugate

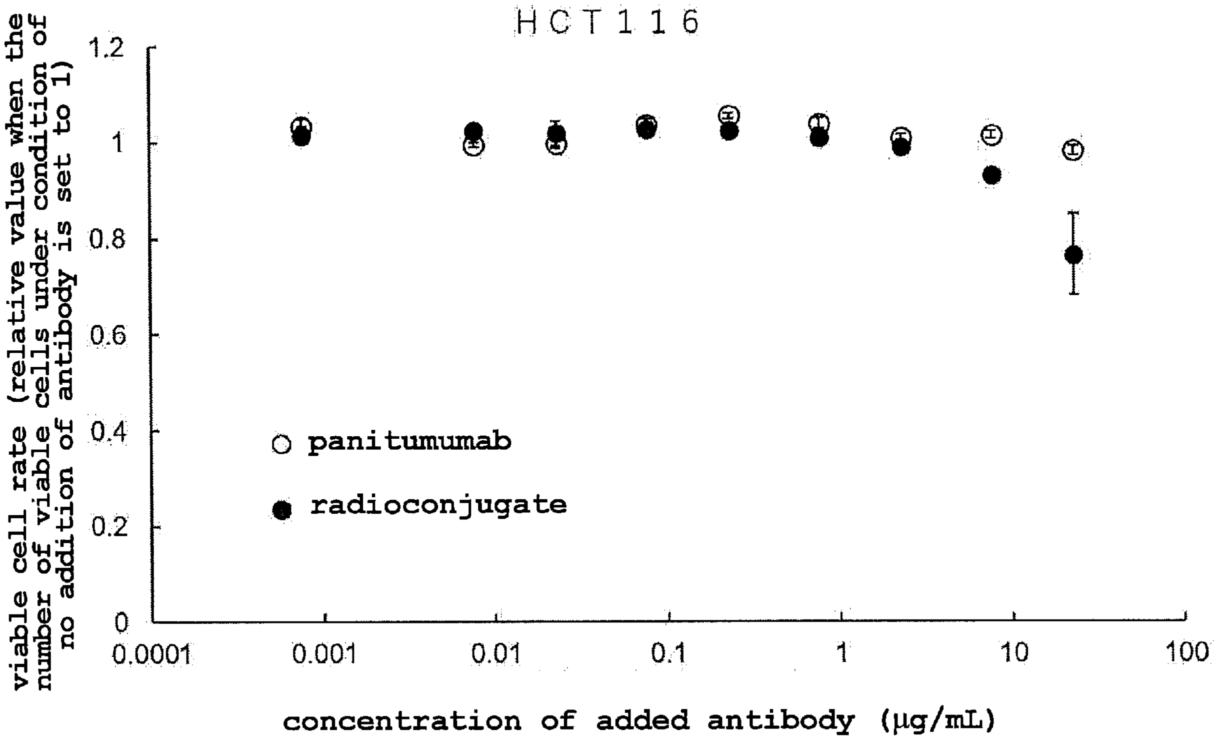
HCT116
viable cell rate (relative value when the number of viable cells under condition of no addition of antibody is set to 1)
1.2
1
0.8
0.6
0.4
0.2
0
0.0001
0.001
0.01
0.1
1
10
100
concentration of added antibody (μg/mL)
panitumumab
radioconjugate

[Fig. 7B]

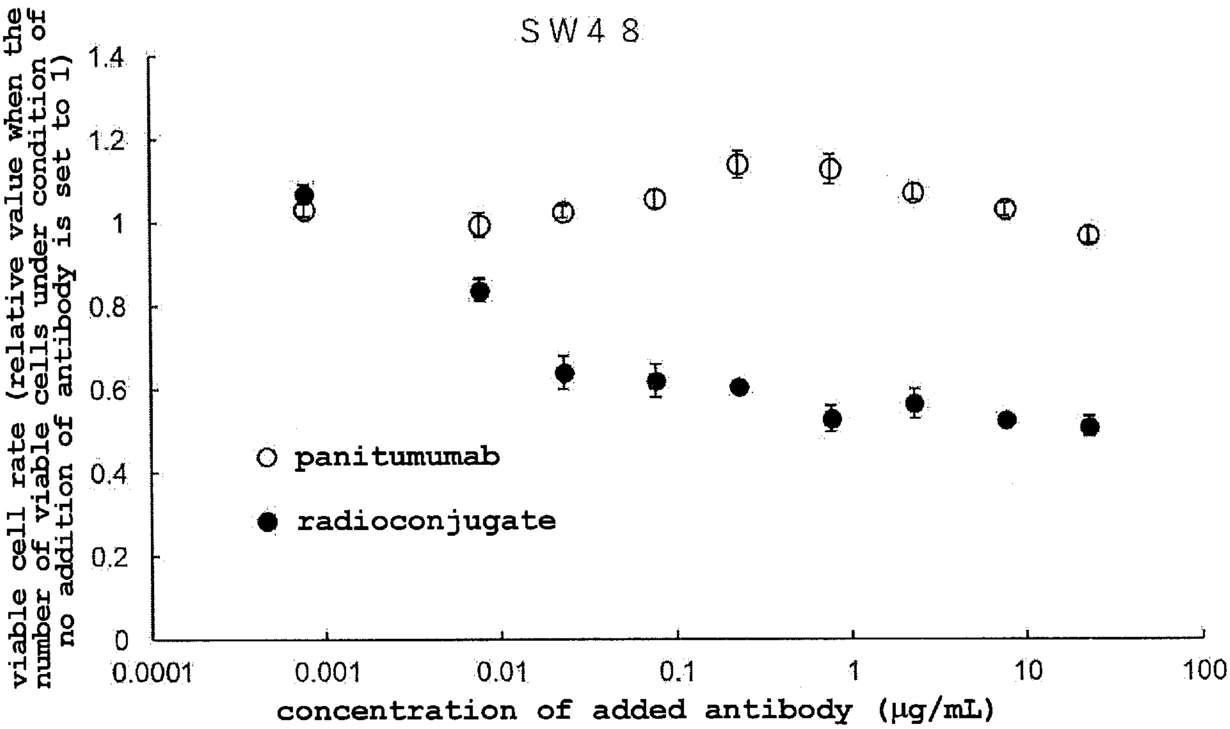
SW48
viable cell rate (relative value when the number of viable cells under condition of no addition of antibody is set to 1)
1.4
1.2
1
0.8
0.6
0.4
0.2
0
0.0001
0.001
0.01
0.1
1
10
100
concentration of added antibody (μg/mL)
panitumumab
radioconjugate

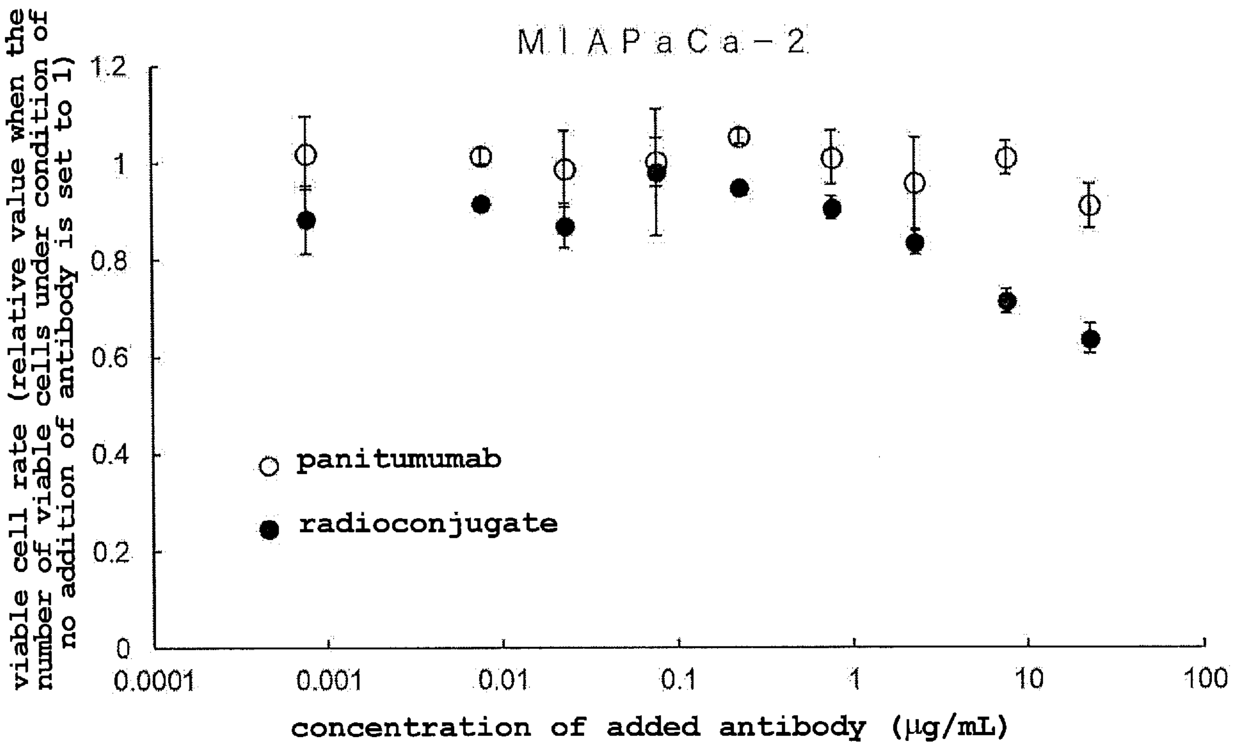
MIAPaCa-2
viable cell rate (relative value when the number of viable cells under condition of no addition of antibody is set to 1)
1.2
1
0.8
0.6
0.4
0.2
0
0.0001
0.001
0.01
0.1
1
10
100
concentration of added antibody (μg/mL)
panitumumab
radioconjugate

[Fig. 7C]

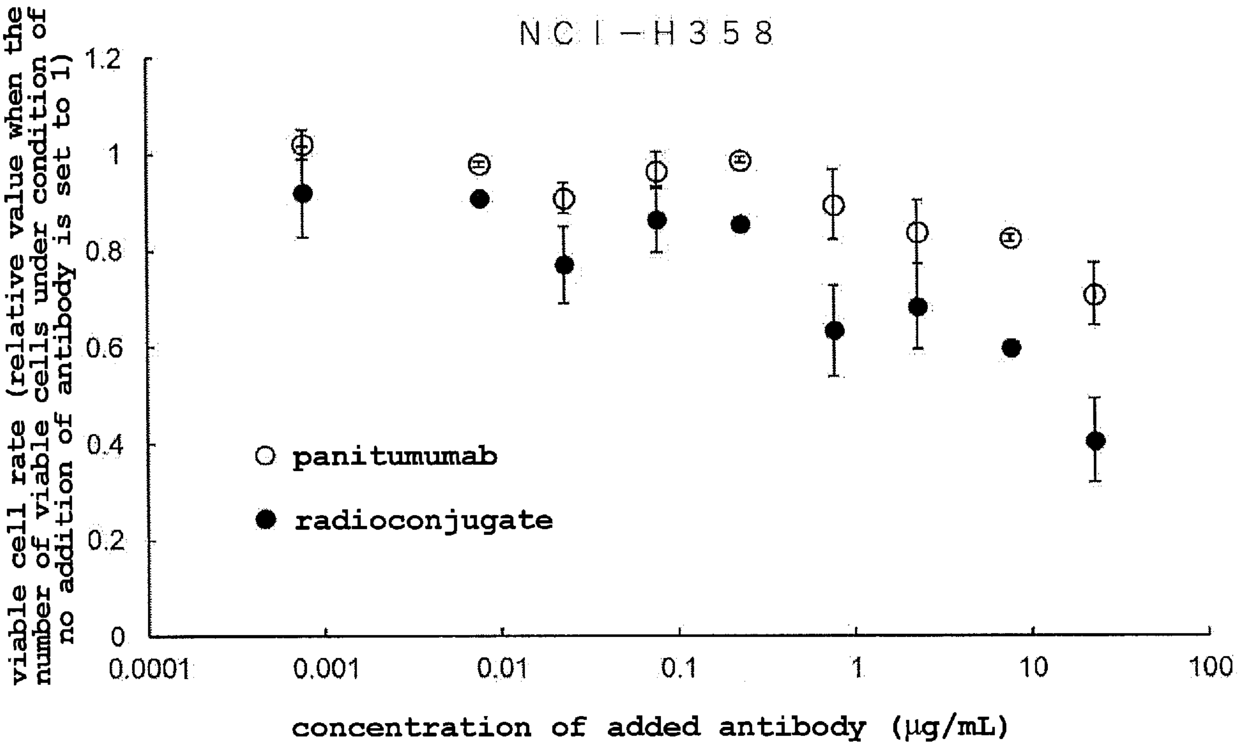
NCI-H358
viable cell rate (relative value when the number of viable cells under condition of no addition of antibody is set to 1)
1.2
1
0.8
0.6
0.4
0.2
0
0.0001
0.001
0.01
0.1
1
10
100
concentration of added antibody (μg/mL)
panitumumab
radioconjugate

[Fig. 8]

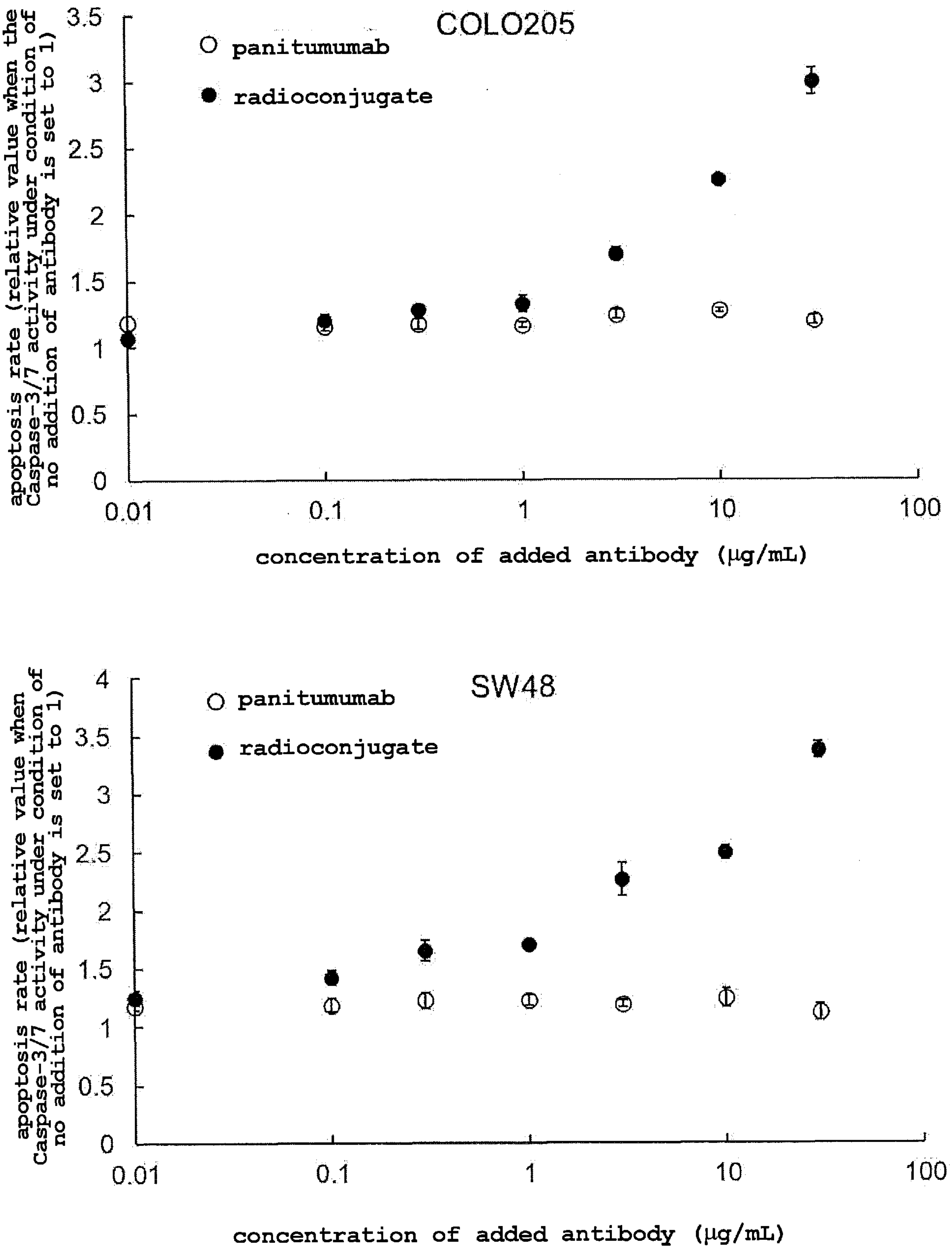
COLO205
panitumumab
radioconjugate
apoptosis rate (relative value when the Caspase-3/7 activity under condition of no addition of antibody is set to 1)
3.5
3
2.5
2
1.5
1
0.5
0
0.01
0.1
1
10
100
concentration of added antibody (μg/mL)
SW48
panitumumab
radioconjugate
apoptosis rate (relative value when Caspase-3/7 activity under condition of no addition of antibody is set to 1)
4
3.5
3
2.5
2
1.5
1
0.5
0
0.01
0.1
1
10
100
concentration of added antibody (μg/mL)

[Fig. 9A]

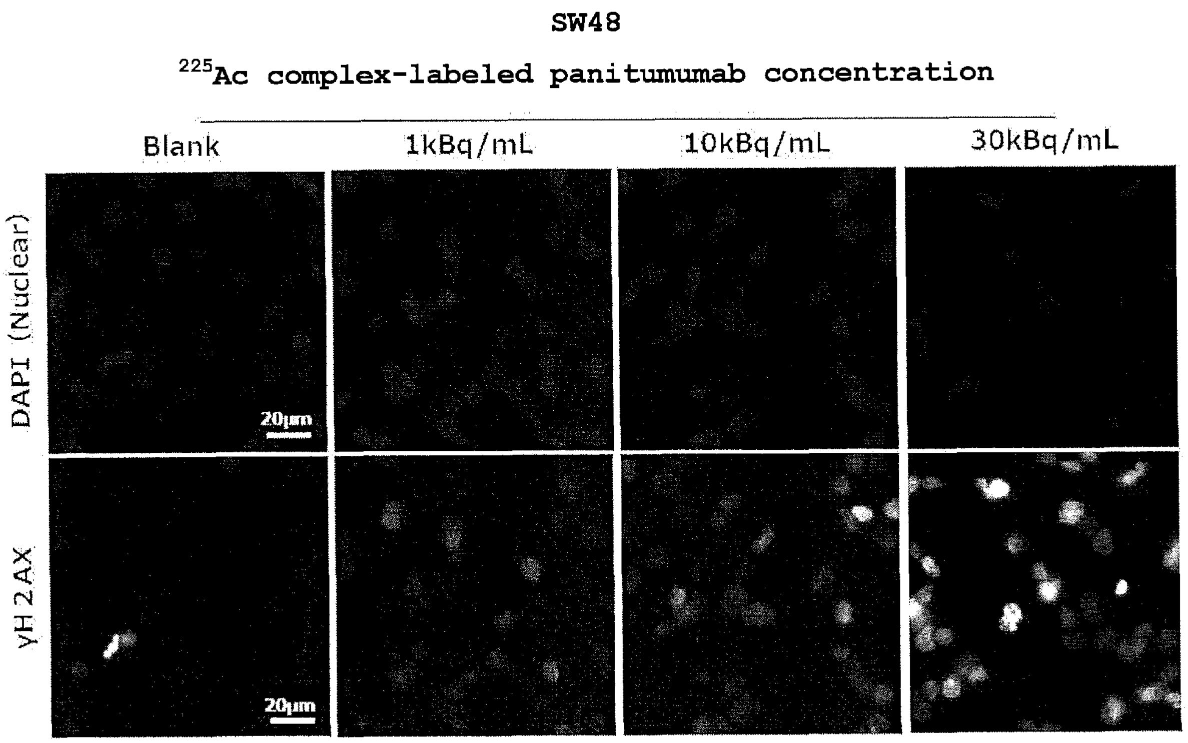
SW48
225Ac complex-labeled panitumumab concentration
Blank
1kBq/mL
10kBq/mL
30kBq/mL
DAPI (Nuclear)
γH2AX
20μm
20μm

[Fig. 9B]

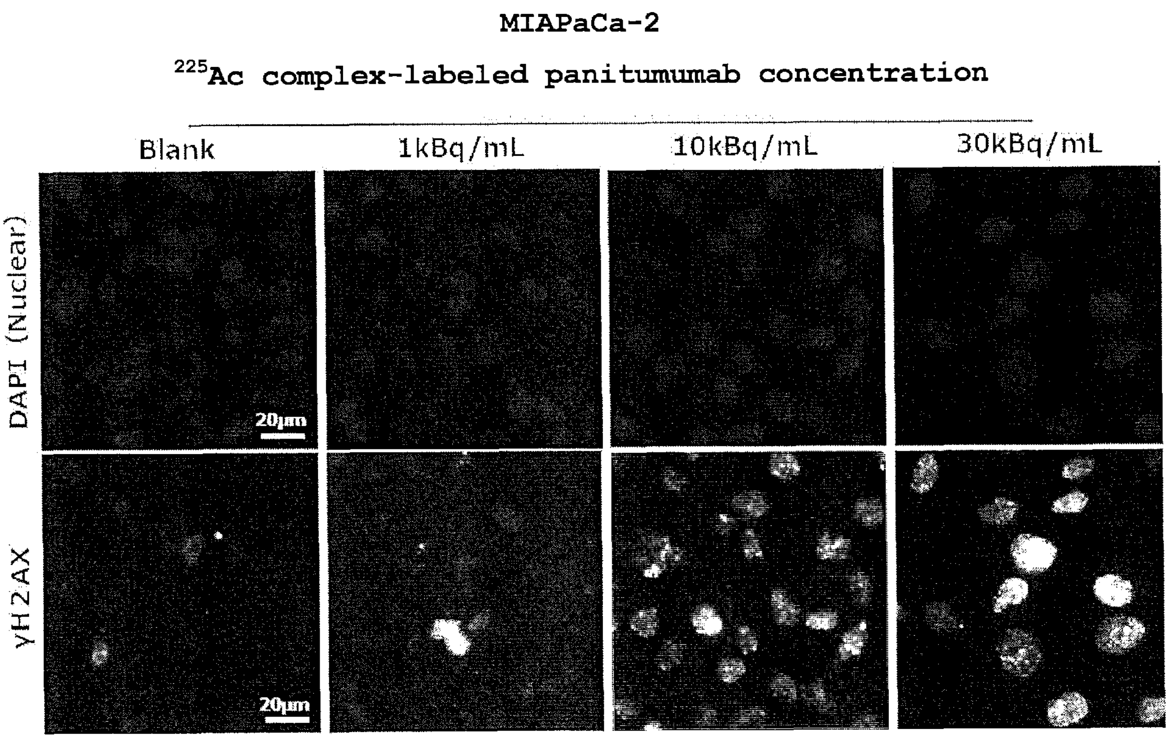
MIAPaCa-2
225Ac complex-labeled panitumumab concentration
Blank
1kBq/mL
10kBq/mL
30kBq/mL
DAPI (Nuclear)
γH2AX
20μm
20μm

[Fig. 9C]

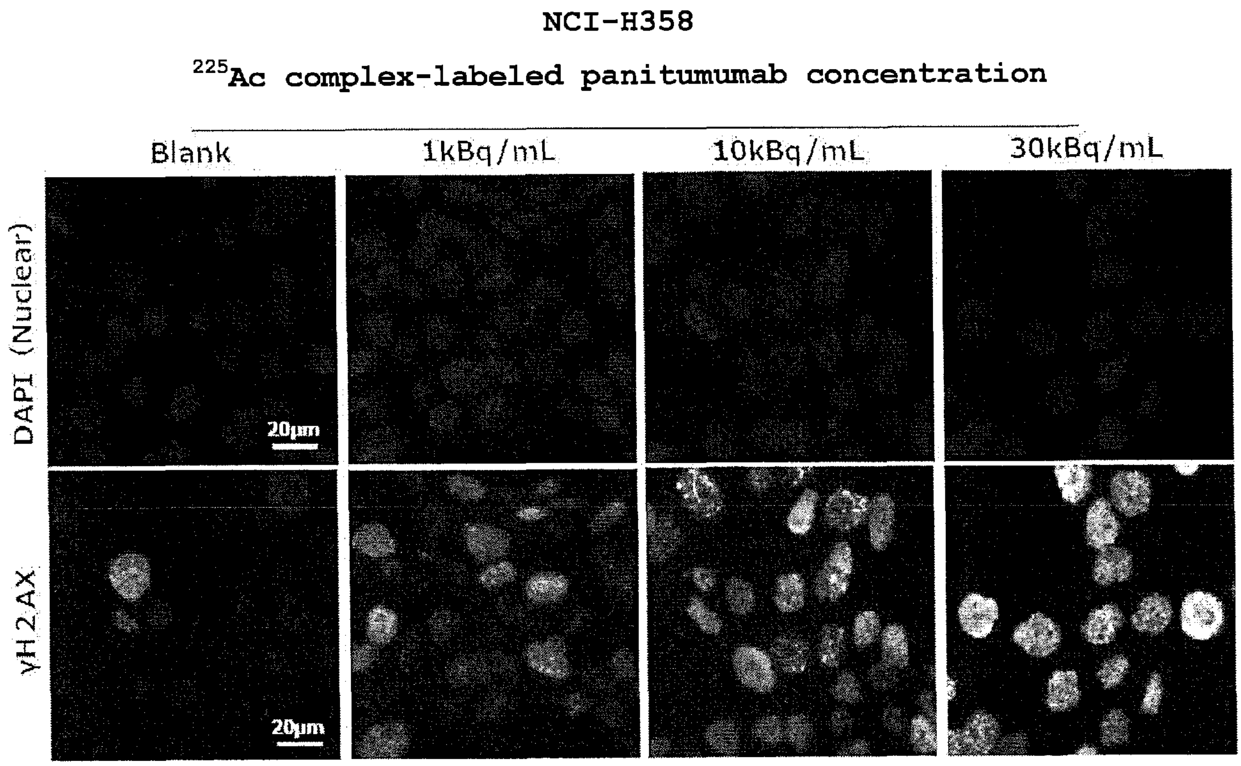
NCI-H358
$^{225}$Ac complex-labeled panitumumab concentration
Blank
1kBq/mL
10kBq/mL
30kBq/mL
DAPI (Nuclear)
γH 2 AX
20μm
20μm

[Fig. 10]

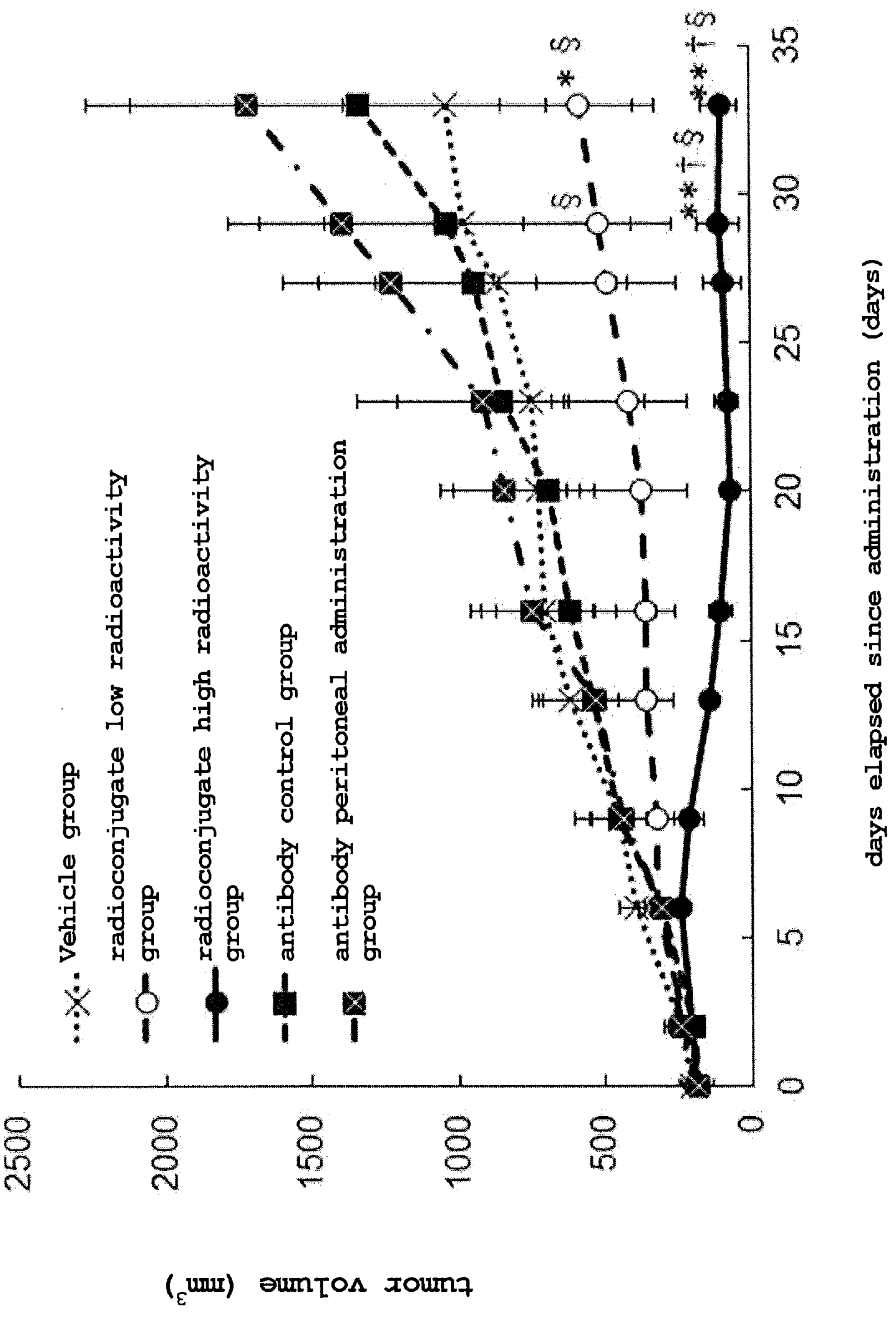
Vehicle group
radioconjugate low radioactivity group
radioconjugate high radioactivity group
antibody control group
antibody peritoneal administration group
tumor volume (mm3)
2500
2000
1500
1000
500
0
0
5
10
15
20
25
30
35
days elapsed since administration (days)
§
* §
** † §
** † §

[Fig. 11]

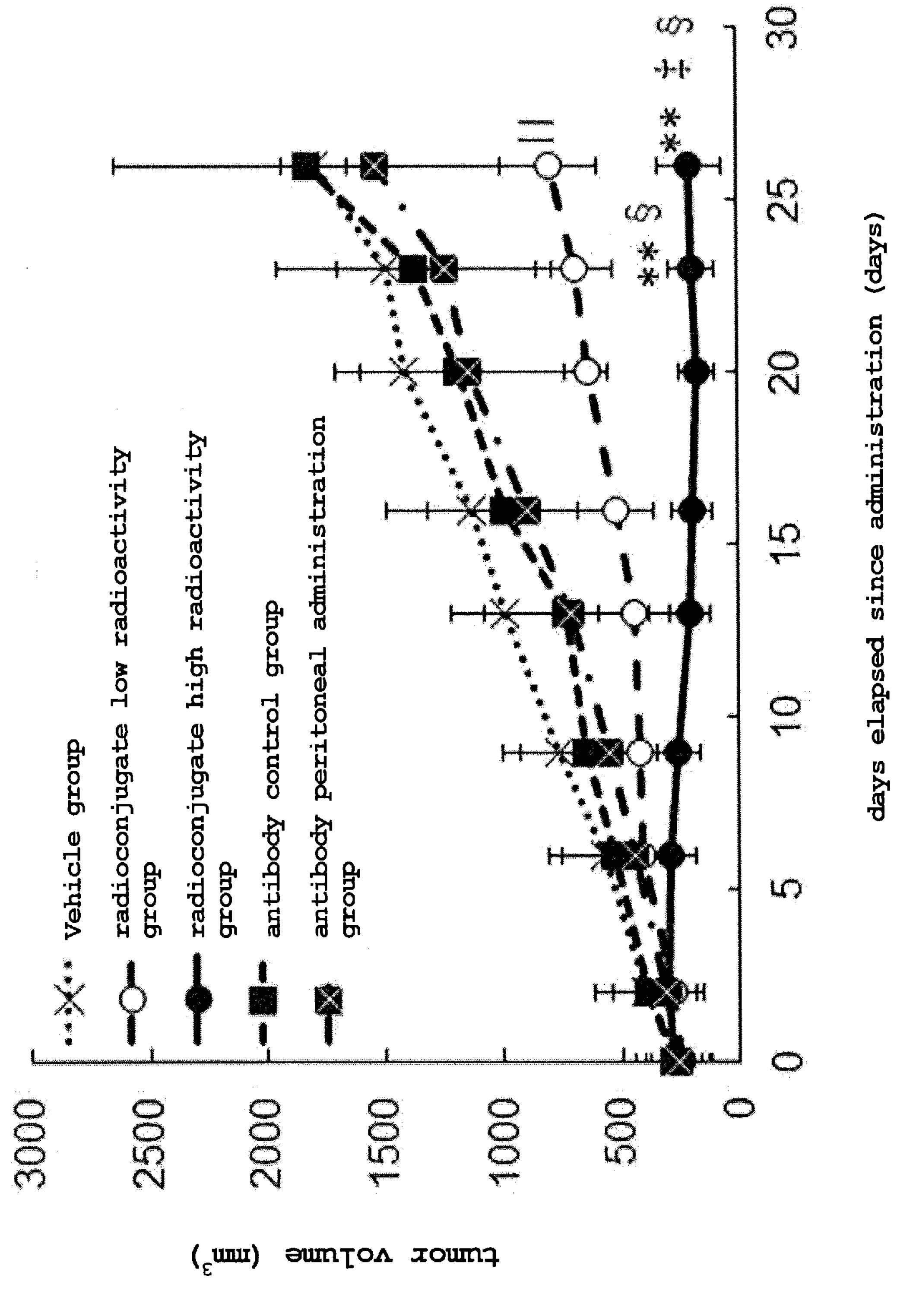
Vehicle group
radioconjugate low radioactivity group
radioconjugate high radioactivity group
antibody control group
antibody peritoneal administration group
tumor volume (mm3)
3000
2500
2000
1500
1000
500
0
0
5
10
15
20
25
30
days elapsed since administration (days)
**
§
||
**
‡
§

11

[Fig. 12]

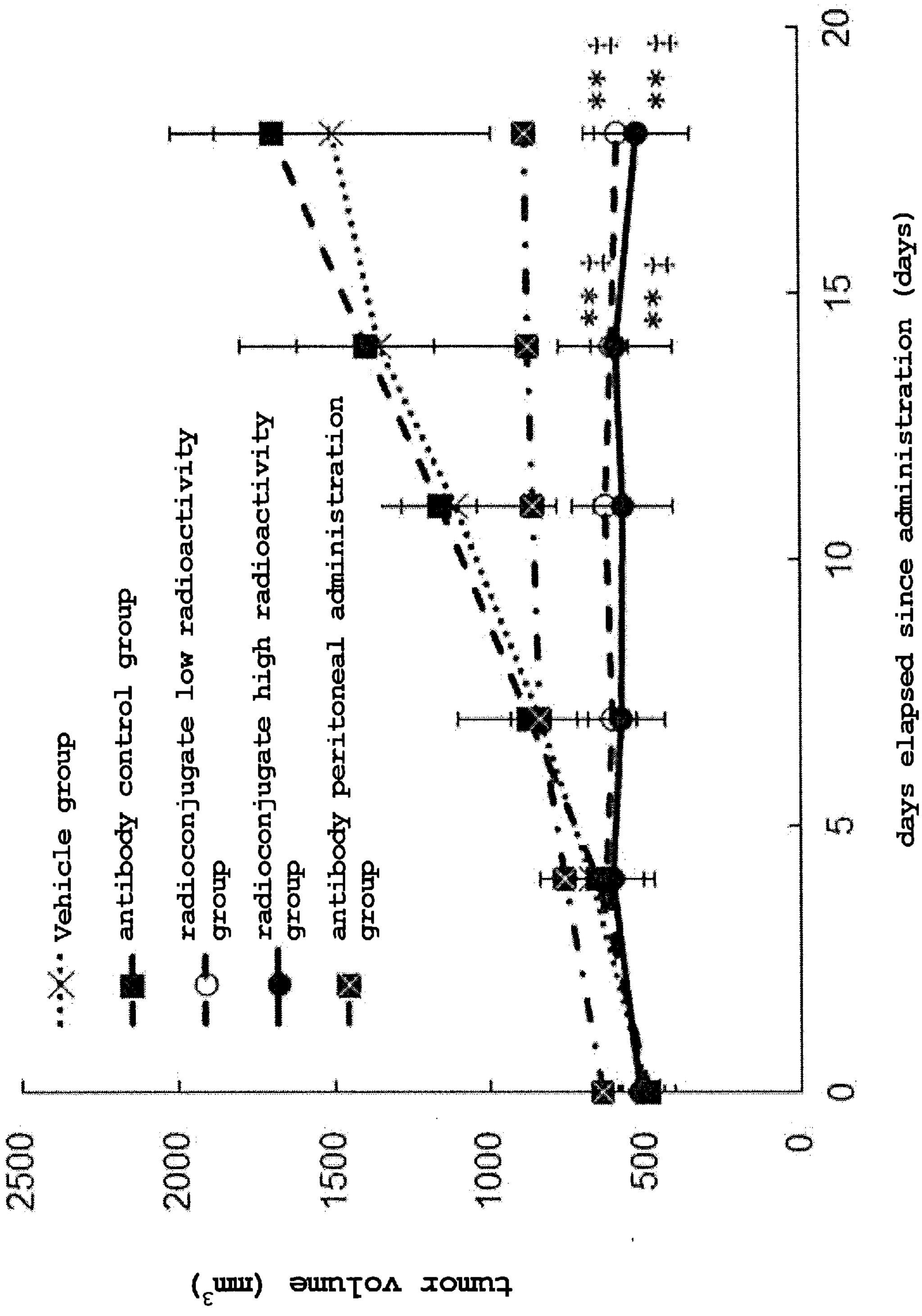
Vehicle group
antibody control group
radioconjugate low radioactivity group
radioconjugate high radioactivity group
antibody peritoneal administration group
tumor volume (mm³)
days elapsed since administration (days)
2500
2000
1500
1000
500
0
0
5
10
15
20
**‡
**‡
**‡
**‡

12

[Fig. 13]

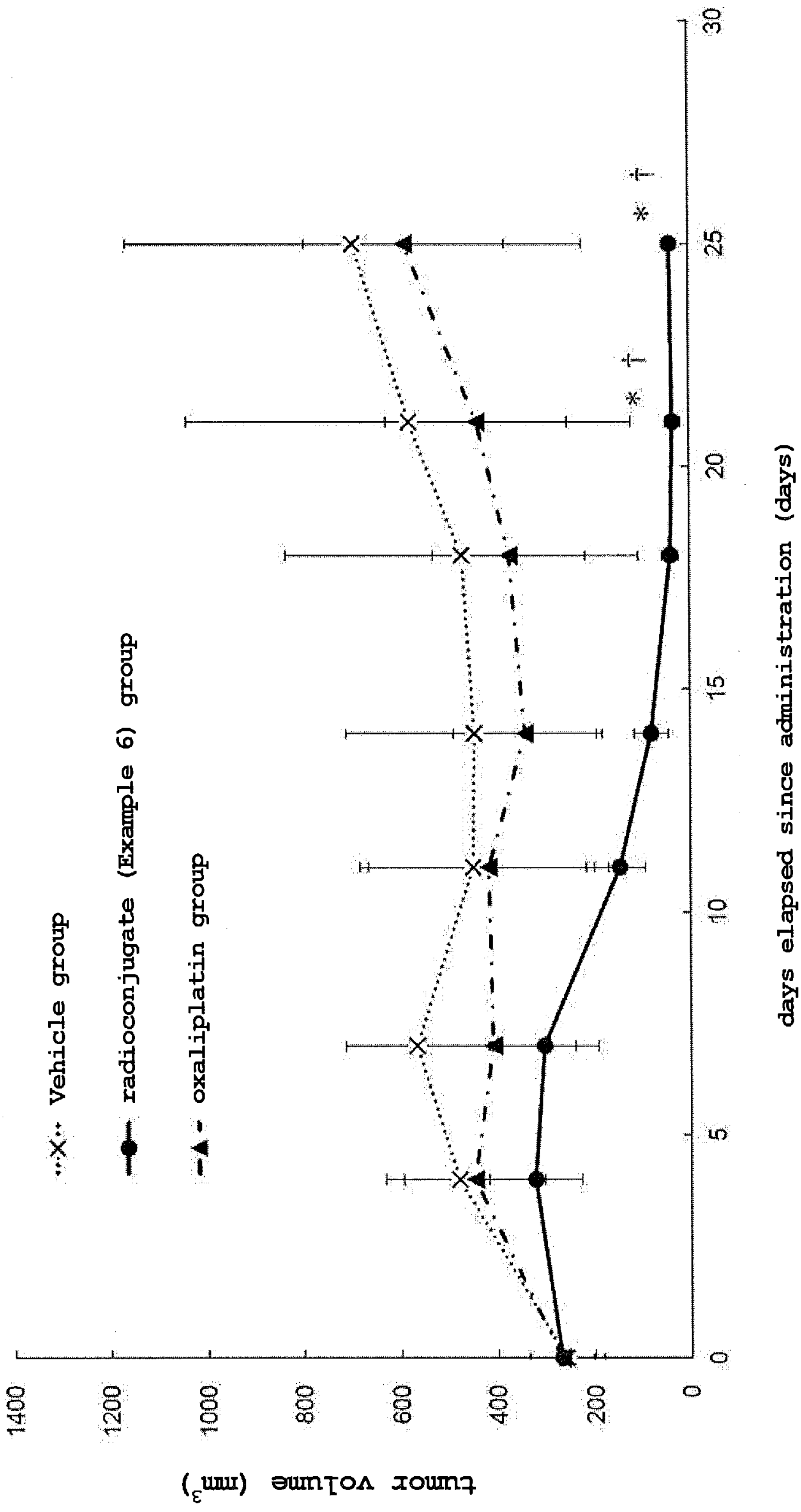
Vehicle group
radioconjugate (Example 6) group
oxaliplatin group
tumor volume (mm3)
0
200
400
600
800
1000
1200
1400
0
5
10
15
20
25
30
days elapsed since administration (days)
*
†

13

[Fig. 14]

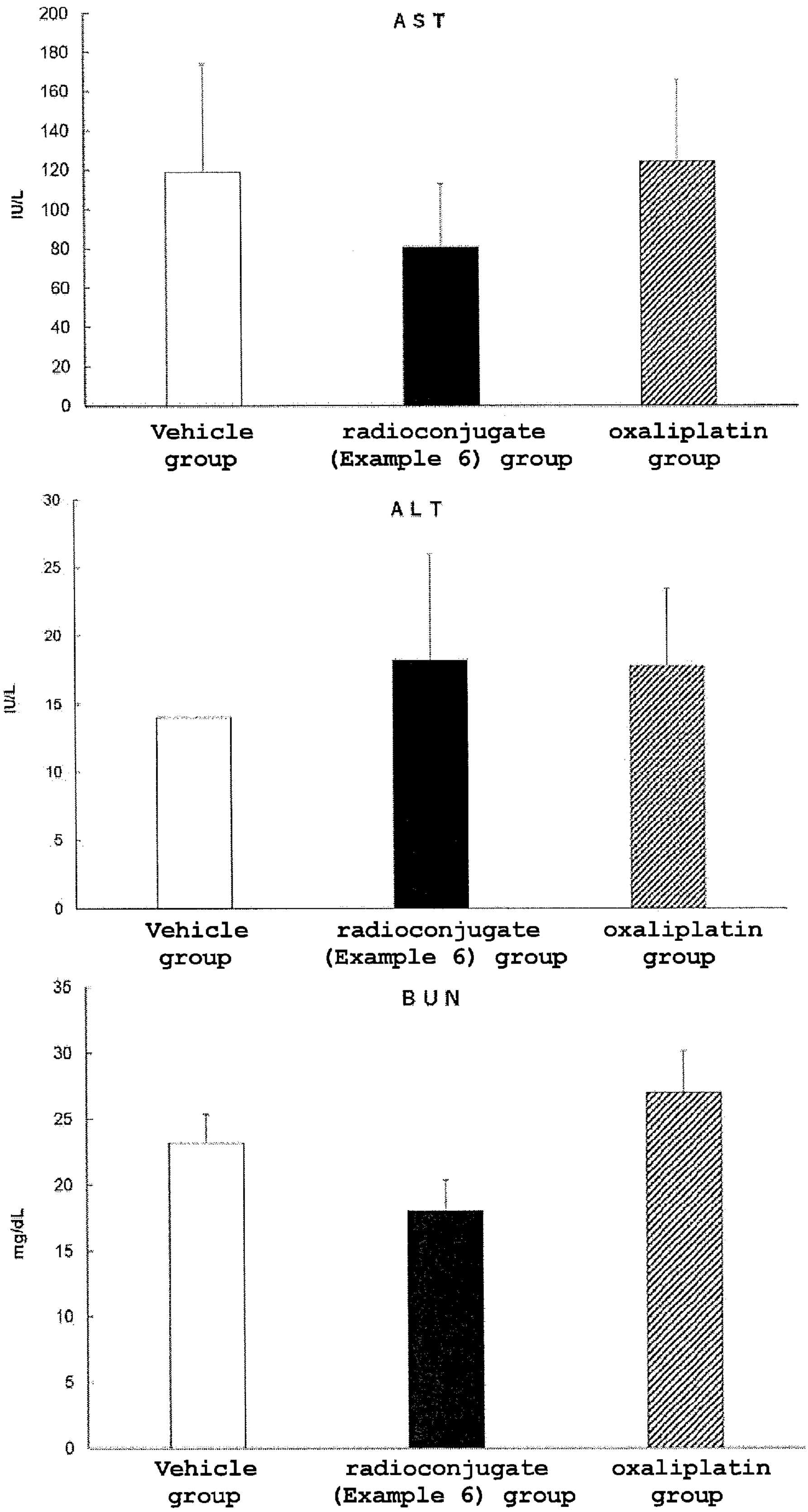
AST
IU/L
200
180
160
140
120
100
80
60
40
20
0
Vehicle group
radioconjugate (Example 6) group
oxaliplatin group
ALT
IU/L
30
25
20
15
10
5
0
Vehicle group
radioconjugate (Example 6) group
oxaliplatin group
BUN
mg/dL
35
30
25
20
15
10
5
0
Vehicle group
radioconjugate (Example 6) group
oxaliplatin group

[Fig. 15]

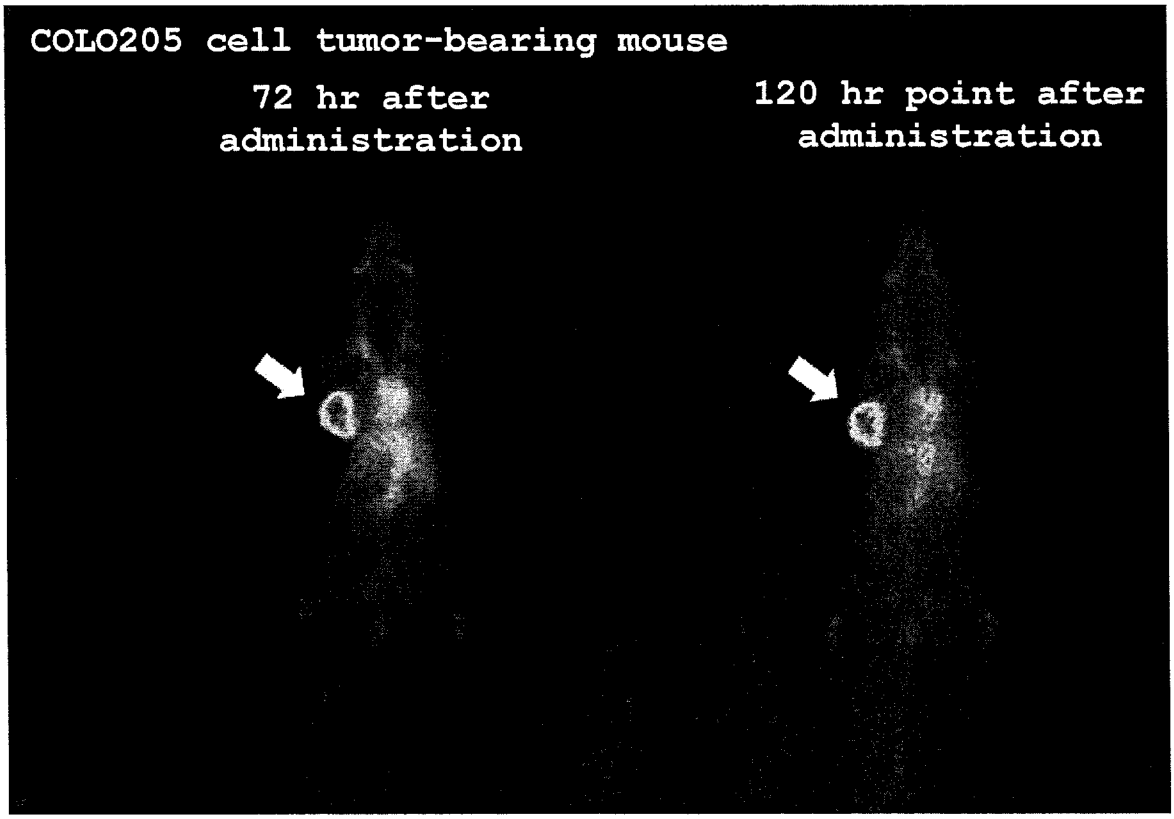
COLO205 cell tumor-bearing mouse
72 hr after administration
120 hr point after administration

**INTERNATIONAL SEARCH REPORT**

International application No.
**PCT/JP2022/016647**

**A. CLASSIFICATION OF SUBJECT MATTER**

***C07K 16/28***(2006.01)i; ***A61K 9/08***(2006.01)i; ***A61K 9/20***(2006.01)i; ***A61K 39/395***(2006.01)i; ***A61K 47/42***(2017.01)i; ***A61K 47/68***(2017.01)i; ***A61K 47/69***(2017.01)i; ***A61K 51/02***(2006.01)i; ***A61K 51/08***(2006.01)i; ***A61K 51/10***(2006.01)i; ***A61P 35/00***(2006.01)i; ***C07K 7/00***(2006.01)i; ***C07K 7/08***(2006.01)i; ***C07K 19/00***(2006.01)i; ***C12N 15/13***(2006.01)i; ***C12P 21/08***(2006.01)i; ***G01T 1/161***(2006.01)i

FI: C07K16/28 ZNA; A61K51/10 200; A61K47/68; A61P35/00 ZNA; A61K51/10 100; G01T1/161 D; A61K51/10; A61K47/69; A61K39/395 N; C07K7/00; C12N15/13; C12P21/08; A61K51/08 100; A61K51/02 100; A61K51/08 200; A61K51/02 200; A61K47/42; A61K9/20; A61K9/08; C07K7/08; A61K39/395 T; C07K19/00

According to International Patent Classification (IPC) or to both national classification and IPC

**B. FIELDS SEARCHED**

Minimum documentation searched (classification system followed by classification symbols)

C07K16/28; A61K9/08; A61K9/20; A61K39/395; A61K47/42; A61K47/68; A61K47/69; A61K51/02; A61K51/08; A61K51/10; A61P35/00; C07K7/00; C07K7/08; C07K19/00; C12N15/13; C12P21/08; G01T1/161

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2019/203191 A1 (NIHON MEDIPHYSICS CO LTD) 24 October 2019 (2019-10-24) claims, paragraphs [0002], [0010], [0059], [0062], [0064], [0067] | 1, 3, 4, 6, 8-14 |
| Y | claims, paragraphs [0002], [0010], [0059], [0062], [0064], [0067] | 1-14 |
| Y | JP 2021-506842 A (JANSSEN BIOTECH, INC) 22 February 2021 (2021-02-22) claims, paragraphs [0039], [0198] | 1-14 |
| A | JP 2013-512918 A (IMMUNOMEDICS, INC.) 18 April 2013 (2013-04-18) paragraph [0134] | 1-14 |
| A | HESKAMP, Sandra et al. 89Zr-Immuno-Positron Emission Tomography in Oncology: State-of-the-Art 89Zr Radiochemistry. Bioconjugate Chemistry. 02 August 2017, vol. 28, pp. 2211-2223 p. 2214, left column | 1-14 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 June 2022** | **14 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/016647**

**C. DOCUMENTS CONSIDERED TO BE RELEVANT**

| Category* | Citation of document, with indication, where appropriate, of the relevant passages | Relevant to claim No. |
|---|---|---|
| Y | JP 2004-509152 A (UNIVERSITY HOSPITAL) 25 March 2004 (2004-03-25) claims, paragraphs [0029], [0048] | 1-14 |
| X | US 2020/0353105 A1 (JANSSEN BIOTECH, INC) 12 November 2020 (2020-11-12) claims, paragraphs [0149], [0241], [0414] | 1, 4-6, 8-10, 13, 14 |
| Y | claims, paragraphs [0149], [0241], [0414] | 3, 4, 11, 12 |

Form PCT/ISA/210 (second sheet) (January 2015)

INTERNATIONAL SEARCH REPORT

International application No.
**PCT/JP2022/016647**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2022/016647**

| Patent document cited in search report | Publication date (day/month/year) | Patent family member(s) | Publication date (day/month/year) |
|---|---|---|---|
| WO 2019/203191 A1 | 24 October 2019 | US 2021/0170058 A1<br>claims, paragraphs [0002], [0020], [0113], [0116], [0120], [0121]<br>EP 3783016 A1<br>CN 112041337 A<br>KR 10-2020-0143366 A | |
| JP 2021-506842 A | 22 February 2021 | US 2021/0017099 A1<br>claims, paragraphs [0059], [0266]<br>WO 2019/125982 A1<br>EP 3727474 A1<br>CN 111491670 A<br>KR 10-2020-0100094 A | |
| JP 2013-512918 A | 18 April 2013 | US 2011/0110854 A1<br>WO 2011/068965 A1<br>paragraph [0145]<br>EP 2507254 A1<br>CN 102666567 A | |
| JP 2004-509152 A | 25 March 2004 | US 2006/0233704 A1<br>WO 2002/024235 A2<br>claims, pp. 7, 12<br>EP 1289571 A2 | |
| US 2020/0353105 A1 | 12 November 2020 | WO 2020/229974 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017214308 A
- WO 2017217347 A
- JP 2005047934 A
- WO 2016186206 A
- WO 2018230257 A
- WO 2021080008 A
- JP 2021062104 A
- JP 2021179348 A
- JP 2022021670 A


### Non-patent literature cited in the description


- **BERNHARD et al.** DOTAGA-Anhydride: A Valuable Building Block for the Preparation of DOTA-Like Chelating Agents. *Chem. Eur. J.,* 2012, vol. 18, 7834-7841